# EUROPEAN PATENT APPLICATION

(11) **EP 3 438 259 A1**
(43) Date of publication of application: **06.02.2019**
(21) Application number: 17775005.6
(22) Date of filing: 28.03.2017
(51) Int. Cl.: C12N 15/09, C12N 1/15, C12N 1/19, C12N 1/21, C12N 5/10, C12P 21/02

(54) **METHANOL-UTILIZING ENZYME-DERIVED NOVEL PROTEIN AND METHOD FOR PRODUCING INTENDED PROTEIN USING SAME**

(30) Priority: 28.03.2016 JP 2016064364
(71) Applicant: Kaneka Corporation, Osaka-shi, Osaka 530-8288 (JP)
(72) Inventor: NISHI, Teruyuki, Takasago-shi Hyogo 676-8688 (JP)
(74) Representative: Hoffmann Eitle
(86) International application number: PCT/JP2017/012510
(87) International publication number: WO 2017/170468

(57) **Abstract**

It is an object of the present invention to provide a new means for improving secretion amount of an endogenous protein and a heterologous protein. The present invention solves the above object by a vector comprising a nucleotide sequence encoding a novel protein derived from a methanol-utilizing yeast, a mutant cell expressing a gene encoding the protein at a high level, and the like.

SEQUENCE LISTING

<110>  Kaneka Corporation

<120>  Novel proteins derived from methanol-utilizing yeast and a method
       for producing proteins of interest using them

<130>  B160401

<150>  JP 2016-064364
<151>  2016-03-28

<160>  120

<170>  PatentIn version 3.5

<210>  1
<211>  30
<212>  DNA
<213>  Artificial

<220>
<223>  Synthetic

<400>  1
aagcttggat ccagatcttc tagagaattc                                        30


<210>  2
<211>  933
<212>  DNA
<213>  Artificial

<220>
<223>  Synthetic

<400>  2
aacatccaaa gacgaaaggt tgaatgaaac ctttttgcca tccgacatcc acaggtccat      60

tctcacacat aagtgccaaa cgcaacagga ggggatacac tagcagcaga ccgttgcaaa     120

cgcaggacct ccactcctct tctcctcaac acccactttt gccatcgaaa aaccagccca     180

gttattgggc ttgattggag ctcgctcatt ccaattcctt ctattaggct actaacacca     240

tgactttatt agcctgtcta tcctggcccc cctggcgagg ttcatgtttg tttatttccg     300

aatgcaacaa gctccgcatt acacccgaac atcactccag atgagggctt tctgagtgtg     360

gggtcaaata gtttcatgtt ccccaaatgg cccaaaactg acagtttaaa cgctgtcttg     420

gaacctaata tgacaaaagc gtgatctcat ccaagatgaa ctaagtttgg ttcgttgaaa     480

tgctaacggc cagttggtca aaaagaaact tccaaaagtc ggcataccgt ttgtcttgtt     540

tggtattgat tgacgaatgc tcaaaaataa tctcattaat gcttagcgca gtctctctat     600

cgcttctgaa ccccggtgca cctgtgccga aacgcaaatg gggaaacacc cgctttttgg     660

atgattatgc attgtctcca cattgtatgc ttccaagatt ctggtgggaa tactgctgat     720

agcctaacgt tcatgatcaa aatttaactg ttctaacccc tacttgacag caatatataa     780

acagaaggaa gctgccctgt cttaaacctt ttttttatca tcattattag cttactttca 840

taattgcgac tggttccaat tgacaagctt ttgattttaa cgacttttaa cgacaacttg 900

agaagatcaa aaaacaacta attattcgaa acg 933

<210> 3
<211> 31
<212> DNA
<213> Artificial

<220>
<223> Primer

<400> 3
ttaggatcca acatccaaag acgaaaggtt g 31

<210> 4
<211> 38
<212> DNA
<213> Artificial

<220>
<223> Primer

<400> 4
ttaggatccc gtttcgaata attagttgtt ttttgatc 38

<210> 5
<211> 500
<212> DNA
<213> Artificial

<220>
<223> Synthetic

<400> 5
tcaagaggat gtcagaatgc catttgcctg agagatgcag gcttcatttt tgatactttt 60

ttatttgtaa cctatatagt ataggatttt ttttgtcatt ttgtttcttc tcgtacgagc 120

ttgctcctga tcagcctatc tcgcagctga tgaatatctt gtggtagggg tttgggaaaa 180

tcattcgagt ttgatgtttt tcttggtatt tcccactcct cttcagagta cagaagatta 240

agtgagacgt tcgtttgtgc aagcttcaac gatgccaaaa gggtataata agcgtcattt 300

gcagcattgt gaagaaaact atgtggcaag ccaagcctgc gaagaatgta ttttaagttt 360

gactttgatg tattcacttg attaagccat aattctcgag tatctatgat tggaagtatg 420

ggaatggtga tacccgcatt cttcagtgtc ttgaggtctc ctatcagatt atgcccaact 480

aaagcaaccg gaggaggaga 500

<210> 6
<211> 39
<212> DNA
<213> Artificial

<220>
<223>  Primer

<400>  6
ttatctagat caagaggatg tcagaatgcc atttgcctg                              39


<210>  7
<211>  31
<212>  DNA
<213>  Artificial

<220>
<223>  Primer

<400>  7
ttatctagat ctcctcctcc ggttgcttta g                                      31


<210>  8
<211>  2656
<212>  DNA
<213>  Artificial

<220>
<223>  Synthetic

<400>  8
agatctcctg atgactgact cactgataat aaaaatacgg cttcagaatt tctcaagact       60

acactcactg tccgacttca agtatgacat ttcccttgct acctgcatac gcaagtgttg      120

cagagtttga taattccttg agtttggtag gaaaagccgt gtttccctat gctgctgacc      180

agctgcacaa cctgatcaag ttcactcaat cgactgagct tcaagttaat gtgcaagttg      240

agtcatccgt tacagaggac caatttgagg agctgatcga caacttgctc aagttgtaca      300

ataatggtat caatgaagtg attttggacc tagatttggc agaaagagtt gtccaaagga      360

tcccaggcgc tagggttatc tataggaccc tggttgataa agttgcatcc ttgcccgcta      420

atgctagtat cgctgtgcct ttttcttctc cactgggcga tttgaaaagt ttcactaatg      480

gcggtagtag aactgtttat gctttttctg agaccgcaaa gttggtagat gtgacttcca      540

ctgttgcttc tggtataatc cccattattg atgctcggca attgactact gaatacgaac      600

tttctgaaga tgtcaaaaag ttccctgtca gtgaaatttt gttggcgtct ttgactactg      660

accgccccga tggtctattc actactttgg tggctgactc ttctaattac tcgttgggcc      720

tggtgtactc gtccaaaaag tctattccgg aggctataag gacacaaact ggagtctacc      780

aatctcgtcg tcacggtttg tggtataaag gtgctacatc tggagcaact caaaagttgc      840

tgggtatcga attggattgt gatggagact gcttgaaatt tgtggttgaa caaacaggtg      900

ttggtttctg tcacttggaa cgcacttcct gttttggcca atcaaagggt cttagagcca      960

tggaagccac cttgtgggat cgtaagagca atgctccaga aggttcttat accaaacggt     1020

tatttgacga cgaagttttg ttgaacgcta aaattaggga ggaagctgat gaacttgcag 1080

aagctaaatc caaggaagat atagcctggg aatgtgctga cttattttat tttgcattag 1140

ttagatgtgc caagtacggt gtgacgttgg acgaggtgga gagaaacctg gatatgaagt 1200

ccctaaaggt cactagaagg aaaggagatg ccaagccagg atacaccaag gaacaaccta 1260

aagaagaatc caaacctaaa gaagtccctt ctgaaggtcg tattgaattg tgcaaaattg 1320

acgtttctaa ggcctcctca caagaaattg aagatgccct tcgtcgtcct atccagaaaa 1380

cggaacagat tatggaatta gtcaaaccaa ttgtcgacaa tgttcgtcaa aatggtgaca 1440

aagccctttt agaactaact gccaagtttg atggagtcgc tttgaagaca cctgtgttag 1500

aagctccttt cccagaggaa cttatgcaat tgccagataa cgttaagaga gccattgatc 1560

tctctataga taacgtcagg aaattccatg aagctcaact aacggagacg ttgcaagttg 1620

agacttgccc tggtgtagtc tgctctcgtt ttgcaagacc tattgagaaa gttggcctct 1680

atattcctgg tggaaccgca attctgcctt ccacttccct gatgctgggt gttcctgcca 1740

aagttgctgg ttgcaaagaa attgtttttg catctccacc taagaaggat ggtaccctta 1800

ccccagaagt catctacgtt gcccacaagg ttggtgctaa gtgtatcgtg ctagcaggag 1860

gcgcccaggc agtagctgct atggcttacg gaacagaaac tgttcctaag tgtgacaaaa 1920

tatttggtcc aggaaaccag ttcgttactg ctgccaagat gatggttcaa aatgacacat 1980

cagccctgtg tagtattgac atgcctgctg ggccttctga agttctagtt attgctgata 2040

aatacgctga tccagatttc gttgcctcag accttctgtc tcaagctgaa catggtattg 2100

attcccaggt gattctgttg gctgtcgata tgacagacaa ggagcttgcc agaattgaag 2160

atgctgttca caaccaagct gtgcagttgc caagggttga aattgtacgc aagtgtattg 2220

cacactctac aaccctatcg gttgcaacct acgagcaggc tttggaaatg tccaatcagt 2280

acgctcctga acacttgatc ctgcaaatcg agaatgcttc ttcttatgtt gatcaagtac 2340

aacacgctgg atctgtgttt gttggtgcct actctccaga gagttgtgga gattactcct 2400

ccggtaccaa ccacactttg ccaacgtacg gatatgcccg tcaatacagc ggagttaaca 2460

ctgcaacctt ccagaagttc atcacttcac aagacgtaac tcctgaggga ctgaaacata 2520

ttggccaagc agtgatggat ctggctgctg ttgaaggtct agatgctcac cgcaatgctg 2580

ttaaggttcg tatggagaaa ctgggactta tttaattatt tagagatttt aacttacatt 2640

tagattcgat agatct 2656

<210> 9
<211> 32
<212> DNA
<213> Artificial

<220>

<223>  Primer

<400>  9
ttagaattcg gatctcctga tgactgactc ac                                    32


<210>  10
<211>  41
<212>  DNA
<213>  Artificial

<220>
<223>  Primer

<400>  10
ttagaattcg gatctatcga atctaaatgt aagttaaaat c                          41


<210>  11
<211>  1056
<212>  DNA
<213>  Artificial

<220>
<223>  Synthetic

<400>  11
atgagatttc cttcaatttt tactgcagtt ttattcgcag catcctccgc attagctgct      60

ccagtcaaca ctacaacaga agatgaaacg gcacaaattc cggctgaagc tgtcatcggt     120

tactcagatt tagaagggga tttcgatgtt gctgttttgc cattttccaa cagcacaaat     180

aacgggttat tgtttataaa tactactatt gccagcattg ctgctaaaga agaaggggta     240

tctctcgaga aaagagaggc tgaagcttac gtagaattcg ctcaggttca attgcaagaa     300

tccggtccag gtttggttaa gccatctgag actttgtcct tgacttgtac tgtttccggt     360

ggttccattt cctcttacca ctggtcctgg attagacagc caccaggtaa aggtttggag     420

tggatcggtt acatctacta ctccggttcc actaactaca acccatcctt gaagaacaga     480

gttactatct ccgttgacac ttccaagaac cagttctcct tgaacttgag atccgttact     540

gctgctgaca ctgctgttta ctactgtgct agaggtactt acggtccagc tggtgatgct     600

ttcgatattt ggggtcaggg tactactgtt acagtttcct ctggtggtgg tggatcaggt     660

ggtggtggtt ctggtggtgg tggatctgat attcaaatga ctcagtcccc atccactttg     720

tccgcttcta ttggtgatag agttacaatt acttgtagag cttccgaggg tatctaccat     780

tggttggctt ggtatcaaca gaagccaggt aaggctccaa agttgttgat ctacaaggct     840

tcctctttgg cttctggtgc tccatctaga ttttctggtt ccggttctgg tactgacttt     900

actttgacta tctcctcctt gcaacctgac gacttcgcta cttactattg tcagcagtac     960

tccaactacc cattgacttt cggtggtggt actaagttgg agatcaagag agctgctgct    1020

ggtggtggtg gttcacatca tcatcatcat cactag                              1056

<210>  12
<211>  35
<212>  DNA
<213>  Artificial

<220>
<223>  Primer

<400>  12
ttaagatcta tgagatttcc ttcaattttt actgc                                35


<210>  13
<211>  36
<212>  DNA
<213>  Artificial

<220>
<223>  Primer

<400>  13
ttaagatctc tagtgatgat gatgatgatg tgaacc                               36


<210>  14
<211>  30
<212>  DNA
<213>  Artificial

<220>
<223>  Synthetic

<400>  14
aagcttggat ccactagttc tagagaattc                                      30


<210>  15
<211>  487
<212>  DNA
<213>  Artificial

<220>
<223>  Synthetic

<400>  15
ttttttgtag aaatgtcttg gtgtcctcgt ccaatcaggt agccatctct gaaatatctg      60

gctccgttgc aactccgaac gacctgctgg caacgtaaaa ttctccgggg taaaacttaa     120

atgtggagta atggaaccag aaacgtctct tcccttctct ctccttccac cgcccgttac     180

cgtccctagg aaattttact ctgctggaga gcttcttcta cggccccctt gcagcaatgc     240

tcttcccagc attacgttgc gggtaaaacg gaggtcgtgt acccgaccta gcagcccagg     300

gatggaaaag tcccggccgt cgctggcaat aatagcgggc ggacgcatgt catgagatta     360

ttggaaacca ccagaatcga atataaaagg cgaacacctt tcccaatttt ggtttctcct     420

gacccaaaga ctttaaattt aatttatttg tccctatttc aatcaattga acaactatca     480

aaacaca                                                               487

<210>  16
<211>  30
<212>  DNA
<213>  Artificial

<220>
<223>  Primer

<400>  16
ttaggatcct tttttgtaga aatgtcttgg                                        30


<210>  17
<211>  37
<212>  DNA
<213>  Artificial

<220>
<223>  Primer

<400>  17
ttaggatcct gtgttttgat agttgttcaa ttgattg                                37


<210>  18
<211>  855
<212>  DNA
<213>  Artificial

<220>
<223>  Synthetic

<400>  18
cccacacacc atagcttcaa aatgtttcta ctcctttttt actcttccag attttctcgg      60

actccgcgca tcgccgtacc acttcaaaac acccaagcac agcatactaa attttccctc     120

tttcttcctc tagggtgtcg ttaattaccc gtactaaagg tttggaaaag aaaaaagaga     180

ccgcctcgtt tctttttctt cgtcgaaaaa ggcaataaaa atttttatca cgtttctttt     240

tcttgaaatt ttttttttta gtttttttct ctttcagtga cctccattga tatttaagtt     300

aataaacggt cttcaatttc tcaagtttca gtttcatttt tcttgttcta ttacaacttt     360

ttttacttct tgttcattag aaagaaagca tagcaatcta atctaagggg cggtgttgac     420

aattaatcat cggcatagta tatcggcata gtataatacg acaaggtgag gaactaaacc     480

atggccaagt tgaccagtgc cgttccggtg ctcaccgcgc gcgacgtcgc cggagcggtc     540

gagttctgga ccgaccggct cgggttctcc cgggacttcg tggaggacga cttcgccggt     600

gtggtccggg acgacgtgac cctgttcatc agcgcggtcc aggaccaggt ggtgccggac     660

aacaccctgg cctgggtgtg ggtgcgcggc ctggacgagc tgtacgccga gtggtcggag     720

gtcgtgtcca cgaacttccg ggacgcctcc gggccggcca tgaccgagat cggcgagcag     780

ccgtgggggc gggagttcgc cctgcgcgac ccggccggca actgcgtgca cttcgtggcc     840

gaggagcagg actga                                                    855

<210>  19
<211>  33
<212>  DNA
<213>  Artificial

<220>
<223>  Primer

<400>  19
ttagaattcc ccacacacca tagcttcaaa atg                                 33

<210>  20
<211>  31
<212>  DNA
<213>  Artificial

<220>
<223>  Primer

<400>  20
ttagaattct cagtcctgct cctcggccac g                                   31

<210>  21
<211>  600
<212>  DNA
<213>  Artificial

<220>
<223>  Synthetic

<400>  21
   gacaataaga agaaaaaaaa agaaaagcgg tgggggaggg attattaaat aaggattatg      60
   taaccccagg gtaccgttct atacatattt aaggattatt taggacaatc gatgaaatcg     120
   gcatcaaact ggatgggagt atagtgtccg gataatcgga taaatcatct tgcgaggagc     180
   cgcttggttg gttggtgaga ggagtgaaat atgtgtctcc tcacccaaga atcgcgatat     240
   cagcaccctg tgggggacac tattggcctc cctcccaaac cttcgatgtg gtagtgcttt     300
   attatattga ttacattgat tacatagcta aaccctgcct ggttgcaagt tgagctccga     360
   attccaatat tagtaaaatg cctgcaagat aacctcggta tggcgtccga ccccgcttaa     420
   ttattttaac tcctttccaa cgaggacttc gtaatttttg attagggagt tgagaaacgg     480
   ggggtcttga tacctcctcg atttcagatc ccaccccctc tcagtcccaa gtgggacccc     540
   cctcggccgt gaaatgcgcg cactttagtt tttttcgcat gtaaacgccg gtgtccgtca     600

<210>  22
<211>  39
<212>  DNA
<213>  Artificial

<220>

<223>  Primer

<400>  22
ttatctagag acaataagaa gaaaaaaaaa gaaaagcgg                              39


<210>  23
<211>  29
<212>  DNA
<213>  Artificial

<220>
<223>  Primer

<400>  23
ttatctagat gacggacacc ggcgtttac                                        29


<210>  24
<211>  1482
<212>  DNA
<213>  Komagataella pastoris

<400>  24
atgcttgctg agattgggga ctatttcagg acaaatagta gcaatggatt cagttctagc      60

tgctataaca aagagaagct ttcaggagac ctcttggagt ccgattttgt gaattcatca     120

ttcgatgaca caacactcca ctctatgcta tcttcttcaa taaatgctga tccggagcaa     180

tcccagaaac cacaacaaag acacaagaat caatccagtt gccatcagga actcaagatt     240

gaaacagaag atcagcaaga accgtatcta ttgcctgata tgcttaccaa aaacatattc     300

aactcacagt cttctatgtt tgtctactcc agcatcccac aaataagctc agtgcaatcg     360

tttatagaca caaacacctt ttccgctgtt gatggtggtg ccgccccgga aggaaattca     420

ttgtctgtgg actcaacttt aggtctatct tctgaccaat catttgagaa gctatactct     480

tacgaccaaa actttgagaa agaaccacaa tcgccagcat gtcccttagt aaaatgtgac     540

ccagtaagca tcatgaaaac atccacaaac tcattggaga cagctgtcaa caccaaaaga     600

cgaatgtttc agcttagaaa ccaaaatgaa attgaaagtc tcaatgtttc tagtgacatc     660

gttgttgcga catcaaaatt tctcaatcat tcatggatcc cgctgatccg tggacgtaat     720

ttcggaggaa gcaactgcaa accgcccaag aaaccattat ttcctggagc gcgctatgtt     780

actgcccgtc tacgattgga atacagtagc tgtaaagata tgtgtctacc ggaatggaac     840

gagtatgaac tcgaagacag tagaagaatc attcgtattg agagactgtt cgactccaat     900

gaaatcgtag cctccttttc cattgtcggg tctgctgtag agaacccgga aactaggcct     960

gtattcaatc ctaatgtcaa agtgttggaa gtttcgtgtc taagatgtct taccaacaac    1020

aacgagtctg acgaggagaa ctgtgtcaac aaagatttgg atggccgtaa ccgcgatatg    1080

gttaaggact catttggatg caagtactat atcaccagtg ttgaagtcat aaagattata    1140

gaattactgg tcggaagctc ttcaatctca gatcctcatc aaatgagaaa agaaagaggg    1200

cgtgtgagat ccaacttggc tcagttctgg tccaaacgcc tagtttcttc gtccaggaaa 1260

accatgaaac aaggtttctt gccaacatgc aacgatgatt attttgcaga gctggcccat 1320

cgtattaaca catacgacgt cagaaaacca cgattatttg acaagtgtat aaagattttg 1380

gaatggtcta aacttaagcc agctctgcag cgggctatgc aaagttacta catggttcaa 1440

ctggatgaat ctagcaacaa aattgcaaca gcaaacaatt ag 1482

<210> 25
<211> 55
<212> DNA
<213> Artificial

<220>
<223> Primer

<400> 25
caatcaattg aacaactatc aaaacacaat gcttgctgag attggggact atttc 55

<210> 26
<211> 59
<212> DNA
<213> Artificial

<220>
<223> Primer

<400> 26
ccgcttttct ttttttttct tcttattgtc ctaattgttt gctgttgcaa ttttgttgc 59

<210> 27
<211> 1557
<212> DNA
<213> Komagataella pastoris

<400> 27
atgaattcat cttcaactcg tggcctgcaa gctatatcac tcacaccaac tagagtttta 60

ttcactttga gcactctgtt cgccgatacg tcaaaaatgt ctgctgagaa tggggactat 120

ttcaggacaa acagtagcaa tgaattcagc aataactgct ttaacaaaga aaagctctcg 180

gaggacctct tggaggccag ttttgtcgat tccccaatcg aagacacaat accccacttt 240

atgctatctt cttcaataaa tgctgattcg gaacagcctc aggaaccaca gcatcgacac 300

aagaatcaat ccagttgcca tcgggaactc aagatcgaaa caaacgatca gcaagaaccg 360

tatctgttgc ctgatatgct taccaaaaac atattcaact cacagtcttc tatgtttgtc 420

tattccagca tcccacaaac aagctcagtg caatcgttta tagacacaaa cattttttcc 480

gctgttgatg gtggtgccgc cccagaagaa aattcattgt ctgtggactc aaccttaggt 540

ccatcttctg accaatcatt tgaaaagcta tactcttacg accaaaattt tgagaaagaa 600

ccacaatcgc cagcatgtcc cttagtaaaa tgtgacctag taggcatcat gaaagcatcc 660

acaaactcat tggaggtagc tgtcaacacc aaaagacaaa tgtttcagct tagacaccaa 720

aatgaaattg agagtctcta tgtttctagt gacatcgtta ttgcgacatc aaattttctt 780

gaccattcat ggattccgct gatccgtgga cgtaatttcg gaggaagcaa ctgcaaaccg 840

cccaagaaac cattatttcc tggagcgcgc tatgttactg cccgtctacg attggaatac 900

agtagctgtg aagatatgtg tttaccggaa tggaacgagt atgaactcaa agacagtaga 960

agaatcattc gtcttgagag gcggttcgac tccaatgaaa tcgtggcctc cttttccatt 1020

gtcgggtctg ctgtagagaa ccctgaaact aggcctgcat tcaatcctaa cgtcaaagtg 1080

ttggaagttt cgtgtctaag gtgtcttacc aacaacaacg aatctgacga ggagaactgt 1140

gtcaacaaag atttggatgg ccgtgtccac aatatggtta aagactcaat tggatgcaag 1200

tactatatta ccagtgttga agtcataaag attatagaat tactggtcgg aagctcttca 1260

atttcagatc cccaacaaat gagaaaagaa agaggccgtg tgagatcaaa cttggctcag 1320

ttctggtcca aacatttagt ttcttcgtcc aggaaaacca ggaaacaacg tttcttgcca 1380

acatgcaacg atgattattt cgcagagctg gtccatcgta tcaacactta tgacgtgaga 1440

aaacctcgac tattcgataa gagcgtaaag attctggaat ggcctaatct tggaccagct 1500

ttgcaacggg ctatccagag ttactacatg gttcgactgg aagaatctag caactaa 1557

<210> 28
<211> 48
<212> DNA
<213> Artificial

<220>
<223> Primer

<400> 28
caatcaattg aacaactatc aaaacacaat gaattcatct tcaactcg 48

<210> 29
<211> 65
<212> DNA
<213> Artificial

<220>
<223> Primer

<400> 29
ccgcttttct ttttttttct tcttattgtc ccatttgctg ctctacccct gtcctaaact 60

tattg 65

<210> 30
<211> 1572
<212> DNA
<213> Komagataella pastoris

<400>  30
atgcggttgt atttagcaac tgtaggaact ccattcatcg gtgcaccgcc agtgattgct      60

agtaatgaag accatagcac tgctcacaat aacaatagca acgactttaa ttatgaatgc     120

tataataaag aaaagctctc tggggatctc ttggaagctg acctttttga tcctccaatt     180

gatgatacaa tgcactgctc catgttttcg tcatcaacaa ctgctgacac tgagcagcta     240

caaaaaccaa agcatgaatc gcaacacaag aatcccttcg attgttgtca ggaactcaag     300

cttaaaatgg ccgatcagca agagttgcat ttgttgccgg agaaaattac caagaatatt     360

tttaattcag agtcgcctgt gttcatatgc accaagattc cccaaacaag tccggtccag     420

tcgttcgtag atgttaatgc ttctttactt gccactaact ccgccctgac tacaaattca     480

ttctctgtag acacaacagt tgatatattg tttgagcaaa cagttcaaga tccatactct     540

taccgtttaa actctaagga aacagcacct ccatccacag ctagcttctt tgggactctc     600

gatctgggac ccaccgtgga agcatccaca acgttactgg aggcagccgt caactctaaa     660

agacgaatag ttgaacttag acagcaaaac agaatcgagg atctcaattt ttcaacatca     720

atgttccttg accattcacg cataccgttg gtgcgtgggc gtagctttgg aggaaatagc     780

agcagacctc ccaagacgcc attaattcct gggacgcggt acgttactgc ccgtttaaga     840

ctggaaaaca gcacaagtga ggatatatgt ttgccccatt ggaacaaagc ggaactcaaa     900

gactctagaa gaataatccg tattgaaaga agataccagt ccaatgagat tgtagcaacc     960

ttttccatag ttggatcggc catagagcac ccaaagacta agcctgcatt cgatcctgac    1020

gtcaaggttt tggaagtctc gtgtctaaaa tgtcccacca acgaaaatga acctaacgag    1080

gagcgatttg tcagtgaatg tacgtcagat gaagcacgag cacaacatgc cttaacagaa    1140

cccctaatgt caggcttcaa cagagaaccg aatggccgta accctaaaac ggctgggaat    1200

tcaattggat gcaggtacta tattaccagt gtcgaagtca tcaagataat agaactactg    1260

attggaagct attcaacctc agaccccgtg caaaggagaa aagagagagg ccgtatacga    1320

tctaacttgg tgcagttctg gtccaaacac ctagtctttt cctcgaagaa ctctatgaag    1380

cgccgttccg tgccaacagg caacgatgct tatcttacgg agctggagca tcgtattaac    1440

acttatgatg tgagaaaacc tcgactattt gataagagtg tcaagattct ggaatggtct    1500

aaacttggac ctgctttgcg tcgggctatc caaagctatt acatggtacg actagacgaa    1560

tccagcaact aa                                                        1572

<210>  31
<211>  54
<212>  DNA
<213>  Artificial

<220>
<223>  Primer

<400>  31
caatcaattg aacaactatc aaaacacaat gcggttgtat ttagcaactg tagg          54


<210>  32
<211>  54
<212>  DNA
<213>  Artificial

<220>
<223>  Primer

<400>  32
ccgcttttct ttttttttct tcttattgtc ttagttgctg gattcgtcta gtcg          54


<210>  33
<211>  1599
<212>  DNA
<213>  Komagataella pastoris

<400>  33
atgcggttgt ctttagcaac acgagaaaca ccgtctaccg gtacatcgcc ggtgtttgct      60

ggtgacgaag atcatagcag tgccaacaat agcagtagca acactttcaa ctatgactac     120

tatataaaag aagaactctc tggaaatctc ctggaaacag ccttttttga atctccgatt     180

aatgatacaa tgctctgctc catgttatcg tatccaaaaa ttgctggtac tgagaaaata     240

caagaaccag accatgaact gcagcaacac aggaatccct tcggttgctg ccaggaactc     300

aaagctgaat tggccgaaca gcaagagctg tatctgctgc caaataaact taccaagaat     360

atttttaatt cagggtctcc tgtgttcata tacaccaaga tcccacaaac aagctcggtt     420

cagtcatttg tggatgttag caattcttta tttgccactg actccgcctt gcctacaagt     480

tcatttcgtg tagactcaac agtcgacata tcgtttgagc actcaattca agattcatac     540

tgttaccgtt caaactctgc agaaatagca cctccatcca cagcactctc ttcagggact     600

cacgacctgg tatccaccgt ggaagcatcc acgacgttac tggaggcagc cgtcaactct     660

aaaagacgaa tgcttgaact tagacagcaa aacagaatcg aggatctcag tttttcaaca     720

tcaatattcc ttgaccattc acgcctaccg ttggttcgtg ggcgtagctt tggaggaaat     780

agcagtagac ctcccaagac gccattgatt cctgggacgc ggtacgttac tgcccgttta     840

agactggaaa acagcacaag tgaggatatg tgtttgcccc agtggaacaa ggcggaactc     900

aaagactcta gaagaataat ccgtattgaa agaagatacc agtctgatga aattgtcgct     960

tccttttcca tagttggatc ggccatagag tacccaaaga ctaagcctgc attcgatcct    1020

gacgtcaagg ttttggaagt ctcgtgtcta aaatgtccca ccaacgaaaa tgaacctaac    1080

gaggagcgat ttgtcagtga atgtacgtca gatgaagcac gaacacaaca tgccttaaca    1140

gaacccctaa cgtcaggctt caacagagaa ccgaatggcc ataaccctaa aacggctggg    1200

aattcaattg gatgcaggta ctatattacc agtgtcgaag tcatcaagat aatagaacta 1260

ctgattggaa gctattcaac ctcagacccc gtgcaaagga gaaaagagag aggccgtata 1320

cgatctaact tggtgcagtt ctggtccaaa cacctagtct tttcctcgaa gaactctatg 1380

aagcgccgtt ccgtgccaac aggcaacgat gcttatctta cggagctgga gcatcgtatt 1440

aacacttatg acgtgagaaa acctcgacta tttgacaaga gtgtcaagat tctggaatgg 1500

tccaaacttg ggccagcttt gcagcgggct atccaaagct attacatggt tcggctagat 1560

aattccagcg acataactgc aacaacaaat agcagctaa 1599

<210> 34
<211> 51
<212> DNA
<213> Artificial

<220>
<223> Primer

<400> 34
caatcaattg aacaactatc aaaacacaat gcggttgtct ttagcaacac g 51

<210> 35
<211> 53
<212> DNA
<213> Artificial

<220>
<223> Primer

<400> 35
ccgcttttct ttttttttct tcttattgtc ttagctgcta tttgttgttg cag 53

<210> 36
<211> 1524
<212> DNA
<213> Komagataella pastoris

<400> 36
atgcagctac tgtcagcaag ttcaagcact ccattcgtcg ctatgtcgac agtaaacgct 60

gggaacgagt gttatcttag gaccaacaat agtaacagca acggtatcag cgataaccgc 120

ttcagaaaaa aaaagctttc tgaggatttg ttgaaagccg attttgtcga agacacagcc 180

ggtaacccaa tgatttaccc catggtttta tctgctccaa catatataaa aactgaacat 240

ctccaagaat tggagcataa acagcaagga tcaaatctgt tgtctgaaat gtttaccaaa 300

aatattttca attcacattc tgcaatgttt gtttactctg aaatccctca aataagttcg 360

gttcagtcat tcattggttc tgacacctct gtccttgttg ctgatgcctc ttcagaaaaa 420

aattcattgt ttgtgtactc aaatactggt atatcttttg atccgtcaga tgaggatcca 480

tgcccttacg aggctaattt tgatccagga caactattct cagcggtatc cgcagtagaa 540

gacggttcta tggcaacatt gacagctccc actatgtcat tagaaggtgt cgttaattcc 600

aaaaaaggaa tatgtcaact tagacagcat aacagaatcg agggatttga ttcttcccat 660

gaattcaaag tcttgggatc aatgttcctt aaccattcgt acgtaccgct ggttcgtgga 720

cgtagtttcg gaggaaacaa ttgtagacct cccaaagagc cattaattcc tggaacccgg 780

tacgttgctg cccatctaag attggaaaac aatacctgcg aggatatgtg tctcccagaa 840

tggaacaagc atgagcttga agatagtaga agaatcattc gtatagagag aagataccag 900

tcgaatgaaa ttgtggcctc tttttctata gttgggtctg ctaaagagaa tccacaaaca 960

aagccttcaa gcgatcctga cgtgaaagtt ttggaagtct cgtgtctgaa atgtttcatc 1020

aacggcaacg aatctgacga gcaatttgtg actgaatgtc agtcagatga agcgcattat 1080

ggcttcacag agttaccaaa acaaaacttt agcagaaaat caaaaggctg cagccacaaa 1140

atggcaaata acacgattgc atgccaatac tatattacca gcgtcgaagt catcaagatt 1200

attgaattac tggtcggagg ctatacagtt tctgatcctc ggcagaggag gaaagaaaga 1260

ggccgcgtga gatcaaactt ggctcaattc tggtccaaac acttagtttc ttcttccaag 1320

aaaacgatga agcgccctgc gatgctagca tgcaacgagg attatcttgc agagctggaa 1380

cagcgtatca acacctatga cgtgagaaag ccgcgactat ttgataagag cgttaagatt 1440

ctcgaatggc ctaatcttgg accagctttg cagcgggcta ttcagagcta ttacatggtg 1500

caactggaag aatccagcaa ctag 1524

<210> 37
<211> 55
<212> DNA
<213> Artificial

<220>
<223> Primer

<400> 37
caatcaattg aacaactatc aaaacacaat gcagctactg tcagcaagtt caagc 55

<210> 38
<211> 55
<212> DNA
<213> Artificial

<220>
<223> Primer

<400> 38
ccgcttttct ttttttttct tcttattgtc ctagttgctg gattcttcca gttgc 55

<210> 39
<211> 1614
<212> DNA
<213> Komagataella pastoris

<400>  39
atgcaattgt ttttagcaac tctaggaatt ccgttcaccg gcacattacc agccattgct      60

ggtaacgagg atcatagcag tgccaacaac aacaatggca acggtttcag ttatgactgg     120

catggaaatg atacgctttt tgcggaactc ttggaaactg acttttttga tcatccgatt     180

gatgacgcaa tgctctgctc cttgttatcc cctccaagag ctgctgagac tgagcaacag     240

caagatacag aatgggagca acaaaaacac acaaatctct ttggttgctg ccagaaacac     300

aaagttgaaa tggccaaaca gcaagaactg tatttgctgc cagataagct taccaagaat     360

attttcaatt cagagtcgcc tgtgttcata tgcaccaaga tcccacaaac aagttcggtt     420

cagtcgttcg tagatgttga tgcttctttg cttgccactg actcctccct aagtaaagat     480

tcattctctg cagactcaac agttgatgta ttgtttgagc agtcagttca agagccatac     540

ttttaccgtt caaactctgc ggaaacagca cctccatcca cagcatgctc ctcagcgact     600

ctcgatctgg tatccacttt ggaagcaacc acaacgttat ttgaggaagc cgccaactcc     660

aaaaaacaaa tgattgggct taaacagcaa tccaagatcg aagatctcag tttttcctgc     720

aaactaaatt tctcagcatc aatgttcctt gaccattcat gcataccgct ggtgcgtggg     780

cgtagctttg gaggaaatag cagacctccc aagacgccat taattcctgg gacgcggtac     840

gttactgccc gtctaagact ggagaacagt acaagtgaag atatgtgttt tccccactgg     900

agcgagcggg aacttagcga ctcaagaaga atcattcgta ttgagagaag ttaccagtcc     960

aatgaaattg tagcatcttt tttcatagtt ggatcggcca tagatcaccc ggagactaaa    1020

ccttcatctg aattagacgt gaaagtttta gaagtttcgt gtctaagatg ttttatcaac    1080

gacaatgaat ctgaggagga gcgatttgtc agtgaatata cctcagatga agcacgagca    1140

caacatgcct taacagaacc gctaacgtca ggcttgaaca aagaaccgaa tggccataac    1200

cctaaaacgg ctgggaattc aattggatgc aggtactata taaccagtgt tgaagtcatc    1260

aagataatag aactactgat tggaagctat tcaacctcag accctgtgca aaggagaaaa    1320

gaaagaggcc gtatgagatc gaacttggtg cagttctggt ccaaacacct agtgtcttcc    1380

tccaaggaca ctatgaagcg ccgttccgtg ccaacatgca acgatgatta tctttcggag    1440

ctggaacatc gtattaacac atatgacgtg agaaaacctc gaaaatttga caagagcgtt    1500

aagattctgg aatggtccaa acttgggcca gctttgcagc gggctatcca aagctattac    1560

atggtccgat tagataattc cagcaataaa actgcagcaa caaatagcag ctaa          1614

<210>  40
<211>  54
<212>  DNA
<213>  Artificial

<220>

<223>  Primer

<400>  40
caatcaattg aacaactatc aaaacacaat gcaattgttt ttagcaactc tagg          54


<210>  41
<211>  53
<212>  DNA
<213>  Artificial

<220>
<223>  Primer

<400>  41
ccgcttttct ttttttttct tcttattgtc ttagctgcta tttgttgctg cag          53


<210>  42
<211>  1596
<212>  DNA
<213>  Komagataella pastoris

<400>  42
atgcagtttt acttagctac tcctaccact cctttcgttg ataacttgac aataccctct          60

ggtagcgggg cttatagcaa atccaacggc aacaatagca gcagcttcgg tcatagttac          120

aacggaaaag aagagctcac tgatgacctt ctgactgcag atttcgtcga cccaccaatc          180

gacgacgcaa tattgtactc gatgctatct cctttcatga atgctactac cgaacaacca          240

caagaattcc agaatgaaca gcagcatcag aacctttccg gttgctacca ggagctgctg          300

gttcaaacag aggaacaaca acaactggat ttgttgccta atatgcaccc cgagaatatt          360

ttcaattctg agtcttctat gttcgtgtac tccagtatcc cgcaaagaaa ttctgttcac          420

tcgctgatca gtgtcaacac ctcttctctt gcccccgatt ctgctgaggg gggaggctta          480

tcgtttgtgg acaccatagg cccatcattt gaccattctg ttgaaacctc actctcttac          540

ggaatgaact ctacggactc aactctaccg tcctcagcac gctcctcggt aggacttgac          600

ctactaccca ccgtggcagc tcctgcgtca tccgtttcct cattggaaac ggtcatcaac          660

tccaagagac aaaagctgca attcagacag cacaacagga ttgaagaact caattctcca          720

cacggtatca aagcacaggc accggtgttg cttaactact caagtcttcc gctggttcgt          780

ggacttagct ctggtgggaa caacagcaga cctcccaaag agccattaat tcctggagca          840

cagtacatta ctgcccttct gagactagag aacagctcct gcgaggactt atgtctacca          900

gaatggaacg aggacgaact gaaggactgc agaaggatca ttcgcataga gagaaaacgc          960

cactccaacg agatagtggc gtcattttct atagtcggtt ctgccacgca gaacccacaa          1020

atcaagccag catccgactc tgacgtgcaa gtgttggaag tctcatgttt acaatgcctc          1080

gtcaaccata acgaaactga cgaggggaaa caaggaagct ccccaaacga aatacagcat          1140

agctctgcag agccaccgaa atcgaacctc aacaggacat tgagtagtag tagccacaga          1200

gcagctggga gctccgtggg ataccagtac tacatcacca gtgtggaagt catcaagatt 1260

atagaactgc tagtcggaag ccatttaatc gaagactcgc agcaaaagag aaaagaaaga 1320

ggccgcgtga gatcaaactt ggtgcccttc tggtcaaaac atcccgtgtc ctcgtctaag 1380

aagaccttga ggccccgttc tggaccagct tgcaacgatg actaccttgc agagttggcc 1440

catcgtatca tgacatacga gatacgcagg ccgcgaatat tcgacaagaa cgtacgaatt 1500

ctggaatggg ccaagcttgc cccggctttg cagcgggctc ttcaaagcta ctatgtggcg 1560

gtgcccctag atgaatccga gaataaactt gtataa 1596

<210> 43
<211> 57
<212> DNA
<213> Artificial

<220>
<223> Primer

<400> 43
caatcaattg aacaactatc aaaacacaat gcagttttac ttagctactc ctaccac 57

<210> 44
<211> 57
<212> DNA
<213> Artificial

<220>
<223> Primer

<400> 44
ccgcttttct ttttttttct tcttattgtc ttatacaagt ttattctcgg attcatc 57

<210> 45
<211> 1353
<212> DNA
<213> Komagataella pastoris

<400> 45
atgttaacag caggttcagt tcagcggcta caaatatcta tgtttcaaac acaggaacag 60

gagcaggtgg acctttcgtc ctctatacat accgagaaca ttttcaattc agaatcgtct 120

ggtcccgcat atccgaacat cccacaaatt agttctgttc actcgtttat aggtgccaaa 180

agctctcttc ttgctgatgc tgctccgaat caatgtttat catttgtgga ctcagcaaaa 240

gttacatcgt ttgaccaatc attccatggt tcaccatctt ataagctaaa ttctgcaaaa 300

ctagcagttt catcgtctga gtacttgtca gtagaatcat ccacagaggt agactccaca 360

atttcattgg aagcacaagt tgaccgcaaa agacaaaaga ttcaacccga acgacacagc 420

aaaaccgaca atctgaacct cttttctgac ttcgatctat tggcaccagc gtttcttacc 480

tactacttgc cattgtttcg tggatgtagc tctggaggca acaacaaacc cccgaaaaag 540

cgatcaatta ctggaacaca gtacgttgcc gccagtttga gactggaaag cagtaccatt 600

gaggacattt gcttaccgaa atggaacgag aacgagctca aggacaatag aagaatcatc 660

cgcatagaga gaagacaaca gggaaatgaa attgcagctt ccttttcaat agttgggtct 720

gctttagaga acccacacac caaacctaca ctggatcctg acgtggaagt attagaagtc 780

tcgtgcttac ggtgtctagt caattacgac gagattgatg acaaacaaac agtgcatgga 840

agccttttac acgaagttca gtctgaattc accgaaatac ggaaacaaga atctaccggg 900

aaacgtagca gttatgcctg tacgtcgaat gggacctcca tcgaatgcca gtactacgtt 960

actagtgtcg aagtcatcaa gataattgag ttactggtgg gaagccattt aatccaagat 1020

cgcaagcaga gaagaaaaga aaggggctgc ataaggtcaa atttgatgcc cttctggtcc 1080

aaacatctca tctcgtcaac cacaaacacc atgaagcgcc gttcagtgac agcatctaac 1140

gagaattata ttgcagagct ggcccatcgt atcaagacat atgaagttcg gagacctcga 1200

aaggtcaata aggatctcag aattttggaa tggtcaaagc ttggaccagc tctgcggcgg 1260

gctctccaca gctattacgt agcagtgcca attgataaat tcaaaaatat agccaagccg 1320

acaaacagtg agctaatgtt taatatccaa taa 1353

<210> 46
<211> 50
<212> DNA
<213> Artificial

<220>
<223> Primer

<400> 46
caatcaattg aacaactatc aaaacacaat gttaacagca ggttcagttc 50

<210> 47
<211> 62
<212> DNA
<213> Artificial

<220>
<223> Primer

<400> 47
ccgcttttct ttttttttct tcttattgtc ttattggata ttaaacatta gctcactgtt 60

tg 62

<210> 48
<211> 1245
<212> DNA
<213> Komagataella pastoris

<400> 48
atgtttcaac cactcgagca agaacaacaa ctggatttgt tgtttgccat gcctccggag 60

aacattttta actctgactc ttctgcactt accttttcca atacttttca aaccagttca 120

gttcactcgg tcacaggtgt tgacagccct tcacttgtta atattaatat cccaaaatca 180

gaatcgttct ttatggactc aatgacgggt gcatcttttg gacaaacttt tcaagaccta 240

ttcccagcgt ttcctttgct aaaacttcct accatggtag cacctccaac ttcgttgaag 300

atggtcatgg accgtaaacg gcgacagtcc caaataacac agcagaatac cactgaaaat 360

ctcaattcct ttcatggcat gaatatgccg gtaccagcgt tccttgaata ctcggccttc 420

ccgctggttc gtggcttttg ctctggagga aaaataaagc cgcctaagaa ccaactagtt 480

cctggaactg agtacgtcaa tgtctgcttg gagttgctca acgtcaacta tgaaggcatt 540

tgtttaccta aatggaatga gaaagaactt gaggaaagta gaaggattgt tcgagtagag 600

aaaagacgtc agtataataa aattgaggct gccttttcaa tagttgattc tgttcaagaa 660

aatccacaaa ccaatattgc agttgaccct aacgttgatg tgctggagtt ttcgtgttta 720

ttgtgtccaa tcaaagaaca caactcagaa gtagcgcaaa ccgtccctac aagctcttca 780

agtgaagaac acaagtacag cgagctagca ggacacgact ttgaagaaaa tttgagctgt 840

ggtagtgata aagaaattga gacttctgtt agatttgaat actacatcac cagtgttgaa 900

gtcattagga ttatagagtt tttaacggaa aatcatctaa tccaagatat ccaggcgaaa 960

agaagagaaa gaagctgtct aagatcaaat ttagtgcgat tctggtccaa agatatagtt 1020

ccatcgtcca caagtaccat gagcaagggt tctaagccat ctcgggaaaa cgattgtatt 1080

gaagagttgg cccaacgtat cagggcttat aaggtgcgga aacctctaat agtcgataaa 1140

agcctaagaa ttctggaatg gtccaaactt ggaccagctt tggagcgggc tcttcaaagt 1200

tattacgtag cgttaccatt ggataaaatt aaaacaaagt tatga 1245

<210> 49
<211> 56
<212> DNA
<213> Artificial

<220>
<223> Primer

<400> 49
caatcaattg aacaactatc aaaacacaat gtttcaacca ctcgagcaag aacaac 56

<210> 50
<211> 70
<212> DNA
<213> Artificial

<220>
<223> Primer

<400> 50

ccgcttttct ttttttttct tcttattgtc tcataacttt gttttaattt tatccaatgg 60

taacgctacg 70

<210> 51
<211> 1596
<212> DNA
<213> Komagataella pastoris

<400> 51
atgcggttgt atttagcaac tgtaggaact ccattcatcg gtgcaccgcc agtgattgct 60

agtaatgaag accatagcac tgctcacaat aacaatagca acgactttaa ttatgaatgc 120

tataataaag aaaagctctc tggggatctc ttggaagctg acctttttga tcctccaatt 180

gatgatacaa tgcactgctc catgttttcg tcatcaacaa ctgctgacac tgagcagcta 240

caaaaaccaa agcatgaatc gcaacacaag aatcccttcg attgttgtca ggaactcaag 300

cttaaaatgg ccgatcagca agagttgcat ttgttgccgg agaaaattac caagaatatt 360

tttaattcag agtcgcctgt gttcatatgc accaagattc cccaaacaag tccggtccag 420

tcgttcgtag atgttaatgc ttctttactt gccactaact ccgccctgac tacaaattca 480

ttctctgtag acacaacagt tgatatattg tttgagcaaa cagttcaaga tccatactct 540

taccgtttaa actctaagga aacagcacct ccatccacag ctagcttctt tgggactctc 600

gatctgggac ccaccgtgga agcatccaca acgttactgg aggcagccgt caactctaaa 660

agacgaatag ttgaacttag acagcaaaac agaatcgagg atctcaattt ttcaacatca 720

atgttccttg accattcacg cataccgttg gtgcgtgggc gtagctttgg aggaaatagc 780

agcagacctc ccaagacgcc attaattcct gggacgcggt acgttactgc ccgtttaaga 840

ctggaaaaca gcacaagtga ggatatatgt ttgccccatt ggaacaaagc ggaactcaaa 900

gactctagaa gaataatccg tattgaaaga agataccagt ccaatgagat tgtagcaacc 960

ttttccatag ttggatcggc catagagcac ccaaagacta agcctgcatt cgatcctgac 1020

gtcaaggttt tggaagtctc gtgtctaaaa tgtcccacca acgaaaatga acctaacgag 1080

gagcgatttg tcagtgaatg tacgtcagat gaagcacgag cacaacatgc cttaacagaa 1140

cccctaatgt caggcttcaa cagagaaccg aatggccgta accctaaaac ggctgggaat 1200

tcaattggat gcaggtacta tattaccagt gtcgaagtca tcaagataat agaactactg 1260

attggaagct attcaacctc agaccccgtg caaaggagaa aagagagagg ccgtatacga 1320

tctaacttgg tgcagttctg gtccaaacac ctagtctttt cctcgaagaa ctctatgaag 1380

cgccgttccg tgccaacagg caacgatgct tatcttacgg agctggagca tcgtattaac 1440

acttatgacg tgagaaaacc tcgactattt gacaagagtg tcaagattct ggaatggtcc 1500

aaacttgggc cagctttgca gcgggctatc caaagctatt acatggttcg gctagataat 1560

tccagcgaca taactgcaac aacaaatagc agctaa 1596

<210> 52
<211> 54
<212> DNA
<213> Artificial

<220>
<223> Primer

<400> 52
caatcaattg aacaactatc aaaacacaat gcggttgtat ttagcaactg tagg 54

<210> 53
<211> 53
<212> DNA
<213> Artificial

<220>
<223> Primer

<400> 53
ccgcttttct ttttttttct tcttattgtc ttagctgcta tttgttgttg cag 53

<210> 54
<211> 1542
<212> DNA
<213> Komagataella pastoris

<400> 54
   atgcagtttt acttagctac tcctaccact cctttcgttg ataacttgac aataccctct 60
   ggtagcgggg cttatagcaa atccaacggc aacaatagca gcagcttcgg tcatagttac 120
   aacggaaaag aagagctcac tgatgacctt ctgactgcag atttcgtcga cccaccaatc 180
   gacgacgcaa tattgtactc gatgctatct cctttcatga atgctactac cgaacaacca 240
   caagaattcc agaatgaaca gcagcatcag aacctttccg gttgctacca ggagctgctg 300
   gttcaaacag aggaacaaca acaactggat ttgttgccta atatgcaccc cgagaatatt 360
   ttcaattctg agtcttctat gttcgtgtac tccagtatcc cgcaaagaaa ttctgttcac 420
   tcgctgatca gtgtcaacac ctcttctctt gcccccgatt ctgctgaggg gggaggctta 480
   tcgtttgtgg acaccatagg cccatcattt gaccattctg ttgaaacctc actctcttac 540
   ggaatgaact ctacggactc aactctaccg tcctcagcac gctcctcggt aggacttgac 600
   ctactaccca ccgtggcagc tcctgcgtca tccgtttcct cattggaaac ggtcatcaac 660
   tccaagagac aaaagctgca attcagacag cacaacagga ttgaagaact caattctcca 720
   cacggtatca aagcacaggc accggtgttg cttaactact caagtcttcc gctggttcgt 780
   ggacttagct ctggtgggaa caacagcaga cctcccaaag agccattaat tcctggagca 840
   cagtacatta ctgcccttct gagactagag aacagctcct gcgaggactt atgtctacca 900
   gaatggaacg aggacgaact gaaggactgc agaaggatca ttcgcataga gagaaaacgc 960
   cactccaacg agatagtggc cgactctgac gtgcaagtgt tggaagtctc atgtttacaa 1020
   tgcctcgtca accataacga aactgacgag gggaaacaag gaagctcccc aaacgaaata 1080
   cagcatagct ctgcagagcc accgaaatcg aacctcaaca ggacattgag tagtagtagc 1140
   cacagagcag ctgggagctc cgtgggatac cagtactaca tcaccagtgt ggaagtcatc 1200
   aagattatag aactgctagt cggaagccat ttaatcgaag actcgcagca aaagagaaaa 1260
   gaaagaggcc gcgtgagatc aaacttggtg cccttctggt caaaacatcc cgtgtcctcg 1320
   tctaagaaga ccttgaggcc ccgttctgga ccagcttgca acgatgacta ccttgcagag 1380
   ttggcccatc gtatcatgac atacgagata cgcaggccgc gaatattcga caagaacgta 1440
   cgaattctgg aatgggccaa gcttgccccg gctttgcagc gggctcttca aagctactat 1500
   gtggcggtgc ccctagatga atccgagaat aaacttgtat aa 1542

<210> 55
<211> 57
<212> DNA
<213> Artificial

<220>
<223> Primer

<400> 55
caatcaattg aacaactatc aaaacacaat gcagttttac ttagctactc ctaccac 57

<210> 56
<211> 57
<212> DNA
<213> Artificial

<220>
<223> Primer

<400> 56
ccgcttttct ttttttttct tcttattgtc ttatacaagt ttattctcgg attcatc 57

<210> 57
<211> 1566
<212> DNA
<213> Komagataella pastoris

<400> 57
atgttaacag caggttcagt tcagcggcta caaatatcta tgtttcaaac acaggaacag 60

gagcaggtgg acctttcgtc ctctatacat accgagaaca ttttcaattc agaatcgtct 120

ggtcccgcat atccgaacat cccacaaatt agttctgttc actcgtttat aggtgccaaa 180

agctctcttc ttgctgatgc tgctccgaat caatgtttat catttgtgga ctcagcaaaa 240

gttacatcgt ttgaccaatc attccatggt tcaccatctt ataagctaaa ttctgcaaaa 300

ctagcagttt catcgtctga gtacttgtca gtagaatcat ccacagaggt agactccaca 360

atttcattgg aagcacaagt tgaccgcaaa agacaaaaga ttcaacccga acgacacagc 420

aaaaccgaca atctgaacct cttttctgac ttcgatctat tggcaccagc gtttcttacc 480

tactacttgc cattgtttcg tggatgtagc tctggaggca acaacaaacc cccgaaaaag 540

cgatcaatta ctggaacaca gtacgttgcc gccagtttga gactggaaag cagtaccatt 600

gaggacattt gcttaccgaa atggaacgag aacgagctca aggacaatag aagaatcatc 660

cgcatagaga gaagacaaca gggaaatgaa attgcagctt ccttttcaat agttgggtct 720

gctttagaga acccacacac caaacctaca ctggatcctg acgtggaagt attagaagtc 780

tcgtgcttac ggtgtctagt caattacgac gagattgatg acaaacaaac agtgcatgga 840

agccttttac acgaagttca gtctgaattc accgaaatac ggaaacaaga atctaccggg 900

aaacgtagca gttatgcctg tacgtcgaat gggacctcca tcgaatgcca gtactacgtt 960

actagtgtcg aagtcatcaa gataattgag ttactggtgg gaagccattt aatccaagat 1020

cgcaagcaga gaagaaaaga aaggggctgc ataaggtcaa atttgatgcc cttctggtcc 1080

aaacatctca tctcgtcaac cacaaacacc atgaagcgcc gttcagtgac agcatctaac 1140

gagaattata ttgcagagct ggcccatcgt atcaagacat atgaagttcg gagacctcga 1200

aaggtcaata aggatctcag aattttggaa tggtcaaagc ttggaccagc tctgcggcgg 1260

gctctccaca gctattacgt agcagtgcca attgataaat tcaaaaatat agccaagccg 1320

acaaacacta gcctaggcac cataaagatg gcaatagctg gtagaagtga ggttttggaa 1380

acagtcttta tagcaagaca aagtccggtt gaaccagatg cttgctcatc gattgctaac 1440

gtcagaaggg aattgtgcga cgcttgcact ggcacacagg ccaatattgt gactgaaaag 1500

attcttctga ctaccatagc cgcggacgcc ggatctacaa ttgtggtgcc agtgtgcgtt 1560

ccttaa 1566

<210> 58
<211> 50
<212> DNA
<213> Artificial

<220>
<223> Primer

<400> 58
caatcaattg aacaactatc aaaacacaat gttaacagca ggttcagttc 50

<210> 59
<211> 52
<212> DNA
<213> Artificial

<220>

<223>  Primer

<400>  59
ccgcttttct ttttttttct tcttattgtc ttaaggaacg cacactggca cc          52


<210>  60
<211>  1575
<212>  DNA
<213>  Komagataella pastoris

<400>  60
atgcggttgt ctttagcaac acgagaaaca ccgtctaccg gtacatcgcc ggtgtttgct      60

ggtgacgaag atcatagcag tgccaacaat agcagtagca acactttcaa ctatgactac     120

tatataaaag aagaactctc tggaaatctc ctggaaacag ccttttttga atctccgatt     180

aatgatacaa tgctctgctc catgttatcg tatccaaaaa ttgctggtac tgagaaaata     240

caagaaccag accatgaact gcagcaacac aggaatccct tcggttgctg ccaggaactc     300

aaagctgaat tggccgaaca gcaagagctg tatctgctgc caaataaact taccaagaat     360

atttttaatt cagggtctcc tgtgttcata tacaccaaga tcccacaaac aagctcggtt     420

cagtcatttg tggatgttag caattcttta tttgccactg actccgcctt gcctacaagt     480

tcatttcgtg tagactcaac agtcgacata tcgtttgagc actcaattca agattcatac     540

tgttaccgtt caaactctgc agaaatagca cctccatcca cagcactctc ttcagggact     600

cacgacctgg tatccaccgt ggaagcatcc acgacgttac tggaggcagc cgtcaactct     660

aaaagacgaa tgcttgaact tagacagcaa aacagaatcg aggatctcag tttttcaaca     720

tcaatattcc ttgaccattc acgcctaccg ttggttcgtg ggcgtagctt tggaggaaat     780

agcagtagac ctcccaagac gccattgatt cctgggacgc ggtacgttac tgcccgttta     840

agactggaaa acagcacaag tgaggatatg tgtttgcccc agtggaacaa ggcggaactc     900

aaagactcta gaagaataat ccgtattgaa agaagatacc agtctgatga aattgtcgct     960

tccttttcca tagttggatc ggccatagag tacccaaaga ctaagcctgc attcgatcct    1020

gacgtcaagg ttttggaagt ctcgtgtcta aaatgtccca ccaacgaaaa tgaacctaac    1080

gaggagcgat ttgtcagtga atgtacgtca gatgaagcac gaacacaaca tgccttaaca    1140

gaacccctaa cgtcaggctt caacagagaa ccgaatggcc ataaccctaa aacggctggg    1200

aattcaattg gatgcaggta ctatattacc agtgtcgaag tcatcaagat aatagaacta    1260

ctgattggaa gctattcaac ctcagacccc gtgcaaagga gaaaagagag aggccgtata    1320

cgatctaact tggtgcagtt ctggtccaaa cacctagtct tttcctcgaa gaactctatg    1380

aagcgccgtt ccgtgccaac aggcaacgat gcttatctta cggagctgga gcatcgtatt    1440

aacacttatg atgtgagaaa acctcgacta tttgataaga gtgtcaagat tctggaatgg    1500

tctaaacttg gacctgcttt gcgtcgggct atccaaagct attacatggt acgactagac    1560

gaatccagca actaa                                                    1575

<210>  61
<211>  51
<212>  DNA
<213>  Artificial

<220>
<223>  Primer

<400>  61
caatcaattg aacaactatc aaaacacaat gcggttgtct ttagcaacac g            51

<210>  62
<211>  54
<212>  DNA
<213>  Artificial

<220>
<223>  Primer

<400>  62
ccgcttttct ttttttttct tcttattgtc ttagttgctg gattcgtcta gtcg          54

<210>  63
<211>  28
<212>  DNA
<213>  Artificial

<220>
<223>  Primer

<400>  63
tgtgttttga tagttgttca attgattg                                      28

<210>  64
<211>  30
<212>  DNA
<213>  Artificial

<220>
<223>  Primer

<400>  64
gacaataaga agaaaaaaaa agaaaagcgg                                     30

<210>  65
<211>  1107
<212>  DNA
<213>  Artificial

<220>
<223>  Synthetic

<400>  65
atgcttgctg agattgggga ctatttcagg acaaatagta gcaatggatt cagttctagc   60

tgctataaca aagagaagct ttcaggagac ctcttggagt ccgattttgt gaattcatca 120

ttcgatgaca caacactcca ctctatgcta tcttcttcaa taaatgctga tccggagcaa 180

tcccagaaac cacaacaaag acacaagaat caatccagtt gccatcagga actcaagatt 240

gaaacagaag atcagcaaga accgtatcta ttgcctgata tgcttaccaa aaacatattc 300

aactcacagt cttctatgtt tgtctactcc agcatcccac aaataagctc agtgcaatcg 360

tttatagaca caaacacctt ttccgctgtt gatggtggtg ccgccccgga aggaaattca 420

ttgtctgtgg actcaacttt aggtctatct tctgaccaat catttgagaa gctatactct 480

tacgaccaaa actttgagaa agaaccacaa tcgccagcat gtcccttagt aaaatgtgac 540

ccagtaagca tcatgaaaac atccacaaac tcattggaga cagctgtcaa caccaaaaga 600

cgaatgtttc agcttagaaa ccaaaatgaa attgaaagtc tcaatgtttc tagtgacatc 660

gttgttgcga catcaaaatt tctcaatcat tcatggatcc cgctgatccg tggacgtaat 720

ttcggaggaa gcaactgcaa accgcccaag aaaccattat ttcctggagc gcgctatgtt 780

actgcccgtc tacgattgga atacagtagc tgtaaagata tgtgtctacc ggaatggaac 840

gagtatgaac tcgaagacag tagaagaatc attcgtattg agagactgtt cgactccaat 900

gaaatcgtag cctccttttc cattgtcggg tctgctgtag agaacccgga aactaggcct 960

gtattcaatc ctaatgtcaa agtgttggaa gtttcgtgtc taagatgtct taccaacaac 1020

aacgagtctg acgaggagaa ctgtgtcaac aaagatttgg atggccgtaa ccgcgatatg 1080

gttaaggact catttggatg caagtaa 1107

<210> 66
<211> 58
<212> DNA
<213> Artificial

<220>
<223> Primer

<400> 66
ccgcttttct ttttttttct tcttattgtc ttacttgcat ccaaatgagt ccttaacc 58

<210> 67
<211> 1203
<212> DNA
<213> Artificial

<220>
<223> Synthetic

<400> 67
atgaattcat cttcaactcg tggcctgcaa gctatatcac tcacaccaac tagagtttta 60

ttcactttga gcactctgtt cgccgatacg tcaaaaatgt ctgctgagaa tggggactat 120

ttcaggacaa acagtagcaa tgaattcagc aataactgct ttaacaaaga aaagctctcg 180

gaggacctct tggaggccag ttttgtcgat tccccaatcg aagacacaat accccacttt 240

atgctatctt cttcaataaa tgctgattcg gaacagcctc aggaaccaca gcatcgacac 300

aagaatcaat ccagttgcca tcgggaactc aagatcgaaa caaacgatca gcaagaaccg 360

tatctgttgc ctgatatgct taccaaaaac atattcaact cacagtcttc tatgtttgtc 420

tattccagca tcccacaaac aagctcagtg caatcgttta tagacacaaa cattttttcc 480

gctgttgatg gtggtgccgc cccagaagaa aattcattgt ctgtggactc aaccttaggt 540

ccatcttctg accaatcatt tgaaaagcta tactcttacg accaaaattt tgagaaagaa 600

ccacaatcgc cagcatgtcc cttagtaaaa tgtgacctag taggcatcat gaaagcatcc 660

acaaactcat tggaggtagc tgtcaacacc aaaagacaaa tgtttcagct tagacaccaa 720

aatgaaattg agagtctcta tgtttctagt gacatcgtta ttgcgacatc aaattttctt 780

gaccattcat ggattccgct gatccgtgga cgtaatttcg gaggaagcaa ctgcaaaccg 840

cccaagaaac cattatttcc tggagcgcgc tatgttactg cccgtctacg attggaatac 900

agtagctgtg aagatatgtg tttaccggaa tggaacgagt atgaactcaa agacagtaga 960

agaatcattc gtcttgagag gcggttcgac tccaatgaaa tcgtggcctc cttttccatt 1020

gtcgggtctg ctgtagagaa ccctgaaact aggcctgcat tcaatcctaa cgtcaaagtg 1080

ttggaagttt cgtgtctaag gtgtcttacc aacaacaacg aatctgacga ggagaactgt 1140

gtcaacaaag atttggatgg ccgtgtccac aatatggtta aagactcaat tggatgcaag 1200

taa 1203

<210> 68
<211> 58
<212> DNA
<213> Artificial

<220>
<223> Primer

<400> 68
ccgcttttct ttttttttct tcttattgtc ttacttgcat ccaattgagt ctttaacc 58

<210> 69
<211> 1218
<212> DNA
<213> Artificial

<220>
<223> Synthetic

<400> 69
atgcggttgt atttagcaac tgtaggaact ccattcatcg gtgcaccgcc agtgattgct 60

agtaatgaag accatagcac tgctcacaat aacaatagca acgactttaa ttatgaatgc 120

tataataaag aaaagctctc tggggatctc ttggaagctg acctttttga tcctccaatt 180

gatgatacaa tgcactgctc catgttttcg tcatcaacaa ctgctgacac tgagcagcta 240

caaaaaccaa agcatgaatc gcaacacaag aatcccttcg attgttgtca ggaactcaag 300

cttaaaatgg ccgatcagca agagttgcat ttgttgccgg agaaaattac caagaatatt 360

tttaattcag agtcgcctgt gttcatatgc accaagattc cccaaacaag tccggtccag 420

tcgttcgtag atgttaatgc ttctttactt gccactaact ccgccctgac tacaaattca 480

ttctctgtag acacaacagt tgatatattg tttgagcaaa cagttcaaga tccatactct 540

taccgtttaa actctaagga aacagcacct ccatccacag ctagcttctt tgggactctc 600

gatctgggac ccaccgtgga agcatccaca acgttactgg aggcagccgt caactctaaa 660

agacgaatag ttgaacttag acagcaaaac agaatcgagg atctcaattt ttcaacatca 720

atgttccttg accattcacg cataccgttg gtgcgtgggc gtagctttgg aggaaatagc 780

agcagacctc ccaagacgcc attaattcct gggacgcggt acgttactgc ccgtttaaga 840

ctggaaaaca gcacaagtga ggatatatgt ttgccccatt ggaacaaagc ggaactcaaa 900

gactctagaa gaataatccg tattgaaaga agataccagt ccaatgagat tgtagcaacc 960

ttttccatag ttggatcggc catagagcac ccaaagacta agcctgcatt cgatcctgac 1020

gtcaaggttt tggaagtctc gtgtctaaaa tgtcccacca acgaaaatga acctaacgag 1080

gagcgatttg tcagtgaatg tacgtcagat gaagcacgag cacaacatgc cttaacagaa 1140

cccctaatgt caggcttcaa cagagaaccg aatggccgta accctaaaac ggctgggaat 1200

tcaattggat gcaggtaa 1218

<210> 70
<211> 54
<212> DNA
<213> Artificial

<220>
<223> Primer

<400> 70
ccgcttttct ttttttttct tcttattgtc ttacctgcat ccaattgaat tccc 54

<210> 71
<211> 1221
<212> DNA
<213> Artificial

<220>
<223> Synthetic

<400> 71
atgcggttgt ctttagcaac acgagaaaca ccgtctaccg gtacatcgcc ggtgtttgct 60

ggtgacgaag atcatagcag tgccaacaat agcagtagca acactttcaa ctatgactac 120

tatataaaag aagaactctc tggaaatctc ctggaaacag ccttttttga atctccgatt 180

aatgatacaa tgctctgctc catgttatcg tatccaaaaa ttgctggtac tgagaaaata 240

caagaaccag accatgaact gcagcaacac aggaatccct tcggttgctg ccaggaactc 300

aaagctgaat tggccgaaca gcaagagctg tatctgctgc caaataaact taccaagaat 360

atttttaatt cagggtctcc tgtgttcata tacaccaaga tcccacaaac aagctcggtt 420

cagtcatttg tggatgttag caattcttta tttgccactg actccgcctt gcctacaagt 480

tcatttcgtg tagactcaac agtcgacata tcgtttgagc actcaattca agattcatac 540

tgttaccgtt caaactctgc agaaatagca cctccatcca cagcactctc ttcagggact 600

cacgacctgg tatccaccgt ggaagcatcc acgacgttac tggaggcagc cgtcaactct 660

aaaagacgaa tgcttgaact tagacagcaa aacagaatcg aggatctcag tttttcaaca 720

tcaatattcc ttgaccattc acgcctaccg ttggttcgtg ggcgtagctt tggaggaaat 780

agcagtagac ctcccaagac gccattgatt cctgggacgc ggtacgttac tgcccgttta 840

agactggaaa acagcacaag tgaggatatg tgtttgcccc agtggaacaa ggcggaactc 900

aaagactcta gaagaataat ccgtattgaa agaagatacc agtctgatga aattgtcgct 960

tccttttcca tagttggatc ggccatagag tacccaaaga ctaagcctgc attcgatcct 1020

gacgtcaagg ttttggaagt ctcgtgtcta aaatgtccca ccaacgaaaa tgaacctaac 1080

gaggagcgat ttgtcagtga atgtacgtca gatgaagcac gaacacaaca tgccttaaca 1140

gaacccctaa cgtcaggctt caacagagaa ccgaatggcc ataaccctaa aacggctggg 1200

aattcaattg gatgcaggta a 1221

<210> 72
<211> 54
<212> DNA
<213> Artificial

<220>
<223> Primer

<400> 72
ccgcttttct ttttttttct tcttattgtc ttacctgcat ccaattgaat tccc 54

<210> 73
<211> 1170
<212> DNA
<213> Artificial

<220>
<223> Synthetic

<400> 73
atgcagctac tgtcagcaag ttcaagcact ccattcgtcg ctatgtcgac agtaaacgct 60

gggaacgagt gttatcttag gaccaacaat agtaacagca acggtatcag cgataaccgc 120

ttcagaaaaa aaaagctttc tgaggatttg ttgaaagccg attttgtcga agacacagcc 180

ggtaacccaa tgatttaccc catggtttta tctgctccaa catatataaa aactgaacat 240

ctccaagaat tggagcataa acagcaagga tcaaatctgt tgtctgaaat gtttaccaaa 300

aatattttca attcacattc tgcaatgttt gtttactctg aaatccctca aataagttcg 360

gttcagtcat tcattggttc tgacacctct gtccttgttg ctgatgcctc ttcagaaaaa 420

aattcattgt ttgtgtactc aaatactggt atatcttttg atccgtcaga tgaggatcca 480

tgcccttacg aggctaattt tgatccagga caactattct cagcggtatc cgcagtagaa 540

gacggttcta tggcaacatt gacagctccc actatgtcat tagaaggtgt cgttaattcc 600

aaaaaaggaa tatgtcaact tagacagcat aacagaatcg agggatttga ttcttcccat 660

gaattcaaag tcttgggatc aatgttcctt aaccattcgt acgtaccgct ggttcgtgga 720

cgtagtttcg gaggaaacaa ttgtagacct cccaaagagc cattaattcc tggaacccgg 780

tacgttgctg cccatctaag attggaaaac aatacctgcg aggatatgtg tctcccagaa 840

tggaacaagc atgagcttga agatagtaga agaatcattc gtatagagag aagataccag 900

tcgaatgaaa ttgtggcctc tttttctata gttgggtctg ctaaagagaa tccacaaaca 960

aagccttcaa gcgatcctga cgtgaaagtt ttggaagtct cgtgtctgaa atgtttcatc 1020

aacggcaacg aatctgacga gcaatttgtg actgaatgtc agtcagatga agcgcattat 1080

ggcttcacag agttaccaaa acaaaacttt agcagaaaat caaaaggctg cagccacaaa 1140

atggcaaata acacgattgc atgccaataa 1170

<210> 74
<211> 58
<212> DNA
<213> Artificial

<220>
<223> Primer

<400> 74
ccgcttttct ttttttttct tcttattgtc ttattggcat gcaatcgtgt tatttgcc 58

<210> 75
<211> 1236
<212> DNA
<213> Artificial

<220>
<223> Synthetic

<400> 75
atgcaattgt ttttagcaac tctaggaatt ccgttcaccg gcacattacc agccattgct 60

ggtaacgagg atcatagcag tgccaacaac aacaatggca acggtttcag ttatgactgg 120

catggaaatg atacgctttt tgcggaactc ttggaaactg acttttttga tcatccgatt 180

gatgacgcaa tgctctgctc cttgttatcc cctccaagag ctgctgagac tgagcaacag 240

caagatacag aatgggagca acaaaaacac acaaatctct ttggttgctg ccagaaacac 300

aaagttgaaa tggccaaaca gcaagaactg tatttgctgc cagataagct taccaagaat 360

attttcaatt cagagtcgcc tgtgttcata tgcaccaaga tcccacaaac aagttcggtt 420

cagtcgttcg tagatgttga tgcttctttg cttgccactg actcctccct aagtaaagat 480

tcattctctg cagactcaac agttgatgta ttgtttgagc agtcagttca agagccatac 540

ttttaccgtt caaactctgc ggaaacagca cctccatcca cagcatgctc ctcagcgact 600

ctcgatctgg tatccacttt ggaagcaacc acaacgttat ttgaggaagc cgccaactcc 660

aaaaaacaaa tgattgggct taaacagcaa tccaagatcg aagatctcag tttttcctgc 720

aaactaaatt tctcagcatc aatgttcctt gaccattcat gcataccgct ggtgcgtggg 780

cgtagctttg gaggaaatag cagacctccc aagacgccat taattcctgg gacgcggtac 840

gttactgccc gtctaagact ggagaacagt acaagtgaag atatgtgttt tccccactgg 900

agcgagcggg aacttagcga ctcaagaaga atcattcgta ttgagagaag ttaccagtcc 960

aatgaaattg tagcatcttt tttcatagtt ggatcggcca tagatcaccc ggagactaaa 1020

ccttcatctg aattagacgt gaaagtttta gaagtttcgt gtctaagatg ttttatcaac 1080

gacaatgaat ctgaggagga gcgatttgtc agtgaatata cctcagatga agcacgagca 1140

caacatgcct taacagaacc gctaacgtca ggcttgaaca aagaaccgaa tggccataac 1200

cctaaaacgg ctgggaattc aattggatgc aggtaa 1236

<210> 76
<211> 54
<212> DNA
<213> Artificial

<220>
<223> Primer

<400> 76
ccgcttttct ttttttttct tcttattgtc ttacctgcat ccaattgaat tccc 54

<210> 77
<211> 1230
<212> DNA
<213> Artificial

<220>
<223> Synthetic

<400> 77
atgcagtttt acttagctac tcctaccact cctttcgttg ataacttgac aataccctct 60

ggtagcgggg cttatagcaa atccaacggc aacaatagca gcagcttcgg tcatagttac 120

aacggaaaag aagagctcac tgatgacctt ctgactgcag atttcgtcga cccaccaatc 180

gacgacgcaa tattgtactc gatgctatct cctttcatga atgctactac cgaacaacca 240

caagaattcc agaatgaaca gcagcatcag aacctttccg gttgctacca ggagctgctg 300

gttcaaacag aggaacaaca acaactggat ttgttgccta atatgcaccc cgagaatatt 360

ttcaattctg agtcttctat gttcgtgtac tccagtatcc cgcaaagaaa ttctgttcac 420

tcgctgatca gtgtcaacac ctcttctctt gcccccgatt ctgctgaggg gggaggctta 480

tcgtttgtgg acaccatagg cccatcattt gaccattctg ttgaaacctc actctcttac 540

ggaatgaact ctacggactc aactctaccg tcctcagcac gctcctcggt aggacttgac 600

ctactaccca ccgtggcagc tcctgcgtca tccgtttcct cattggaaac ggtcatcaac 660

tccaagagac aaaagctgca attcagacag cacaacagga ttgaagaact caattctcca 720

cacggtatca aagcacaggc accggtgttg cttaactact caagtcttcc gctggttcgt 780

ggacttagct ctggtgggaa caacagcaga cctcccaaag agccattaat tcctggagca 840

cagtacatta ctgcccttct gagactagag aacagctcct gcgaggactt atgtctacca 900

gaatggaacg aggacgaact gaaggactgc agaaggatca ttcgcataga gagaaaacgc 960

cactccaacg agatagtggc gtcattttct atagtcggtt ctgccacgca gaacccacaa 1020

atcaagccag catccgactc tgacgtgcaa gtgttggaag tctcatgttt acaatgcctc 1080

gtcaaccata acgaaactga cgaggggaaa caaggaagct ccccaaacga aatacagcat 1140

agctctgcag agccaccgaa atcgaacctc aacaggacat tgagtagtag tagccacaga 1200

gcagctggga gctccgtggg ataccagtaa 1230

<210> 78
<211> 54
<212> DNA
<213> Artificial

<220>
<223> Primer

<400> 78
ccgcttttct ttttttttct tcttattgtc ttactggtat cccacggagc tccc 54

<210> 79
<211> 954
<212> DNA
<213> Artificial

<220>
<223> Synthetic

<400> 79
atgttaacag caggttcagt tcagcggcta caaatatcta tgtttcaaac acaggaacag 60

gagcaggtgg acctttcgtc ctctatacat accgagaaca ttttcaattc agaatcgtct 120

ggtcccgcat atccgaacat cccacaaatt agttctgttc actcgtttat aggtgccaaa 180

agctctcttc ttgctgatgc tgctccgaat caatgtttat catttgtgga ctcagcaaaa 240

gttacatcgt ttgaccaatc attccatggt tcaccatctt ataagctaaa ttctgcaaaa 300

ctagcagttt catcgtctga gtacttgtca gtagaatcat ccacagaggt agactccaca 360

atttcattgg aagcacaagt tgaccgcaaa agacaaaaga ttcaacccga acgacacagc 420

aaaaccgaca atctgaacct cttttctgac ttcgatctat tggcaccagc gtttcttacc 480

tactacttgc cattgtttcg tggatgtagc tctggaggca acaacaaacc cccgaaaaag 540

cgatcaatta ctggaacaca gtacgttgcc gccagtttga gactggaaag cagtaccatt 600

gaggacattt gcttaccgaa atggaacgag aacgagctca aggacaatag aagaatcatc 660

cgcatagaga gaagacaaca gggaaatgaa attgcagctt ccttttcaat agttgggtct 720

gctttagaga acccacacac caaacctaca ctggatcctg acgtggaagt attagaagtc 780

tcgtgcttac ggtgtctagt caattacgac gagattgatg acaaacaaac agtgcatgga 840

agccttttac acgaagttca gtctgaattc accgaaatac ggaaacaaga atctaccggg 900

aaacgtagca gttatgcctg tacgtcgaat gggacctcca tcgaatgcca gtaa 954

<210> 80
<211> 54
<212> DNA
<213> Artificial

<220>
<223> Primer

<400> 80
ccgcttttct ttttttttct tcttattgtc ttactggcat tcgatggagg tccc 54

<210> 81
<211> 882
<212> DNA
<213> Artificial

<220>
<223> Synthetic

<400> 81
atgtttcaac cactcgagca agaacaacaa ctggatttgt tgtttgccat gcctccggag 60

aacattttta actctgactc ttctgcactt accttttcca atacttttca aaccagttca 120

gttcactcgg tcacaggtgt tgacagccct tcacttgtta atattaatat cccaaaatca 180

gaatcgttct ttatggactc aatgacgggt gcatcttttg gacaaacttt tcaagaccta 240

ttcccagcgt ttcctttgct aaaacttcct accatggtag cacctccaac ttcgttgaag 300

atggtcatgg accgtaaacg gcgacagtcc caaataacac agcagaatac cactgaaaat 360

ctcaattcct ttcatggcat gaatatgccg gtaccagcgt tccttgaata ctcggccttc 420

ccgctggttc gtggcttttg ctctggagga aaaataaagc cgcctaagaa ccaactagtt 480

cctggaactg agtacgtcaa tgtctgcttg gagttgctca acgtcaacta tgaaggcatt 540

tgtttaccta aatggaatga gaaagaactt gaggaaagta gaaggattgt tcgagtagag 600

aaaagacgtc agtataataa aattgaggct gccttttcaa tagttgattc tgttcaagaa 660

aatccacaaa ccaatattgc agttgaccct aacgttgatg tgctggagtt ttcgtgttta 720

ttgtgtccaa tcaaagaaca caactcagaa gtagcgcaaa ccgtccctac aagctcttca 780

agtgaagaac acaagtacag cgagctagca ggacacgact ttgaagaaaa tttgagctgt 840

ggtagtgata aagaaattga gacttctgtt agatttgaat aa 882

<210> 82
<211> 60
<212> DNA
<213> Artificial

<220>
<223> Primer

<400> 82
ccgcttttct ttttttttct tcttattgtc ttattcaaat ctaacagaag tctcaatttc 60

<210> 83
<211> 1218
<212> DNA
<213> Artificial

<220>
<223> Synthetic

<400> 83
atgcggttgt atttagcaac tgtaggaact ccattcatcg gtgcaccgcc agtgattgct 60

agtaatgaag accatagcac tgctcacaat aacaatagca acgactttaa ttatgaatgc 120

tataataaag aaaagctctc tggggatctc ttggaagctg accttttgga tcctccaatt 180

gatgatacaa tgcactgctc catgttttcg tcatcaacaa ctgctgacac tgagcagcta 240

caaaaaccaa agcatgaatc gcaacacaag aatcccttcg attgttgtca ggaactcaag 300

cttaaaatgg ccgatcagca agagttgcat ttgttgccgg agaaaattac caagaatatt 360

tttaattcag agtcgcctgt gttcatatgc accaagattc cccaaacaag tccggtccag 420

tcgttcgtag atgttaatgc ttctttactt gccactaact ccgccctgac tacaaattca 480

ttctctgtag acacaacagt tgatatattg tttgagcaaa cagttcaaga tccatactct 540

taccgtttaa actctaagga aacagcacct ccatccacag ctagcttctt tgggactctc 600

gatctgggac ccaccgtgga agcatccaca acgttactgg aggcagccgt caactctaaa 660

agacgaatag ttgaacttag acagcaaaac agaatcgagg atctcaattt ttcaacatca 720

atgttccttg accattcacg cataccgttg gtgcgtgggc gtagctttgg aggaaatagc 780

agcagacctc ccaagacgcc attaattcct gggacgcggt acgttactgc ccgtttaaga 840

ctggaaaaca gcacaagtga ggatatatgt ttgccccatt ggaacaaagc ggaactcaaa 900

gactctagaa gaataatccg tattgaaaga agataccagt ccaatgagat tgtagcaacc 960

ttttccatag ttggatcggc catagagcac ccaaagacta agcctgcatt cgatcctgac 1020

gtcaaggttt tggaagtctc gtgtctaaaa tgtcccacca acgaaaatga acctaacgag 1080

gagcgatttg tcagtgaatg tacgtcagat gaagcacgag cacaacatgc cttaacagaa 1140

cccctaatgt caggcttcaa cagagaaccg aatggccgta accctaaaac ggctgggaat 1200

tcaattggat gcaggtaa 1218

<210> 84
<211> 54
<212> DNA
<213> Artificial

<220>
<223> Primer

<400> 84
ccgcttttct ttttttttct tcttattgtc ttacctgcat ccaattgaat tccc 54

<210> 85
<211> 1176
<212> DNA
<213> Artificial

<220>
<223> Synthetic

<400> 85
atgcagtttt acttagctac tcctaccact cctttcgttg ataacttgac aataccctct 60

ggtagcgggg cttatagcaa atccaacggc aacaatagca gcagcttcgg tcatagttac 120

aacggaaaag aagagctcac tgatgacctt ctgactgcag atttcgtcga cccaccaatc 180

gacgacgcaa tattgtactc gatgctatct cctttcatga atgctactac cgaacaacca 240

caagaattcc agaatgaaca gcagcatcag aacctttccg gttgctacca ggagctgctg 300

gttcaaacag aggaacaaca acaactggat ttgttgccta atatgcaccc cgagaatatt 360

ttcaattctg agtcttctat gttcgtgtac tccagtatcc cgcaaagaaa ttctgttcac 420

tcgctgatca gtgtcaacac ctcttctctt gcccccgatt ctgctgaggg gggaggctta 480

tcgtttgtgg acaccatagg cccatcattt gaccattctg ttgaaacctc actctcttac 540

ggaatgaact ctacggactc aactctaccg tcctcagcac gctcctcggt aggacttgac 600

ctactaccca ccgtggcagc tcctgcgtca tccgtttcct cattggaaac ggtcatcaac 660

tccaagagac aaaagctgca attcagacag cacaacagga ttgaagaact caattctcca 720

cacggtatca aagcacaggc accggtgttg cttaactact caagtcttcc gctggttcgt 780

ggacttagct ctggtgggaa caacagcaga cctcccaaag agccattaat tcctggagca 840

cagtacatta ctgcccttct gagactagag aacagctcct gcgaggactt atgtctacca 900

gaatggaacg aggacgaact gaaggactgc agaaggatca ttcgcataga gagaaaacgc 960

cactccaacg agatagtggc cgactctgac gtgcaagtgt tggaagtctc atgtttacaa 1020

tgcctcgtca accataacga aactgacgag gggaaacaag gaagctcccc aaacgaaata 1080

cagcatagct ctgcagagcc accgaaatcg aacctcaaca ggacattgag tagtagtagc 1140

cacagagcag ctgggagctc cgtgggatac cagtaa 1176

<210> 86
<211> 54
<212> DNA
<213> Artificial

<220>
<223> Primer

<400> 86
ccgcttttct ttttttttct tcttattgtc ttactggtat cccacggagc tccc 54

<210> 87
<211> 954
<212> DNA
<213> Artificial

<220>
<223> Synthetic

<400> 87
atgttaacag caggttcagt tcagcggcta caaatatcta tgtttcaaac acaggaacag 60

gagcaggtgg acctttcgtc ctctatacat accgagaaca ttttcaattc agaatcgtct 120

ggtcccgcat atccgaacat cccacaaatt agttctgttc actcgtttat aggtgccaaa 180

agctctcttc ttgctgatgc tgctccgaat caatgtttat catttgtgga ctcagcaaaa 240

gttacatcgt ttgaccaatc attccatggt tcaccatctt ataagctaaa ttctgcaaaa 300

ctagcagttt catcgtctga gtacttgtca gtagaatcat ccacagaggt agactccaca 360

atttcattgg aagcacaagt tgaccgcaaa agacaaaaga ttcaacccga acgacacagc 420

aaaaccgaca atctgaacct cttttctgac ttcgatctat tggcaccagc gtttcttacc 480

tactacttgc cattgtttcg tggatgtagc tctggaggca acaacaaacc cccgaaaaag 540

cgatcaatta ctggaacaca gtacgttgcc gccagtttga gactggaaag cagtaccatt 600

gaggacattt gcttaccgaa atggaacgag aacgagctca aggacaatag aagaatcatc 660

cgcatagaga gaagacaaca gggaaatgaa attgcagctt ccttttcaat agttgggtct 720

gctttagaga acccacacac caaacctaca ctggatcctg acgtggaagt attagaagtc 780

tcgtgcttac ggtgtctagt caattacgac gagattgatg acaaacaaac agtgcatgga 840

agccttttac acgaagttca gtctgaattc accgaaatac ggaaacaaga atctaccggg 900

aaacgtagca gttatgcctg tacgtcgaat gggacctcca tcgaatgcca gtaa 954

<210> 88
<211> 54
<212> DNA
<213> Artificial

<220>
<223> Primer

<400> 88
ccgcttttct ttttttttct tcttattgtc ttactggcat tcgatggagg tccc 54

<210> 89
<211> 1221
<212> DNA
<213> Artificial

<220>
<223> Synthetic

<400> 89
atgcggttgt ctttagcaac acgagaaaca ccgtctaccg gtacatcgcc ggtgtttgct 60

ggtgacgaag atcatagcag tgccaacaat agcagtagca acactttcaa ctatgactac 120

tatataaaag aagaactctc tggaaatctc ctggaaacag ccttttttga atctccgatt 180

aatgatacaa tgctctgctc catgttatcg tatccaaaaa ttgctggtac tgagaaaata 240

caagaaccag accatgaact gcagcaacac aggaatccct tcggttgctg ccaggaactc 300

aaagctgaat tggccgaaca gcaagagctg tatctgctgc caaataaact taccaagaat 360

atttttaatt cagggtctcc tgtgttcata tacaccaaga tcccacaaac aagctcggtt 420

cagtcatttg tggatgttag caattcttta tttgccactg actccgcctt gcctacaagt 480

tcatttcgtg tagactcaac agtcgacata tcgtttgagc actcaattca agattcatac 540

tgttaccgtt caaactctgc agaaatagca cctccatcca cagcactctc ttcagggact 600

cacgacctgg tatccaccgt ggaagcatcc acgacgttac tggaggcagc cgtcaactct 660

aaaagacgaa tgcttgaact tagacagcaa aacagaatcg aggatctcag tttttcaaca 720

tcaatattcc ttgaccattc acgcctaccg ttggttcgtg ggcgtagctt tggaggaaat 780

agcagtagac ctcccaagac gccattgatt cctgggacgc ggtacgttac tgcccgttta 840

agactggaaa acagcacaag tgaggatatg tgtttgcccc agtggaacaa ggcggaactc 900

aaagactcta gaagaataat ccgtattgaa agaagatacc agtctgatga aattgtcgct 960

tccttttcca tagttggatc ggccatagag tacccaaaga ctaagcctgc attcgatcct 1020

gacgtcaagg ttttggaagt ctcgtgtcta aaatgtccca ccaacgaaaa tgaacctaac 1080

gaggagcgat ttgtcagtga atgtacgtca gatgaagcac gaacacaaca tgccttaaca 1140

gaacccctaa cgtcaggctt caacagagaa ccgaatggcc ataaccctaa aacggctggg 1200

aattcaattg gatgcaggta a 1221

<210> 90
<211> 54
<212> DNA
<213> Artificial

<220>
<223> Primer

<400> 90
ccgcttttct ttttttttct tcttattgtc ttacctgcat ccaattgaat tccc 54

<210> 91
<211> 54
<212> DNA
<213> Artificial

<220>
<223> Synthetic

<400> 91
tactatatca ccagtgttga agtcataaag attatagaat tactggtcgg aagc 54

<210> 92
<211> 57
<212> DNA
<213> Artificial

<220>
<223> Synthetic

<400> 92
attctggaat ggtccaaact tgggccagct ttgcagcggg ctatccaaag ctattac 57

<210> 93
<211> 18
<212> PRT
<213> Artificial

<220>
<223> Synthetic

<400> 93

Tyr Tyr Ile Thr Ser Val Glu Val Ile Lys Ile Ile Glu Leu Leu Val
1               5                   10                  15

Gly Ser

<210> 94
<211> 19
<212> PRT
<213> Artificial

<220>
<223> Synthetic

<400> 94

Ile Leu Glu Trp Ser Lys Leu Gly Pro Ala Leu Gln Arg Ala Ile Gln
1               5                   10                  15

Ser Tyr Tyr

<210> 95
<211> 493
<212> PRT
<213> Komagataella pastoris

<400> 95

Met Leu Ala Glu Ile Gly Asp Tyr Phe Arg Thr Asn Ser Ser Asn Gly
1               5                   10                  15

Phe Ser Ser Ser Cys Tyr Asn Lys Glu Lys Leu Ser Gly Asp Leu Leu
            20                  25                  30

Glu Ser Asp Phe Val Asn Ser Ser Phe Asp Asp Thr Thr Leu His Ser
        35                  40                  45

Met Leu Ser Ser Ser Ile Asn Ala Asp Pro Glu Gln Ser Gln Lys Pro
    50                  55                  60

Gln Gln Arg His Lys Asn Gln Ser Ser Cys His Gln Glu Leu Lys Ile
65                  70                  75                  80

Glu Thr Glu Asp Gln Gln Glu Pro Tyr Leu Leu Pro Asp Met Leu Thr
                85                  90                  95

Lys Asn Ile Phe Asn Ser Gln Ser Ser Met Phe Val Tyr Ser Ser Ile
            100                 105                 110

Pro Gln Ile Ser Ser Val Gln Ser Phe Ile Asp Thr Asn Thr Phe Ser
        115                 120                 125

Ala Val Asp Gly Gly Ala Ala Pro Glu Gly Asn Ser Leu Ser Val Asp
    130                 135                 140

Ser Thr Leu Gly Leu Ser Ser Asp Gln Ser Phe Glu Lys Leu Tyr Ser
145                 150                 155                 160

Tyr Asp Gln Asn Phe Glu Lys Glu Pro Gln Ser Pro Ala Cys Pro Leu
                165                 170                 175

Val Lys Cys Asp Pro Val Ser Ile Met Lys Thr Ser Thr Asn Ser Leu
            180                 185                 190

Glu Thr Ala Val Asn Thr Lys Arg Arg Met Phe Gln Leu Arg Asn Gln
        195                 200                 205

Asn Glu Ile Glu Ser Leu Asn Val Ser Ser Asp Ile Val Val Ala Thr
    210                 215                 220

Ser Lys Phe Leu Asn His Ser Trp Ile Pro Leu Ile Arg Gly Arg Asn
225                 230                 235                 240

Phe Gly Gly Ser Asn Cys Lys Pro Pro Lys Lys Pro Leu Phe Pro Gly
                245                 250                 255

Ala Arg Tyr Val Thr Ala Arg Leu Arg Leu Glu Tyr Ser Ser Cys Lys
            260                 265                 270

Asp Met Cys Leu Pro Glu Trp Asn Glu Tyr Glu Leu Glu Asp Ser Arg
        275                 280                 285

Arg Ile Ile Arg Ile Glu Arg Leu Phe Asp Ser Asn Glu Ile Val Ala
    290                 295                 300

Ser Phe Ser Ile Val Gly Ser Ala Val Glu Asn Pro Glu Thr Arg Pro
305                 310                 315                 320

Val Phe Asn Pro Asn Val Lys Val Leu Glu Val Ser Cys Leu Arg Cys
                325                 330                 335

Leu Thr Asn Asn Asn Glu Ser Asp Glu Glu Asn Cys Val Asn Lys Asp
            340                 345                 350

Leu Asp Gly Arg Asn Arg Asp Met Val Lys Asp Ser Phe Gly Cys Lys
        355                 360                 365

Tyr Tyr Ile Thr Ser Val Glu Val Ile Lys Ile Ile Glu Leu Leu Val
    370                 375                 380

Gly Ser Ser Ser Ile Ser Asp Pro His Gln Met Arg Lys Glu Arg Gly

385                 390                 395                 400

Arg Val Arg Ser Asn Leu Ala Gln Phe Trp Ser Lys Arg Leu Val Ser
                405                 410                 415

Ser Ser Arg Lys Thr Met Lys Gln Gly Phe Leu Pro Thr Cys Asn Asp
            420                 425                 430

Asp Tyr Phe Ala Glu Leu Ala His Arg Ile Asn Thr Tyr Asp Val Arg
        435                 440                 445

Lys Pro Arg Leu Phe Asp Lys Cys Ile Lys Ile Leu Glu Trp Ser Lys
    450                 455                 460

Leu Lys Pro Ala Leu Gln Arg Ala Met Gln Ser Tyr Tyr Met Val Gln
465                 470                 475                 480

Leu Asp Glu Ser Ser Asn Lys Ile Ala Thr Ala Asn Asn
                485                 490

<210>  96
<211>  518
<212>  PRT
<213>  Komagataella pastoris

<400>  96

Met Asn Ser Ser Ser Thr Arg Gly Leu Gln Ala Ile Ser Leu Thr Pro
1               5                   10                  15

Thr Arg Val Leu Phe Thr Leu Ser Thr Leu Phe Ala Asp Thr Ser Lys
            20                  25                  30

Met Ser Ala Glu Asn Gly Asp Tyr Phe Arg Thr Asn Ser Ser Asn Glu
        35                  40                  45

Phe Ser Asn Asn Cys Phe Asn Lys Glu Lys Leu Ser Glu Asp Leu Leu
    50                  55                  60

Glu Ala Ser Phe Val Asp Ser Pro Ile Glu Asp Thr Ile Pro His Phe
65                  70                  75                  80

Met Leu Ser Ser Ser Ile Asn Ala Asp Ser Glu Gln Pro Gln Glu Pro
                85                  90                  95

Gln His Arg His Lys Asn Gln Ser Ser Cys His Arg Glu Leu Lys Ile
            100                 105                 110

Glu Thr Asn Asp Gln Gln Glu Pro Tyr Leu Leu Pro Asp Met Leu Thr

115                 120                 125

Lys Asn Ile Phe Asn Ser Gln Ser Ser Met Phe Val Tyr Ser Ser Ile
    130                 135                 140

Pro Gln Thr Ser Ser Val Gln Ser Phe Ile Asp Thr Asn Ile Phe Ser
145                 150                 155                 160

Ala Val Asp Gly Gly Ala Ala Pro Glu Glu Asn Ser Leu Ser Val Asp
                165                 170                 175

Ser Thr Leu Gly Pro Ser Ser Asp Gln Ser Phe Glu Lys Leu Tyr Ser
            180                 185                 190

Tyr Asp Gln Asn Phe Glu Lys Glu Pro Gln Ser Pro Ala Cys Pro Leu
        195                 200                 205

Val Lys Cys Asp Leu Val Gly Ile Met Lys Ala Ser Thr Asn Ser Leu
    210                 215                 220

Glu Val Ala Val Asn Thr Lys Arg Gln Met Phe Gln Leu Arg His Gln
225                 230                 235                 240

Asn Glu Ile Glu Ser Leu Tyr Val Ser Ser Asp Ile Val Ile Ala Thr
                245                 250                 255

Ser Asn Phe Leu Asp His Ser Trp Ile Pro Leu Ile Arg Gly Arg Asn
            260                 265                 270

Phe Gly Gly Ser Asn Cys Lys Pro Pro Lys Lys Pro Leu Phe Pro Gly
        275                 280                 285

Ala Arg Tyr Val Thr Ala Arg Leu Arg Leu Glu Tyr Ser Ser Cys Glu
    290                 295                 300

Asp Met Cys Leu Pro Glu Trp Asn Glu Tyr Glu Leu Lys Asp Ser Arg
305                 310                 315                 320

Arg Ile Ile Arg Leu Glu Arg Arg Phe Asp Ser Asn Glu Ile Val Ala
                325                 330                 335

Ser Phe Ser Ile Val Gly Ser Ala Val Glu Asn Pro Glu Thr Arg Pro
            340                 345                 350

Ala Phe Asn Pro Asn Val Lys Val Leu Glu Val Ser Cys Leu Arg Cys
        355                 360                 365

Leu Thr Asn Asn Asn Glu Ser Asp Glu Glu Asn Cys Val Asn Lys Asp
    370                 375                 380

Leu Asp Gly Arg Val His Asn Met Val Lys Asp Ser Ile Gly Cys Lys
385                 390                 395                 400

Tyr Tyr Ile Thr Ser Val Glu Val Ile Lys Ile Ile Glu Leu Leu Val
                405                 410                 415

Gly Ser Ser Ser Ile Ser Asp Pro Gln Gln Met Arg Lys Glu Arg Gly
            420                 425                 430

Arg Val Arg Ser Asn Leu Ala Gln Phe Trp Ser Lys His Leu Val Ser
        435                 440                 445

Ser Ser Arg Lys Thr Arg Lys Gln Arg Phe Leu Pro Thr Cys Asn Asp
    450                 455                 460

Asp Tyr Phe Ala Glu Leu Val His Arg Ile Asn Thr Tyr Asp Val Arg
465                 470                 475                 480

Lys Pro Arg Leu Phe Asp Lys Ser Val Lys Ile Leu Glu Trp Pro Asn
                485                 490                 495

Leu Gly Pro Ala Leu Gln Arg Ala Ile Gln Ser Tyr Tyr Met Val Arg
            500                 505                 510

Leu Glu Glu Ser Ser Asn
        515

<210>  97
<211>  523
<212>  PRT
<213>  Komagataella pastoris

<400>  97

Met Arg Leu Tyr Leu Ala Thr Val Gly Thr Pro Phe Ile Gly Ala Pro
1               5                   10                  15

Pro Val Ile Ala Ser Asn Glu Asp His Ser Thr Ala His Asn Asn Asn
            20                  25                  30

Ser Asn Asp Phe Asn Tyr Glu Cys Tyr Asn Lys Glu Lys Leu Ser Gly
        35                  40                  45

Asp Leu Leu Glu Ala Asp Leu Phe Asp Pro Pro Ile Asp Asp Thr Met
    50                  55                  60

His Cys Ser Met Phe Ser Ser Ser Thr Thr Ala Asp Thr Glu Gln Leu
65                  70                  75                  80

Gln Lys Pro Lys His Glu Ser Gln His Lys Asn Pro Phe Asp Cys Cys
                85                  90                  95

Gln Glu Leu Lys Leu Lys Met Ala Asp Gln Gln Glu Leu His Leu Leu
            100                 105                 110

Pro Glu Lys Ile Thr Lys Asn Ile Phe Asn Ser Glu Ser Pro Val Phe
        115                 120                 125

Ile Cys Thr Lys Ile Pro Gln Thr Ser Pro Val Gln Ser Phe Val Asp
    130                 135                 140

Val Asn Ala Ser Leu Leu Ala Thr Asn Ser Ala Leu Thr Thr Asn Ser
145                 150                 155                 160

Phe Ser Val Asp Thr Thr Val Asp Ile Leu Phe Glu Gln Thr Val Gln
                165                 170                 175

Asp Pro Tyr Ser Tyr Arg Leu Asn Ser Lys Glu Thr Ala Pro Pro Ser
            180                 185                 190

Thr Ala Ser Phe Phe Gly Thr Leu Asp Leu Gly Pro Thr Val Glu Ala
        195                 200                 205

Ser Thr Thr Leu Leu Glu Ala Ala Val Asn Ser Lys Arg Arg Ile Val
    210                 215                 220

Glu Leu Arg Gln Gln Asn Arg Ile Glu Asp Leu Asn Phe Ser Thr Ser
225                 230                 235                 240

Met Phe Leu Asp His Ser Arg Ile Pro Leu Val Arg Gly Arg Ser Phe
                245                 250                 255

Gly Gly Asn Ser Ser Arg Pro Pro Lys Thr Pro Leu Ile Pro Gly Thr
            260                 265                 270

Arg Tyr Val Thr Ala Arg Leu Arg Leu Glu Asn Ser Thr Ser Glu Asp
        275                 280                 285

Ile Cys Leu Pro His Trp Asn Lys Ala Glu Leu Lys Asp Ser Arg Arg
    290                 295                 300

Ile Ile Arg Ile Glu Arg Arg Tyr Gln Ser Asn Glu Ile Val Ala Thr
305                 310                 315                 320

Phe Ser Ile Val Gly Ser Ala Ile Glu His Pro Lys Thr Lys Pro Ala
                325                 330                 335

Phe Asp Pro Asp Val Lys Val Leu Glu Val Ser Cys Leu Lys Cys Pro
            340                 345                 350

Thr Asn Glu Asn Glu Pro Asn Glu Glu Arg Phe Val Ser Glu Cys Thr
        355                 360                 365

Ser Asp Glu Ala Arg Ala Gln His Ala Leu Thr Glu Pro Leu Met Ser
    370                 375                 380

Gly Phe Asn Arg Glu Pro Asn Gly Arg Asn Pro Lys Thr Ala Gly Asn
385                 390                 395                 400

Ser Ile Gly Cys Arg Tyr Tyr Ile Thr Ser Val Glu Val Ile Lys Ile
                405                 410                 415

Ile Glu Leu Leu Ile Gly Ser Tyr Ser Thr Ser Asp Pro Val Gln Arg
            420                 425                 430

Arg Lys Glu Arg Gly Arg Ile Arg Ser Asn Leu Val Gln Phe Trp Ser
        435                 440                 445

Lys His Leu Val Phe Ser Ser Lys Asn Ser Met Lys Arg Arg Ser Val
    450                 455                 460

Pro Thr Gly Asn Asp Ala Tyr Leu Thr Glu Leu Glu His Arg Ile Asn
465                 470                 475                 480

Thr Tyr Asp Val Arg Lys Pro Arg Leu Phe Asp Lys Ser Val Lys Ile
                485                 490                 495

Leu Glu Trp Ser Lys Leu Gly Pro Ala Leu Arg Arg Ala Ile Gln Ser
            500                 505                 510

Tyr Tyr Met Val Arg Leu Asp Glu Ser Ser Asn
        515                 520

<210>  98
<211>  532
<212>  PRT
<213>  Komagataella pastoris

<400>  98

Met Arg Leu Ser Leu Ala Thr Arg Glu Thr Pro Ser Thr Gly Thr Ser
1               5                   10                  15

Pro Val Phe Ala Gly Asp Glu Asp His Ser Ser Ala Asn Asn Ser Ser
            20                  25                  30

Ser Asn Thr Phe Asn Tyr Asp Tyr Tyr Ile Lys Glu Glu Leu Ser Gly
        35                  40                  45

Asn Leu Leu Glu Thr Ala Phe Phe Glu Ser Pro Ile Asn Asp Thr Met
    50                  55                  60

Leu Cys Ser Met Leu Ser Tyr Pro Lys Ile Ala Gly Thr Glu Lys Ile
65                  70                  75                  80

Gln Glu Pro Asp His Glu Leu Gln Gln His Arg Asn Pro Phe Gly Cys
                85                  90                  95

Cys Gln Glu Leu Lys Ala Glu Leu Ala Glu Gln Gln Glu Leu Tyr Leu
            100                 105                 110

Leu Pro Asn Lys Leu Thr Lys Asn Ile Phe Asn Ser Gly Ser Pro Val
        115                 120                 125

Phe Ile Tyr Thr Lys Ile Pro Gln Thr Ser Ser Val Gln Ser Phe Val
    130                 135                 140

Asp Val Ser Asn Ser Leu Phe Ala Thr Asp Ser Ala Leu Pro Thr Ser
145                 150                 155                 160

Ser Phe Arg Val Asp Ser Thr Val Asp Ile Ser Phe Glu His Ser Ile
                165                 170                 175

Gln Asp Ser Tyr Cys Tyr Arg Ser Asn Ser Ala Glu Ile Ala Pro Pro
            180                 185                 190

Ser Thr Ala Leu Ser Ser Gly Thr His Asp Leu Val Ser Thr Val Glu
        195                 200                 205

Ala Ser Thr Thr Leu Leu Glu Ala Ala Val Asn Ser Lys Arg Arg Met
    210                 215                 220

Leu Glu Leu Arg Gln Gln Asn Arg Ile Glu Asp Leu Ser Phe Ser Thr
225                 230                 235                 240

Ser Ile Phe Leu Asp His Ser Arg Leu Pro Leu Val Arg Gly Arg Ser
                245                 250                 255

Phe Gly Gly Asn Ser Ser Arg Pro Pro Lys Thr Pro Leu Ile Pro Gly
            260                 265                 270

Thr Arg Tyr Val Thr Ala Arg Leu Arg Leu Glu Asn Ser Thr Ser Glu
        275                 280                 285

Asp Met Cys Leu Pro Gln Trp Asn Lys Ala Glu Leu Lys Asp Ser Arg
    290                 295                 300

Arg Ile Ile Arg Ile Glu Arg Arg Tyr Gln Ser Asp Glu Ile Val Ala
305                 310                 315                 320

Ser Phe Ser Ile Val Gly Ser Ala Ile Glu Tyr Pro Lys Thr Lys Pro
                325                 330                 335

Ala Phe Asp Pro Asp Val Lys Val Leu Glu Val Ser Cys Leu Lys Cys
            340                 345                 350

Pro Thr Asn Glu Asn Glu Pro Asn Glu Glu Arg Phe Val Ser Glu Cys
        355                 360                 365

Thr Ser Asp Glu Ala Arg Thr Gln His Ala Leu Thr Glu Pro Leu Thr
    370                 375                 380

Ser Gly Phe Asn Arg Glu Pro Asn Gly His Asn Pro Lys Thr Ala Gly
385                 390                 395                 400

Asn Ser Ile Gly Cys Arg Tyr Tyr Ile Thr Ser Val Glu Val Ile Lys
                405                 410                 415

Ile Ile Glu Leu Leu Ile Gly Ser Tyr Ser Thr Ser Asp Pro Val Gln
            420                 425                 430

Arg Arg Lys Glu Arg Gly Arg Ile Arg Ser Asn Leu Val Gln Phe Trp
        435                 440                 445

Ser Lys His Leu Val Phe Ser Ser Lys Asn Ser Met Lys Arg Arg Ser
    450                 455                 460

Val Pro Thr Gly Asn Asp Ala Tyr Leu Thr Glu Leu Glu His Arg Ile
465                 470                 475                 480

Asn Thr Tyr Asp Val Arg Lys Pro Arg Leu Phe Asp Lys Ser Val Lys
                485                 490                 495

Ile Leu Glu Trp Ser Lys Leu Gly Pro Ala Leu Gln Arg Ala Ile Gln
            500                 505                 510

Ser Tyr Tyr Met Val Arg Leu Asp Asn Ser Ser Asp Ile Thr Ala Thr

515                 520                 525

Thr Asn Ser Ser
    530

<210>  99
<211>  507
<212>  PRT
<213>  Komagataella pastoris

<400>  99

Met Gln Leu Leu Ser Ala Ser Ser Ser Thr Pro Phe Val Ala Met Ser
1               5                   10                  15

Thr Val Asn Ala Gly Asn Glu Cys Tyr Leu Arg Thr Asn Asn Ser Asn
            20                  25                  30

Ser Asn Gly Ile Ser Asp Asn Arg Phe Arg Lys Lys Lys Leu Ser Glu
        35                  40                  45

Asp Leu Leu Lys Ala Asp Phe Val Glu Asp Thr Ala Gly Asn Pro Met
    50                  55                  60

Ile Tyr Pro Met Val Leu Ser Ala Pro Thr Tyr Ile Lys Thr Glu His
65                  70                  75                  80

Leu Gln Glu Leu Glu His Lys Gln Gln Gly Ser Asn Leu Leu Ser Glu
                85                  90                  95

Met Phe Thr Lys Asn Ile Phe Asn Ser His Ser Ala Met Phe Val Tyr
            100                 105                 110

Ser Glu Ile Pro Gln Ile Ser Ser Val Gln Ser Phe Ile Gly Ser Asp
        115                 120                 125

Thr Ser Val Leu Val Ala Asp Ala Ser Ser Glu Lys Asn Ser Leu Phe
    130                 135                 140

Val Tyr Ser Asn Thr Gly Ile Ser Phe Asp Pro Ser Asp Glu Asp Pro
145                 150                 155                 160

Cys Pro Tyr Glu Ala Asn Phe Asp Pro Gly Gln Leu Phe Ser Ala Val
                165                 170                 175

Ser Ala Val Glu Asp Gly Ser Met Ala Thr Leu Thr Ala Pro Thr Met
            180                 185                 190

Ser Leu Glu Gly Val Val Asn Ser Lys Lys Gly Ile Cys Gln Leu Arg

195                 200                 205

Gln His Asn Arg Ile Glu Gly Phe Asp Ser Ser His Glu Phe Lys Val
    210                 215                 220

Leu Gly Ser Met Phe Leu Asn His Ser Tyr Val Pro Leu Val Arg Gly
225                 230                 235                 240

Arg Ser Phe Gly Gly Asn Asn Cys Arg Pro Pro Lys Glu Pro Leu Ile
                245                 250                 255

Pro Gly Thr Arg Tyr Val Ala Ala His Leu Arg Leu Glu Asn Asn Thr
            260                 265                 270

Cys Glu Asp Met Cys Leu Pro Glu Trp Asn Lys His Glu Leu Glu Asp
        275                 280                 285

Ser Arg Arg Ile Ile Arg Ile Glu Arg Arg Tyr Gln Ser Asn Glu Ile
    290                 295                 300

Val Ala Ser Phe Ser Ile Val Gly Ser Ala Lys Glu Asn Pro Gln Thr
305                 310                 315                 320

Lys Pro Ser Ser Asp Pro Asp Val Lys Val Leu Glu Val Ser Cys Leu
                325                 330                 335

Lys Cys Phe Ile Asn Gly Asn Glu Ser Asp Glu Gln Phe Val Thr Glu
            340                 345                 350

Cys Gln Ser Asp Glu Ala His Tyr Gly Phe Thr Glu Leu Pro Lys Gln
        355                 360                 365

Asn Phe Ser Arg Lys Ser Lys Gly Cys Ser His Lys Met Ala Asn Asn
    370                 375                 380

Thr Ile Ala Cys Gln Tyr Tyr Ile Thr Ser Val Glu Val Ile Lys Ile
385                 390                 395                 400

Ile Glu Leu Leu Val Gly Gly Tyr Thr Val Ser Asp Pro Arg Gln Arg
                405                 410                 415

Arg Lys Glu Arg Gly Arg Val Arg Ser Asn Leu Ala Gln Phe Trp Ser
            420                 425                 430

Lys His Leu Val Ser Ser Ser Lys Lys Thr Met Lys Arg Pro Ala Met
        435                 440                 445

Leu Ala Cys Asn Glu Asp Tyr Leu Ala Glu Leu Glu Gln Arg Ile Asn
    450                 455                 460

Thr Tyr Asp Val Arg Lys Pro Arg Leu Phe Asp Lys Ser Val Lys Ile
465                 470                 475                 480

Leu Glu Trp Pro Asn Leu Gly Pro Ala Leu Gln Arg Ala Ile Gln Ser
                485                 490                 495

Tyr Tyr Met Val Gln Leu Glu Glu Ser Ser Asn
            500                 505

<210>  100
<211>  537
<212>  PRT
<213>  Komagataella pastoris

<400>  100

Met Gln Leu Phe Leu Ala Thr Leu Gly Ile Pro Phe Thr Gly Thr Leu
1               5                   10                  15

Pro Ala Ile Ala Gly Asn Glu Asp His Ser Ser Ala Asn Asn Asn Asn
            20                  25                  30

Gly Asn Gly Phe Ser Tyr Asp Trp His Gly Asn Asp Thr Leu Phe Ala
        35                  40                  45

Glu Leu Leu Glu Thr Asp Phe Phe Asp His Pro Ile Asp Asp Ala Met
    50                  55                  60

Leu Cys Ser Leu Leu Ser Pro Pro Arg Ala Ala Glu Thr Glu Gln Gln
65                  70                  75                  80

Gln Asp Thr Glu Trp Glu Gln Gln Lys His Thr Asn Leu Phe Gly Cys
                85                  90                  95

Cys Gln Lys His Lys Val Glu Met Ala Lys Gln Gln Glu Leu Tyr Leu
            100                 105                 110

Leu Pro Asp Lys Leu Thr Lys Asn Ile Phe Asn Ser Glu Ser Pro Val
        115                 120                 125

Phe Ile Cys Thr Lys Ile Pro Gln Thr Ser Ser Val Gln Ser Phe Val
    130                 135                 140

Asp Val Asp Ala Ser Leu Leu Ala Thr Asp Ser Ser Leu Ser Lys Asp
145                 150                 155                 160

Ser Phe Ser Ala Asp Ser Thr Val Asp Val Leu Phe Glu Gln Ser Val
                165                 170                 175

Gln Glu Pro Tyr Phe Tyr Arg Ser Asn Ser Ala Glu Thr Ala Pro Pro
            180                 185                 190

Ser Thr Ala Cys Ser Ser Ala Thr Leu Asp Leu Val Ser Thr Leu Glu
        195                 200                 205

Ala Thr Thr Thr Leu Phe Glu Glu Ala Ala Asn Ser Lys Lys Gln Met
    210                 215                 220

Ile Gly Leu Lys Gln Gln Ser Lys Ile Glu Asp Leu Ser Phe Ser Cys
225                 230                 235                 240

Lys Leu Asn Phe Ser Ala Ser Met Phe Leu Asp His Ser Cys Ile Pro
                245                 250                 255

Leu Val Arg Gly Arg Ser Phe Gly Gly Asn Ser Arg Pro Pro Lys Thr
            260                 265                 270

Pro Leu Ile Pro Gly Thr Arg Tyr Val Thr Ala Arg Leu Arg Leu Glu
        275                 280                 285

Asn Ser Thr Ser Glu Asp Met Cys Phe Pro His Trp Ser Glu Arg Glu
    290                 295                 300

Leu Ser Asp Ser Arg Arg Ile Ile Arg Ile Glu Arg Ser Tyr Gln Ser
305                 310                 315                 320

Asn Glu Ile Val Ala Ser Phe Phe Ile Val Gly Ser Ala Ile Asp His
                325                 330                 335

Pro Glu Thr Lys Pro Ser Ser Glu Leu Asp Val Lys Val Leu Glu Val
            340                 345                 350

Ser Cys Leu Arg Cys Phe Ile Asn Asp Asn Glu Ser Glu Glu Glu Arg
        355                 360                 365

Phe Val Ser Glu Tyr Thr Ser Asp Glu Ala Arg Ala Gln His Ala Leu
    370                 375                 380

Thr Glu Pro Leu Thr Ser Gly Leu Asn Lys Glu Pro Asn Gly His Asn
385                 390                 395                 400

Pro Lys Thr Ala Gly Asn Ser Ile Gly Cys Arg Tyr Tyr Ile Thr Ser
                405                 410                 415

Val Glu Val Ile Lys Ile Ile Glu Leu Leu Ile Gly Ser Tyr Ser Thr
            420                 425                 430

Ser Asp Pro Val Gln Arg Arg Lys Glu Arg Gly Arg Met Arg Ser Asn
        435                 440                 445

Leu Val Gln Phe Trp Ser Lys His Leu Val Ser Ser Ser Lys Asp Thr
    450                 455                 460

Met Lys Arg Arg Ser Val Pro Thr Cys Asn Asp Asp Tyr Leu Ser Glu
465                 470                 475                 480

Leu Glu His Arg Ile Asn Thr Tyr Asp Val Arg Lys Pro Arg Lys Phe
                485                 490                 495

Asp Lys Ser Val Lys Ile Leu Glu Trp Ser Lys Leu Gly Pro Ala Leu
            500                 505                 510

Gln Arg Ala Ile Gln Ser Tyr Tyr Met Val Arg Leu Asp Asn Ser Ser
        515                 520                 525

Asn Lys Thr Ala Ala Thr Asn Ser Ser
    530                 535

<210>  101
<211>  531
<212>  PRT
<213>  Komagataella pastoris

<400>  101

Met Gln Phe Tyr Leu Ala Thr Pro Thr Thr Pro Phe Val Asp Asn Leu
1               5                   10                  15

Thr Ile Pro Ser Gly Ser Gly Ala Tyr Ser Lys Ser Asn Gly Asn Asn
            20                  25                  30

Ser Ser Ser Phe Gly His Ser Tyr Asn Gly Lys Glu Glu Leu Thr Asp
        35                  40                  45

Asp Leu Leu Thr Ala Asp Phe Val Asp Pro Pro Ile Asp Asp Ala Ile
    50                  55                  60

Leu Tyr Ser Met Leu Ser Pro Phe Met Asn Ala Thr Thr Glu Gln Pro
65                  70                  75                  80

Gln Glu Phe Gln Asn Glu Gln Gln His Gln Asn Leu Ser Gly Cys Tyr
                85                  90                  95

Gln Glu Leu Leu Val Gln Thr Glu Glu Gln Gln Gln Leu Asp Leu Leu
        100                 105                 110

Pro Asn Met His Pro Glu Asn Ile Phe Asn Ser Glu Ser Ser Met Phe
    115                 120                 125

Val Tyr Ser Ser Ile Pro Gln Arg Asn Ser Val His Ser Leu Ile Ser
    130                 135                 140

Val Asn Thr Ser Ser Leu Ala Pro Asp Ser Ala Glu Gly Gly Gly Leu
145                 150                 155                 160

Ser Phe Val Asp Thr Ile Gly Pro Ser Phe Asp His Ser Val Glu Thr
                165                 170                 175

Ser Leu Ser Tyr Gly Met Asn Ser Thr Asp Ser Thr Leu Pro Ser Ser
        180                 185                 190

Ala Arg Ser Ser Val Gly Leu Asp Leu Leu Pro Thr Val Ala Ala Pro
    195                 200                 205

Ala Ser Ser Val Ser Ser Leu Glu Thr Val Ile Asn Ser Lys Arg Gln
    210                 215                 220

Lys Leu Gln Phe Arg Gln His Asn Arg Ile Glu Glu Leu Asn Ser Pro
225                 230                 235                 240

His Gly Ile Lys Ala Gln Ala Pro Val Leu Leu Asn Tyr Ser Ser Leu
                245                 250                 255

Pro Leu Val Arg Gly Leu Ser Ser Gly Gly Asn Asn Ser Arg Pro Pro
        260                 265                 270

Lys Glu Pro Leu Ile Pro Gly Ala Gln Tyr Ile Thr Ala Leu Leu Arg
    275                 280                 285

Leu Glu Asn Ser Ser Cys Glu Asp Leu Cys Leu Pro Glu Trp Asn Glu
    290                 295                 300

Asp Glu Leu Lys Asp Cys Arg Arg Ile Ile Arg Ile Glu Arg Lys Arg
305                 310                 315                 320

His Ser Asn Glu Ile Val Ala Ser Phe Ser Ile Val Gly Ser Ala Thr
                325                 330                 335

Gln Asn Pro Gln Ile Lys Pro Ala Ser Asp Ser Asp Val Gln Val Leu
        340                 345                 350

Glu Val Ser Cys Leu Gln Cys Leu Val Asn His Asn Glu Thr Asp Glu
        355                 360                 365

Gly Lys Gln Gly Ser Ser Pro Asn Glu Ile Gln His Ser Ser Ala Glu
    370                 375                 380

Pro Pro Lys Ser Asn Leu Asn Arg Thr Leu Ser Ser Ser Ser His Arg
385                 390                 395                 400

Ala Ala Gly Ser Ser Val Gly Tyr Gln Tyr Tyr Ile Thr Ser Val Glu
                405                 410                 415

Val Ile Lys Ile Ile Glu Leu Leu Val Gly Ser His Leu Ile Glu Asp
            420                 425                 430

Ser Gln Gln Lys Arg Lys Glu Arg Gly Arg Val Arg Ser Asn Leu Val
        435                 440                 445

Pro Phe Trp Ser Lys His Pro Val Ser Ser Ser Lys Lys Thr Leu Arg
    450                 455                 460

Pro Arg Ser Gly Pro Ala Cys Asn Asp Asp Tyr Leu Ala Glu Leu Ala
465                 470                 475                 480

His Arg Ile Met Thr Tyr Glu Ile Arg Arg Pro Arg Ile Phe Asp Lys
                485                 490                 495

Asn Val Arg Ile Leu Glu Trp Ala Lys Leu Ala Pro Ala Leu Gln Arg
            500                 505                 510

Ala Leu Gln Ser Tyr Tyr Val Ala Val Pro Leu Asp Glu Ser Glu Asn
        515                 520                 525

Lys Leu Val
    530

<210>  102
<211>  450
<212>  PRT
<213>  Komagataella pastoris

<400>  102

Met Leu Thr Ala Gly Ser Val Gln Arg Leu Gln Ile Ser Met Phe Gln
1               5                   10                  15

Thr Gln Glu Gln Glu Gln Val Asp Leu Ser Ser Ser Ile His Thr Glu
            20                  25                  30

Asn Ile Phe Asn Ser Glu Ser Ser Gly Pro Ala Tyr Pro Asn Ile Pro
        35                  40                  45

Gln Ile Ser Ser Val His Ser Phe Ile Gly Ala Lys Ser Ser Leu Leu
    50                  55                  60

Ala Asp Ala Ala Pro Asn Gln Cys Leu Ser Phe Val Asp Ser Ala Lys
65                  70                  75                  80

Val Thr Ser Phe Asp Gln Ser Phe His Gly Ser Pro Ser Tyr Lys Leu
                85                  90                  95

Asn Ser Ala Lys Leu Ala Val Ser Ser Ser Glu Tyr Leu Ser Val Glu
            100                 105                 110

Ser Ser Thr Glu Val Asp Ser Thr Ile Ser Leu Glu Ala Gln Val Asp
        115                 120                 125

Arg Lys Arg Gln Lys Ile Gln Pro Glu Arg His Ser Lys Thr Asp Asn
    130                 135                 140

Leu Asn Leu Phe Ser Asp Phe Asp Leu Leu Ala Pro Ala Phe Leu Thr
145                 150                 155                 160

Tyr Tyr Leu Pro Leu Phe Arg Gly Cys Ser Ser Gly Gly Asn Asn Lys
                165                 170                 175

Pro Pro Lys Lys Arg Ser Ile Thr Gly Thr Gln Tyr Val Ala Ala Ser
            180                 185                 190

Leu Arg Leu Glu Ser Ser Thr Ile Glu Asp Ile Cys Leu Pro Lys Trp
        195                 200                 205

Asn Glu Asn Glu Leu Lys Asp Asn Arg Arg Ile Ile Arg Ile Glu Arg
    210                 215                 220

Arg Gln Gln Gly Asn Glu Ile Ala Ala Ser Phe Ser Ile Val Gly Ser
225                 230                 235                 240

Ala Leu Glu Asn Pro His Thr Lys Pro Thr Leu Asp Pro Asp Val Glu
                245                 250                 255

Val Leu Glu Val Ser Cys Leu Arg Cys Leu Val Asn Tyr Asp Glu Ile
            260                 265                 270

Asp Asp Lys Gln Thr Val His Gly Ser Leu Leu His Glu Val Gln Ser

275                 280                 285

Glu Phe Thr Glu Ile Arg Lys Gln Glu Ser Thr Gly Lys Arg Ser Ser
    290                 295                 300

Tyr Ala Cys Thr Ser Asn Gly Thr Ser Ile Glu Cys Gln Tyr Tyr Val
305                 310                 315                 320

Thr Ser Val Glu Val Ile Lys Ile Ile Glu Leu Leu Val Gly Ser His
                325                 330                 335

Leu Ile Gln Asp Arg Lys Gln Arg Arg Lys Glu Arg Gly Cys Ile Arg
            340                 345                 350

Ser Asn Leu Met Pro Phe Trp Ser Lys His Leu Ile Ser Ser Thr Thr
        355                 360                 365

Asn Thr Met Lys Arg Arg Ser Val Thr Ala Ser Asn Glu Asn Tyr Ile
    370                 375                 380

Ala Glu Leu Ala His Arg Ile Lys Thr Tyr Glu Val Arg Arg Pro Arg
385                 390                 395                 400

Lys Val Asn Lys Asp Leu Arg Ile Leu Glu Trp Ser Lys Leu Gly Pro
                405                 410                 415

Ala Leu Arg Arg Ala Leu His Ser Tyr Tyr Val Ala Val Pro Ile Asp
            420                 425                 430

Lys Phe Lys Asn Ile Ala Lys Pro Thr Asn Ser Glu Leu Met Phe Asn
        435                 440                 445

Ile Gln
    450

<210>  103
<211>  414
<212>  PRT
<213>  Komagataella pastoris

<400>  103

Met Phe Gln Pro Leu Glu Gln Glu Gln Gln Leu Asp Leu Leu Phe Ala
1               5                   10                  15

Met Pro Pro Glu Asn Ile Phe Asn Ser Asp Ser Ser Ala Leu Thr Phe
            20                  25                  30

Ser Asn Thr Phe Gln Thr Ser Ser Val His Ser Val Thr Gly Val Asp

35                  40                  45

Ser Pro Ser Leu Val Asn Ile Asn Ile Pro Lys Ser Glu Ser Phe Phe
    50                  55                  60

Met Asp Ser Met Thr Gly Ala Ser Phe Gly Gln Thr Phe Gln Asp Leu
65                  70                  75                  80

Phe Pro Ala Phe Pro Leu Leu Lys Leu Pro Thr Met Val Ala Pro Pro
                85                  90                  95

Thr Ser Leu Lys Met Val Met Asp Arg Lys Arg Arg Gln Ser Gln Ile
            100                 105                 110

Thr Gln Gln Asn Thr Thr Glu Asn Leu Asn Ser Phe His Gly Met Asn
        115                 120                 125

Met Pro Val Pro Ala Phe Leu Glu Tyr Ser Ala Phe Pro Leu Val Arg
    130                 135                 140

Gly Phe Cys Ser Gly Gly Lys Ile Lys Pro Pro Lys Asn Gln Leu Val
145                 150                 155                 160

Pro Gly Thr Glu Tyr Val Asn Val Cys Leu Glu Leu Leu Asn Val Asn
                165                 170                 175

Tyr Glu Gly Ile Cys Leu Pro Lys Trp Asn Glu Lys Glu Leu Glu Glu
            180                 185                 190

Ser Arg Arg Ile Val Arg Val Glu Lys Arg Arg Gln Tyr Asn Lys Ile
        195                 200                 205

Glu Ala Ala Phe Ser Ile Val Asp Ser Val Gln Glu Asn Pro Gln Thr
    210                 215                 220

Asn Ile Ala Val Asp Pro Asn Val Asp Val Leu Glu Phe Ser Cys Leu
225                 230                 235                 240

Leu Cys Pro Ile Lys Glu His Asn Ser Glu Val Ala Gln Thr Val Pro
                245                 250                 255

Thr Ser Ser Ser Ser Glu Glu His Lys Tyr Ser Glu Leu Ala Gly His
            260                 265                 270

Asp Phe Glu Glu Asn Leu Ser Cys Gly Ser Asp Lys Glu Ile Glu Thr
        275                 280                 285

Ser Val Arg Phe Glu Tyr Tyr Ile Thr Ser Val Glu Val Ile Arg Ile
    290                 295                 300

Ile Glu Phe Leu Thr Glu Asn His Leu Ile Gln Asp Ile Gln Ala Lys
305                 310                 315                 320

Arg Arg Glu Arg Ser Cys Leu Arg Ser Asn Leu Val Arg Phe Trp Ser
                325                 330                 335

Lys Asp Ile Val Pro Ser Ser Thr Ser Thr Met Ser Lys Gly Ser Lys
            340                 345                 350

Pro Ser Arg Glu Asn Asp Cys Ile Glu Glu Leu Ala Gln Arg Ile Arg
        355                 360                 365

Ala Tyr Lys Val Arg Lys Pro Leu Ile Val Asp Lys Ser Leu Arg Ile
    370                 375                 380

Leu Glu Trp Ser Lys Leu Gly Pro Ala Leu Glu Arg Ala Leu Gln Ser
385                 390                 395                 400

Tyr Tyr Val Ala Leu Pro Leu Asp Lys Ile Lys Thr Lys Leu
                405                 410

<210>  104
<211>  531
<212>  PRT
<213>  Komagataella pastoris

<400>  104

Met Arg Leu Tyr Leu Ala Thr Val Gly Thr Pro Phe Ile Gly Ala Pro
1               5                   10                  15

Pro Val Ile Ala Ser Asn Glu Asp His Ser Thr Ala His Asn Asn Asn
            20                  25                  30

Ser Asn Asp Phe Asn Tyr Glu Cys Tyr Asn Lys Glu Lys Leu Ser Gly
        35                  40                  45

Asp Leu Leu Glu Ala Asp Leu Phe Asp Pro Pro Ile Asp Asp Thr Met
    50                  55                  60

His Cys Ser Met Phe Ser Ser Ser Thr Thr Ala Asp Thr Glu Gln Leu
65                  70                  75                  80

Gln Lys Pro Lys His Glu Ser Gln His Lys Asn Pro Phe Asp Cys Cys
                85                  90                  95

Gln Glu Leu Lys Leu Lys Met Ala Asp Gln Gln Glu Leu His Leu Leu
            100                 105                 110

Pro Glu Lys Ile Thr Lys Asn Ile Phe Asn Ser Glu Ser Pro Val Phe
        115                 120                 125

Ile Cys Thr Lys Ile Pro Gln Thr Ser Pro Val Gln Ser Phe Val Asp
    130                 135                 140

Val Asn Ala Ser Leu Leu Ala Thr Asn Ser Ala Leu Thr Thr Asn Ser
145                 150                 155                 160

Phe Ser Val Asp Thr Thr Val Asp Ile Leu Phe Glu Gln Thr Val Gln
                165                 170                 175

Asp Pro Tyr Ser Tyr Arg Leu Asn Ser Lys Glu Thr Ala Pro Pro Ser
            180                 185                 190

Thr Ala Ser Phe Phe Gly Thr Leu Asp Leu Gly Pro Thr Val Glu Ala
        195                 200                 205

Ser Thr Thr Leu Leu Glu Ala Ala Val Asn Ser Lys Arg Arg Ile Val
    210                 215                 220

Glu Leu Arg Gln Gln Asn Arg Ile Glu Asp Leu Asn Phe Ser Thr Ser
225                 230                 235                 240

Met Phe Leu Asp His Ser Arg Ile Pro Leu Val Arg Gly Arg Ser Phe
                245                 250                 255

Gly Gly Asn Ser Ser Arg Pro Pro Lys Thr Pro Leu Ile Pro Gly Thr
            260                 265                 270

Arg Tyr Val Thr Ala Arg Leu Arg Leu Glu Asn Ser Thr Ser Glu Asp
        275                 280                 285

Ile Cys Leu Pro His Trp Asn Lys Ala Glu Leu Lys Asp Ser Arg Arg
    290                 295                 300

Ile Ile Arg Ile Glu Arg Arg Tyr Gln Ser Asn Glu Ile Val Ala Thr
305                 310                 315                 320

Phe Ser Ile Val Gly Ser Ala Ile Glu His Pro Lys Thr Lys Pro Ala
                325                 330                 335

Phe Asp Pro Asp Val Lys Val Leu Glu Val Ser Cys Leu Lys Cys Pro
            340                 345                 350

Thr Asn Glu Asn Glu Pro Asn Glu Glu Arg Phe Val Ser Glu Cys Thr
        355                 360                 365

Ser Asp Glu Ala Arg Ala Gln His Ala Leu Thr Glu Pro Leu Met Ser
    370                 375                 380

Gly Phe Asn Arg Glu Pro Asn Gly Arg Asn Pro Lys Thr Ala Gly Asn
385                 390                 395                 400

Ser Ile Gly Cys Arg Tyr Tyr Ile Thr Ser Val Glu Val Ile Lys Ile
                405                 410                 415

Ile Glu Leu Leu Ile Gly Ser Tyr Ser Thr Ser Asp Pro Val Gln Arg
            420                 425                 430

Arg Lys Glu Arg Gly Arg Ile Arg Ser Asn Leu Val Gln Phe Trp Ser
        435                 440                 445

Lys His Leu Val Phe Ser Ser Lys Asn Ser Met Lys Arg Arg Ser Val
    450                 455                 460

Pro Thr Gly Asn Asp Ala Tyr Leu Thr Glu Leu Glu His Arg Ile Asn
465                 470                 475                 480

Thr Tyr Asp Val Arg Lys Pro Arg Leu Phe Asp Lys Ser Val Lys Ile
                485                 490                 495

Leu Glu Trp Ser Lys Leu Gly Pro Ala Leu Gln Arg Ala Ile Gln Ser
            500                 505                 510

Tyr Tyr Met Val Arg Leu Asp Asn Ser Ser Asp Ile Thr Ala Thr Thr
        515                 520                 525

Asn Ser Ser
    530

<210>  105
<211>  513
<212>  PRT
<213>  Komagataella pastoris

<400>  105

Met Gln Phe Tyr Leu Ala Thr Pro Thr Thr Pro Phe Val Asp Asn Leu
1               5                   10                  15

Thr Ile Pro Ser Gly Ser Gly Ala Tyr Ser Lys Ser Asn Gly Asn Asn
            20                  25                  30

Ser Ser Ser Phe Gly His Ser Tyr Asn Gly Lys Glu Glu Leu Thr Asp
        35                  40                  45

Asp Leu Leu Thr Ala Asp Phe Val Asp Pro Pro Ile Asp Asp Ala Ile
    50                  55                  60

Leu Tyr Ser Met Leu Ser Pro Phe Met Asn Ala Thr Thr Glu Gln Pro
65                  70                  75                  80

Gln Glu Phe Gln Asn Glu Gln Gln His Gln Asn Leu Ser Gly Cys Tyr
                85                  90                  95

Gln Glu Leu Leu Val Gln Thr Glu Glu Gln Gln Gln Leu Asp Leu Leu
            100                 105                 110

Pro Asn Met His Pro Glu Asn Ile Phe Asn Ser Glu Ser Ser Met Phe
        115                 120                 125

Val Tyr Ser Ser Ile Pro Gln Arg Asn Ser Val His Ser Leu Ile Ser
    130                 135                 140

Val Asn Thr Ser Ser Leu Ala Pro Asp Ser Ala Glu Gly Gly Gly Leu
145                 150                 155                 160

Ser Phe Val Asp Thr Ile Gly Pro Ser Phe Asp His Ser Val Glu Thr
                165                 170                 175

Ser Leu Ser Tyr Gly Met Asn Ser Thr Asp Ser Thr Leu Pro Ser Ser
            180                 185                 190

Ala Arg Ser Ser Val Gly Leu Asp Leu Leu Pro Thr Val Ala Ala Pro
        195                 200                 205

Ala Ser Ser Val Ser Ser Leu Glu Thr Val Ile Asn Ser Lys Arg Gln
    210                 215                 220

Lys Leu Gln Phe Arg Gln His Asn Arg Ile Glu Glu Leu Asn Ser Pro
225                 230                 235                 240

His Gly Ile Lys Ala Gln Ala Pro Val Leu Leu Asn Tyr Ser Ser Leu
                245                 250                 255

Pro Leu Val Arg Gly Leu Ser Ser Gly Gly Asn Asn Ser Arg Pro Pro
            260                 265                 270

Lys Glu Pro Leu Ile Pro Gly Ala Gln Tyr Ile Thr Ala Leu Leu Arg
        275                 280                 285

Leu Glu Asn Ser Ser Cys Glu Asp Leu Cys Leu Pro Glu Trp Asn Glu
    290                 295                 300

Asp Glu Leu Lys Asp Cys Arg Arg Ile Ile Arg Ile Glu Arg Lys Arg
305                 310                 315                 320

His Ser Asn Glu Ile Val Ala Asp Ser Asp Val Gln Val Leu Glu Val
                325                 330                 335

Ser Cys Leu Gln Cys Leu Val Asn His Asn Glu Thr Asp Glu Gly Lys
            340                 345                 350

Gln Gly Ser Ser Pro Asn Glu Ile Gln His Ser Ser Ala Glu Pro Pro
        355                 360                 365

Lys Ser Asn Leu Asn Arg Thr Leu Ser Ser Ser Ser His Arg Ala Ala
    370                 375                 380

Gly Ser Ser Val Gly Tyr Gln Tyr Tyr Ile Thr Ser Val Glu Val Ile
385                 390                 395                 400

Lys Ile Ile Glu Leu Leu Val Gly Ser His Leu Ile Glu Asp Ser Gln
                405                 410                 415

Gln Lys Arg Lys Glu Arg Gly Arg Val Arg Ser Asn Leu Val Pro Phe
            420                 425                 430

Trp Ser Lys His Pro Val Ser Ser Ser Lys Lys Thr Leu Arg Pro Arg
        435                 440                 445

Ser Gly Pro Ala Cys Asn Asp Asp Tyr Leu Ala Glu Leu Ala His Arg
    450                 455                 460

Ile Met Thr Tyr Glu Ile Arg Arg Pro Arg Ile Phe Asp Lys Asn Val
465                 470                 475                 480

Arg Ile Leu Glu Trp Ala Lys Leu Ala Pro Ala Leu Gln Arg Ala Leu
                485                 490                 495

Gln Ser Tyr Tyr Val Ala Val Pro Leu Asp Glu Ser Glu Asn Lys Leu
            500                 505                 510

Val

<210>  106

<211>  521
<212>  PRT
<213>  Komagataella pastoris

<400>  106

Met Leu Thr Ala Gly Ser Val Gln Arg Leu Gln Ile Ser Met Phe Gln
1               5                   10                  15

Thr Gln Glu Gln Glu Gln Val Asp Leu Ser Ser Ser Ile His Thr Glu
            20                  25                  30

Asn Ile Phe Asn Ser Glu Ser Ser Gly Pro Ala Tyr Pro Asn Ile Pro
        35                  40                  45

Gln Ile Ser Ser Val His Ser Phe Ile Gly Ala Lys Ser Ser Leu Leu
    50                  55                  60

Ala Asp Ala Ala Pro Asn Gln Cys Leu Ser Phe Val Asp Ser Ala Lys
65                  70                  75                  80

Val Thr Ser Phe Asp Gln Ser Phe His Gly Ser Pro Ser Tyr Lys Leu
                85                  90                  95

Asn Ser Ala Lys Leu Ala Val Ser Ser Ser Glu Tyr Leu Ser Val Glu
            100                 105                 110

Ser Ser Thr Glu Val Asp Ser Thr Ile Ser Leu Glu Ala Gln Val Asp
        115                 120                 125

Arg Lys Arg Gln Lys Ile Gln Pro Glu Arg His Ser Lys Thr Asp Asn
    130                 135                 140

Leu Asn Leu Phe Ser Asp Phe Asp Leu Leu Ala Pro Ala Phe Leu Thr
145                 150                 155                 160

Tyr Tyr Leu Pro Leu Phe Arg Gly Cys Ser Ser Gly Gly Asn Asn Lys
                165                 170                 175

Pro Pro Lys Lys Arg Ser Ile Thr Gly Thr Gln Tyr Val Ala Ala Ser
            180                 185                 190

Leu Arg Leu Glu Ser Ser Thr Ile Glu Asp Ile Cys Leu Pro Lys Trp
        195                 200                 205

Asn Glu Asn Glu Leu Lys Asp Asn Arg Arg Ile Ile Arg Ile Glu Arg
    210                 215                 220

Arg Gln Gln Gly Asn Glu Ile Ala Ala Ser Phe Ser Ile Val Gly Ser

225                 230                 235                 240

Ala Leu Glu Asn Pro His Thr Lys Pro Thr Leu Asp Pro Asp Val Glu
                245                 250                 255

Val Leu Glu Val Ser Cys Leu Arg Cys Leu Val Asn Tyr Asp Glu Ile
            260                 265                 270

Asp Asp Lys Gln Thr Val His Gly Ser Leu Leu His Glu Val Gln Ser
        275                 280                 285

Glu Phe Thr Glu Ile Arg Lys Gln Glu Ser Thr Gly Lys Arg Ser Ser
    290                 295                 300

Tyr Ala Cys Thr Ser Asn Gly Thr Ser Ile Glu Cys Gln Tyr Tyr Val
305                 310                 315                 320

Thr Ser Val Glu Val Ile Lys Ile Ile Glu Leu Leu Val Gly Ser His
                325                 330                 335

Leu Ile Gln Asp Arg Lys Gln Arg Arg Lys Glu Arg Gly Cys Ile Arg
            340                 345                 350

Ser Asn Leu Met Pro Phe Trp Ser Lys His Leu Ile Ser Ser Thr Thr
        355                 360                 365

Asn Thr Met Lys Arg Arg Ser Val Thr Ala Ser Asn Glu Asn Tyr Ile
    370                 375                 380

Ala Glu Leu Ala His Arg Ile Lys Thr Tyr Glu Val Arg Arg Pro Arg
385                 390                 395                 400

Lys Val Asn Lys Asp Leu Arg Ile Leu Glu Trp Ser Lys Leu Gly Pro
                405                 410                 415

Ala Leu Arg Arg Ala Leu His Ser Tyr Tyr Val Ala Val Pro Ile Asp
            420                 425                 430

Lys Phe Lys Asn Ile Ala Lys Pro Thr Asn Thr Ser Leu Gly Thr Ile
        435                 440                 445

Lys Met Ala Ile Ala Gly Arg Ser Glu Val Leu Glu Thr Val Phe Ile
    450                 455                 460

Ala Arg Gln Ser Pro Val Glu Pro Asp Ala Cys Ser Ser Ile Ala Asn
465                 470                 475                 480

Val Arg Arg Glu Leu Cys Asp Ala Cys Thr Gly Thr Gln Ala Asn Ile
                485                 490                 495

Val Thr Glu Lys Ile Leu Leu Thr Thr Ile Ala Ala Asp Ala Gly Ser
            500                 505                 510

Thr Ile Val Val Pro Val Cys Val Pro
        515                 520

<210> 107
<211> 524
<212> PRT
<213> Komagataella pastoris

<400> 107

Met Arg Leu Ser Leu Ala Thr Arg Glu Thr Pro Ser Thr Gly Thr Ser
1               5                   10                  15

Pro Val Phe Ala Gly Asp Glu Asp His Ser Ser Ala Asn Asn Ser Ser
            20                  25                  30

Ser Asn Thr Phe Asn Tyr Asp Tyr Tyr Ile Lys Glu Glu Leu Ser Gly
        35                  40                  45

Asn Leu Leu Glu Thr Ala Phe Phe Glu Ser Pro Ile Asn Asp Thr Met
    50                  55                  60

Leu Cys Ser Met Leu Ser Tyr Pro Lys Ile Ala Gly Thr Glu Lys Ile
65                  70                  75                  80

Gln Glu Pro Asp His Glu Leu Gln Gln His Arg Asn Pro Phe Gly Cys
                85                  90                  95

Cys Gln Glu Leu Lys Ala Glu Leu Ala Glu Gln Gln Glu Leu Tyr Leu
            100                 105                 110

Leu Pro Asn Lys Leu Thr Lys Asn Ile Phe Asn Ser Gly Ser Pro Val
        115                 120                 125

Phe Ile Tyr Thr Lys Ile Pro Gln Thr Ser Ser Val Gln Ser Phe Val
    130                 135                 140

Asp Val Ser Asn Ser Leu Phe Ala Thr Asp Ser Ala Leu Pro Thr Ser
145                 150                 155                 160

Ser Phe Arg Val Asp Ser Thr Val Asp Ile Ser Phe Glu His Ser Ile
                165                 170                 175

Gln Asp Ser Tyr Cys Tyr Arg Ser Asn Ser Ala Glu Ile Ala Pro Pro
            180                 185                 190

Ser Thr Ala Leu Ser Ser Gly Thr His Asp Leu Val Ser Thr Val Glu
        195                 200                 205

Ala Ser Thr Thr Leu Leu Glu Ala Ala Val Asn Ser Lys Arg Arg Met
    210                 215                 220

Leu Glu Leu Arg Gln Gln Asn Arg Ile Glu Asp Leu Ser Phe Ser Thr
225                 230                 235                 240

Ser Ile Phe Leu Asp His Ser Arg Leu Pro Leu Val Arg Gly Arg Ser
                245                 250                 255

Phe Gly Gly Asn Ser Ser Arg Pro Pro Lys Thr Pro Leu Ile Pro Gly
            260                 265                 270

Thr Arg Tyr Val Thr Ala Arg Leu Arg Leu Glu Asn Ser Thr Ser Glu
        275                 280                 285

Asp Met Cys Leu Pro Gln Trp Asn Lys Ala Glu Leu Lys Asp Ser Arg
    290                 295                 300

Arg Ile Ile Arg Ile Glu Arg Arg Tyr Gln Ser Asp Glu Ile Val Ala
305                 310                 315                 320

Ser Phe Ser Ile Val Gly Ser Ala Ile Glu Tyr Pro Lys Thr Lys Pro
                325                 330                 335

Ala Phe Asp Pro Asp Val Lys Val Leu Glu Val Ser Cys Leu Lys Cys
            340                 345                 350

Pro Thr Asn Glu Asn Glu Pro Asn Glu Glu Arg Phe Val Ser Glu Cys
        355                 360                 365

Thr Ser Asp Glu Ala Arg Thr Gln His Ala Leu Thr Glu Pro Leu Thr
    370                 375                 380

Ser Gly Phe Asn Arg Glu Pro Asn Gly His Asn Pro Lys Thr Ala Gly
385                 390                 395                 400

Asn Ser Ile Gly Cys Arg Tyr Tyr Ile Thr Ser Val Glu Val Ile Lys
                405                 410                 415

Ile Ile Glu Leu Leu Ile Gly Ser Tyr Ser Thr Ser Asp Pro Val Gln
            420                 425                 430

Arg Arg Lys Glu Arg Gly Arg Ile Arg Ser Asn Leu Val Gln Phe Trp
        435                 440                 445

Ser Lys His Leu Val Phe Ser Ser Lys Asn Ser Met Lys Arg Arg Ser
    450                 455                 460

Val Pro Thr Gly Asn Asp Ala Tyr Leu Thr Glu Leu Glu His Arg Ile
465                 470                 475                 480

Asn Thr Tyr Asp Val Arg Lys Pro Arg Leu Phe Asp Lys Ser Val Lys
                485                 490                 495

Ile Leu Glu Trp Ser Lys Leu Gly Pro Ala Leu Arg Arg Ala Ile Gln
            500                 505                 510

Ser Tyr Tyr Met Val Arg Leu Asp Glu Ser Ser Asn
        515                 520

<210>  108
<211>  368
<212>  PRT
<213>  Artificial

<220>
<223>  Synthetic

<400>  108

Met Leu Ala Glu Ile Gly Asp Tyr Phe Arg Thr Asn Ser Ser Asn Gly
1               5                   10                  15

Phe Ser Ser Ser Cys Tyr Asn Lys Glu Lys Leu Ser Gly Asp Leu Leu
            20                  25                  30

Glu Ser Asp Phe Val Asn Ser Ser Phe Asp Asp Thr Thr Leu His Ser
        35                  40                  45

Met Leu Ser Ser Ser Ile Asn Ala Asp Pro Glu Gln Ser Gln Lys Pro
    50                  55                  60

Gln Gln Arg His Lys Asn Gln Ser Ser Cys His Gln Glu Leu Lys Ile
65                  70                  75                  80

Glu Thr Glu Asp Gln Gln Glu Pro Tyr Leu Leu Pro Asp Met Leu Thr
                85                  90                  95

Lys Asn Ile Phe Asn Ser Gln Ser Ser Met Phe Val Tyr Ser Ser Ile
            100                 105                 110

Pro Gln Ile Ser Ser Val Gln Ser Phe Ile Asp Thr Asn Thr Phe Ser
        115                 120                 125

Ala Val Asp Gly Gly Ala Ala Pro Glu Gly Asn Ser Leu Ser Val Asp
    130                 135                 140

Ser Thr Leu Gly Leu Ser Ser Asp Gln Ser Phe Glu Lys Leu Tyr Ser
145                 150                 155                 160

Tyr Asp Gln Asn Phe Glu Lys Glu Pro Gln Ser Pro Ala Cys Pro Leu
                165                 170                 175

Val Lys Cys Asp Pro Val Ser Ile Met Lys Thr Ser Thr Asn Ser Leu
            180                 185                 190

Glu Thr Ala Val Asn Thr Lys Arg Arg Met Phe Gln Leu Arg Asn Gln
        195                 200                 205

Asn Glu Ile Glu Ser Leu Asn Val Ser Ser Asp Ile Val Val Ala Thr
    210                 215                 220

Ser Lys Phe Leu Asn His Ser Trp Ile Pro Leu Ile Arg Gly Arg Asn
225                 230                 235                 240

Phe Gly Gly Ser Asn Cys Lys Pro Pro Lys Lys Pro Leu Phe Pro Gly
                245                 250                 255

Ala Arg Tyr Val Thr Ala Arg Leu Arg Leu Glu Tyr Ser Ser Cys Lys
            260                 265                 270

Asp Met Cys Leu Pro Glu Trp Asn Glu Tyr Glu Leu Glu Asp Ser Arg
        275                 280                 285

Arg Ile Ile Arg Ile Glu Arg Leu Phe Asp Ser Asn Glu Ile Val Ala
    290                 295                 300

Ser Phe Ser Ile Val Gly Ser Ala Val Glu Asn Pro Glu Thr Arg Pro
305                 310                 315                 320

Val Phe Asn Pro Asn Val Lys Val Leu Glu Val Ser Cys Leu Arg Cys
                325                 330                 335

Leu Thr Asn Asn Asn Glu Ser Asp Glu Glu Asn Cys Val Asn Lys Asp
            340                 345                 350

Leu Asp Gly Arg Asn Arg Asp Met Val Lys Asp Ser Phe Gly Cys Lys
        355                 360                 365

<210>  109
<211>  400
<212>  PRT
<213>  Artificial

<220>
<223>  Synthetic

<400>  109

Met Asn Ser Ser Ser Thr Arg Gly Leu Gln Ala Ile Ser Leu Thr Pro
1               5                   10                  15

Thr Arg Val Leu Phe Thr Leu Ser Thr Leu Phe Ala Asp Thr Ser Lys
            20                  25                  30

Met Ser Ala Glu Asn Gly Asp Tyr Phe Arg Thr Asn Ser Ser Asn Glu
        35                  40                  45

Phe Ser Asn Asn Cys Phe Asn Lys Glu Lys Leu Ser Glu Asp Leu Leu
    50                  55                  60

Glu Ala Ser Phe Val Asp Ser Pro Ile Glu Asp Thr Ile Pro His Phe
65                  70                  75                  80

Met Leu Ser Ser Ser Ile Asn Ala Asp Ser Glu Gln Pro Gln Glu Pro
                85                  90                  95

Gln His Arg His Lys Asn Gln Ser Ser Cys His Arg Glu Leu Lys Ile
            100                 105                 110

Glu Thr Asn Asp Gln Gln Glu Pro Tyr Leu Leu Pro Asp Met Leu Thr
        115                 120                 125

Lys Asn Ile Phe Asn Ser Gln Ser Ser Met Phe Val Tyr Ser Ser Ile
    130                 135                 140

Pro Gln Thr Ser Ser Val Gln Ser Phe Ile Asp Thr Asn Ile Phe Ser
145                 150                 155                 160

Ala Val Asp Gly Gly Ala Ala Pro Glu Glu Asn Ser Leu Ser Val Asp
                165                 170                 175

Ser Thr Leu Gly Pro Ser Ser Asp Gln Ser Phe Glu Lys Leu Tyr Ser
            180                 185                 190

Tyr Asp Gln Asn Phe Glu Lys Glu Pro Gln Ser Pro Ala Cys Pro Leu
        195                 200                 205

Val Lys Cys Asp Leu Val Gly Ile Met Lys Ala Ser Thr Asn Ser Leu
    210                 215                 220

Glu Val Ala Val Asn Thr Lys Arg Gln Met Phe Gln Leu Arg His Gln
225                 230                 235                 240

Asn Glu Ile Glu Ser Leu Tyr Val Ser Ser Asp Ile Val Ile Ala Thr
                245                 250                 255

Ser Asn Phe Leu Asp His Ser Trp Ile Pro Leu Ile Arg Gly Arg Asn
            260                 265                 270

Phe Gly Gly Ser Asn Cys Lys Pro Pro Lys Lys Pro Leu Phe Pro Gly
        275                 280                 285

Ala Arg Tyr Val Thr Ala Arg Leu Arg Leu Glu Tyr Ser Ser Cys Glu
    290                 295                 300

Asp Met Cys Leu Pro Glu Trp Asn Glu Tyr Glu Leu Lys Asp Ser Arg
305                 310                 315                 320

Arg Ile Ile Arg Leu Glu Arg Arg Phe Asp Ser Asn Glu Ile Val Ala
                325                 330                 335

Ser Phe Ser Ile Val Gly Ser Ala Val Glu Asn Pro Glu Thr Arg Pro
            340                 345                 350

Ala Phe Asn Pro Asn Val Lys Val Leu Glu Val Ser Cys Leu Arg Cys
        355                 360                 365

Leu Thr Asn Asn Asn Glu Ser Asp Glu Glu Asn Cys Val Asn Lys Asp
    370                 375                 380

Leu Asp Gly Arg Val His Asn Met Val Lys Asp Ser Ile Gly Cys Lys
385                 390                 395                 400

<210>  110
<211>  405
<212>  PRT
<213>  Artificial

<220>
<223>  Synthetic

<400>  110

Met Arg Leu Tyr Leu Ala Thr Val Gly Thr Pro Phe Ile Gly Ala Pro
1               5                   10                  15

Pro Val Ile Ala Ser Asn Glu Asp His Ser Thr Ala His Asn Asn Asn

20                  25                  30

Ser Asn Asp Phe Asn Tyr Glu Cys Tyr Asn Lys Glu Lys Leu Ser Gly
        35                  40                  45

Asp Leu Leu Glu Ala Asp Leu Phe Asp Pro Pro Ile Asp Asp Thr Met
    50                  55                  60

His Cys Ser Met Phe Ser Ser Ser Thr Thr Ala Asp Thr Glu Gln Leu
65                  70                  75                  80

Gln Lys Pro Lys His Glu Ser Gln His Lys Asn Pro Phe Asp Cys Cys
                85                  90                  95

Gln Glu Leu Lys Leu Lys Met Ala Asp Gln Gln Glu Leu His Leu Leu
            100                 105                 110

Pro Glu Lys Ile Thr Lys Asn Ile Phe Asn Ser Glu Ser Pro Val Phe
        115                 120                 125

Ile Cys Thr Lys Ile Pro Gln Thr Ser Pro Val Gln Ser Phe Val Asp
    130                 135                 140

Val Asn Ala Ser Leu Leu Ala Thr Asn Ser Ala Leu Thr Thr Asn Ser
145                 150                 155                 160

Phe Ser Val Asp Thr Thr Val Asp Ile Leu Phe Glu Gln Thr Val Gln
                165                 170                 175

Asp Pro Tyr Ser Tyr Arg Leu Asn Ser Lys Glu Thr Ala Pro Pro Ser
            180                 185                 190

Thr Ala Ser Phe Phe Gly Thr Leu Asp Leu Gly Pro Thr Val Glu Ala
        195                 200                 205

Ser Thr Thr Leu Leu Glu Ala Ala Val Asn Ser Lys Arg Arg Ile Val
    210                 215                 220

Glu Leu Arg Gln Gln Asn Arg Ile Glu Asp Leu Asn Phe Ser Thr Ser
225                 230                 235                 240

Met Phe Leu Asp His Ser Arg Ile Pro Leu Val Arg Gly Arg Ser Phe
                245                 250                 255

Gly Gly Asn Ser Ser Arg Pro Pro Lys Thr Pro Leu Ile Pro Gly Thr
            260                 265                 270

Arg Tyr Val Thr Ala Arg Leu Arg Leu Glu Asn Ser Thr Ser Glu Asp
        275                 280                 285

Ile Cys Leu Pro His Trp Asn Lys Ala Glu Leu Lys Asp Ser Arg Arg
    290                 295                 300

Ile Ile Arg Ile Glu Arg Arg Tyr Gln Ser Asn Glu Ile Val Ala Thr
305                 310                 315                 320

Phe Ser Ile Val Gly Ser Ala Ile Glu His Pro Lys Thr Lys Pro Ala
                325                 330                 335

Phe Asp Pro Asp Val Lys Val Leu Glu Val Ser Cys Leu Lys Cys Pro
            340                 345                 350

Thr Asn Glu Asn Glu Pro Asn Glu Glu Arg Phe Val Ser Glu Cys Thr
        355                 360                 365

Ser Asp Glu Ala Arg Ala Gln His Ala Leu Thr Glu Pro Leu Met Ser
    370                 375                 380

Gly Phe Asn Arg Glu Pro Asn Gly Arg Asn Pro Lys Thr Ala Gly Asn
385                 390                 395                 400

Ser Ile Gly Cys Arg
                405

<210>  111
<211>  406
<212>  PRT
<213>  Artificial

<220>
<223>  Synthetic

<400>  111

Met Arg Leu Ser Leu Ala Thr Arg Glu Thr Pro Ser Thr Gly Thr Ser
1               5                   10                  15

Pro Val Phe Ala Gly Asp Glu Asp His Ser Ser Ala Asn Asn Ser Ser
            20                  25                  30

Ser Asn Thr Phe Asn Tyr Asp Tyr Tyr Ile Lys Glu Glu Leu Ser Gly
        35                  40                  45

Asn Leu Leu Glu Thr Ala Phe Phe Glu Ser Pro Ile Asn Asp Thr Met
    50                  55                  60

Leu Cys Ser Met Leu Ser Tyr Pro Lys Ile Ala Gly Thr Glu Lys Ile

65                  70                  75                  80

Gln Glu Pro Asp His Glu Leu Gln Gln His Arg Asn Pro Phe Gly Cys
                85                  90                  95

Cys Gln Glu Leu Lys Ala Glu Leu Ala Glu Gln Gln Glu Leu Tyr Leu
            100                 105                 110

Leu Pro Asn Lys Leu Thr Lys Asn Ile Phe Asn Ser Gly Ser Pro Val
        115                 120                 125

Phe Ile Tyr Thr Lys Ile Pro Gln Thr Ser Ser Val Gln Ser Phe Val
    130                 135                 140

Asp Val Ser Asn Ser Leu Phe Ala Thr Asp Ser Ala Leu Pro Thr Ser
145                 150                 155                 160

Ser Phe Arg Val Asp Ser Thr Val Asp Ile Ser Phe Glu His Ser Ile
                165                 170                 175

Gln Asp Ser Tyr Cys Tyr Arg Ser Asn Ser Ala Glu Ile Ala Pro Pro
            180                 185                 190

Ser Thr Ala Leu Ser Ser Gly Thr His Asp Leu Val Ser Thr Val Glu
        195                 200                 205

Ala Ser Thr Thr Leu Leu Glu Ala Ala Val Asn Ser Lys Arg Arg Met
    210                 215                 220

Leu Glu Leu Arg Gln Gln Asn Arg Ile Glu Asp Leu Ser Phe Ser Thr
225                 230                 235                 240

Ser Ile Phe Leu Asp His Ser Arg Leu Pro Leu Val Arg Gly Arg Ser
                245                 250                 255

Phe Gly Gly Asn Ser Ser Arg Pro Pro Lys Thr Pro Leu Ile Pro Gly
            260                 265                 270

Thr Arg Tyr Val Thr Ala Arg Leu Arg Leu Glu Asn Ser Thr Ser Glu
        275                 280                 285

Asp Met Cys Leu Pro Gln Trp Asn Lys Ala Glu Leu Lys Asp Ser Arg
    290                 295                 300

Arg Ile Ile Arg Ile Glu Arg Arg Tyr Gln Ser Asp Glu Ile Val Ala
305                 310                 315                 320

Ser Phe Ser Ile Val Gly Ser Ala Ile Glu Tyr Pro Lys Thr Lys Pro
                325                 330                 335

Ala Phe Asp Pro Asp Val Lys Val Leu Glu Val Ser Cys Leu Lys Cys
            340                 345                 350

Pro Thr Asn Glu Asn Glu Pro Asn Glu Glu Arg Phe Val Ser Glu Cys
        355                 360                 365

Thr Ser Asp Glu Ala Arg Thr Gln His Ala Leu Thr Glu Pro Leu Thr
    370                 375                 380

Ser Gly Phe Asn Arg Glu Pro Asn Gly His Asn Pro Lys Thr Ala Gly
385                 390                 395                 400

Asn Ser Ile Gly Cys Arg
                405

<210>  112
<211>  389
<212>  PRT
<213>  Artificial

<220>
<223>  Synthetic

<400>  112

Met Gln Leu Leu Ser Ala Ser Ser Ser Thr Pro Phe Val Ala Met Ser
1               5                   10                  15

Thr Val Asn Ala Gly Asn Glu Cys Tyr Leu Arg Thr Asn Asn Ser Asn
            20                  25                  30

Ser Asn Gly Ile Ser Asp Asn Arg Phe Arg Lys Lys Lys Leu Ser Glu
        35                  40                  45

Asp Leu Leu Lys Ala Asp Phe Val Glu Asp Thr Ala Gly Asn Pro Met
    50                  55                  60

Ile Tyr Pro Met Val Leu Ser Ala Pro Thr Tyr Ile Lys Thr Glu His
65                  70                  75                  80

Leu Gln Glu Leu Glu His Lys Gln Gln Gly Ser Asn Leu Leu Ser Glu
                85                  90                  95

Met Phe Thr Lys Asn Ile Phe Asn Ser His Ser Ala Met Phe Val Tyr
            100                 105                 110

Ser Glu Ile Pro Gln Ile Ser Ser Val Gln Ser Phe Ile Gly Ser Asp

115                 120                 125

Thr Ser Val Leu Val Ala Asp Ala Ser Ser Glu Lys Asn Ser Leu Phe
    130                 135                 140

Val Tyr Ser Asn Thr Gly Ile Ser Phe Asp Pro Ser Asp Glu Asp Pro
145                 150                 155                 160

Cys Pro Tyr Glu Ala Asn Phe Asp Pro Gly Gln Leu Phe Ser Ala Val
                165                 170                 175

Ser Ala Val Glu Asp Gly Ser Met Ala Thr Leu Thr Ala Pro Thr Met
            180                 185                 190

Ser Leu Glu Gly Val Val Asn Ser Lys Lys Gly Ile Cys Gln Leu Arg
        195                 200                 205

Gln His Asn Arg Ile Glu Gly Phe Asp Ser Ser His Glu Phe Lys Val
    210                 215                 220

Leu Gly Ser Met Phe Leu Asn His Ser Tyr Val Pro Leu Val Arg Gly
225                 230                 235                 240

Arg Ser Phe Gly Gly Asn Asn Cys Arg Pro Pro Lys Glu Pro Leu Ile
                245                 250                 255

Pro Gly Thr Arg Tyr Val Ala Ala His Leu Arg Leu Glu Asn Asn Thr
            260                 265                 270

Cys Glu Asp Met Cys Leu Pro Glu Trp Asn Lys His Glu Leu Glu Asp
        275                 280                 285

Ser Arg Arg Ile Ile Arg Ile Glu Arg Arg Tyr Gln Ser Asn Glu Ile
    290                 295                 300

Val Ala Ser Phe Ser Ile Val Gly Ser Ala Lys Glu Asn Pro Gln Thr
305                 310                 315                 320

Lys Pro Ser Ser Asp Pro Asp Val Lys Val Leu Glu Val Ser Cys Leu
                325                 330                 335

Lys Cys Phe Ile Asn Gly Asn Glu Ser Asp Glu Gln Phe Val Thr Glu
            340                 345                 350

Cys Gln Ser Asp Glu Ala His Tyr Gly Phe Thr Glu Leu Pro Lys Gln
        355                 360                 365

Asn Phe Ser Arg Lys Ser Lys Gly Cys Ser His Lys Met Ala Asn Asn
    370                 375                 380

Thr Ile Ala Cys Gln
385

<210>  113
<211>  411
<212>  PRT
<213>  Artificial

<220>
<223>  Synthetic

<400>  113

Met Gln Leu Phe Leu Ala Thr Leu Gly Ile Pro Phe Thr Gly Thr Leu
1               5                   10                  15

Pro Ala Ile Ala Gly Asn Glu Asp His Ser Ser Ala Asn Asn Asn Asn
            20                  25                  30

Gly Asn Gly Phe Ser Tyr Asp Trp His Gly Asn Asp Thr Leu Phe Ala
        35                  40                  45

Glu Leu Leu Glu Thr Asp Phe Phe Asp His Pro Ile Asp Asp Ala Met
    50                  55                  60

Leu Cys Ser Leu Leu Ser Pro Pro Arg Ala Ala Glu Thr Glu Gln Gln
65                  70                  75                  80

Gln Asp Thr Glu Trp Glu Gln Gln Lys His Thr Asn Leu Phe Gly Cys
                85                  90                  95

Cys Gln Lys His Lys Val Glu Met Ala Lys Gln Gln Glu Leu Tyr Leu
            100                 105                 110

Leu Pro Asp Lys Leu Thr Lys Asn Ile Phe Asn Ser Glu Ser Pro Val
        115                 120                 125

Phe Ile Cys Thr Lys Ile Pro Gln Thr Ser Ser Val Gln Ser Phe Val
    130                 135                 140

Asp Val Asp Ala Ser Leu Leu Ala Thr Asp Ser Ser Leu Ser Lys Asp
145                 150                 155                 160

Ser Phe Ser Ala Asp Ser Thr Val Asp Val Leu Phe Glu Gln Ser Val
                165                 170                 175

Gln Glu Pro Tyr Phe Tyr Arg Ser Asn Ser Ala Glu Thr Ala Pro Pro

180                 185                 190

Ser Thr Ala Cys Ser Ser Ala Thr Leu Asp Leu Val Ser Thr Leu Glu
        195                 200                 205

Ala Thr Thr Thr Leu Phe Glu Glu Ala Ala Asn Ser Lys Lys Gln Met
    210                 215                 220

Ile Gly Leu Lys Gln Gln Ser Lys Ile Glu Asp Leu Ser Phe Ser Cys
225                 230                 235                 240

Lys Leu Asn Phe Ser Ala Ser Met Phe Leu Asp His Ser Cys Ile Pro
                245                 250                 255

Leu Val Arg Gly Arg Ser Phe Gly Gly Asn Ser Arg Pro Pro Lys Thr
            260                 265                 270

Pro Leu Ile Pro Gly Thr Arg Tyr Val Thr Ala Arg Leu Arg Leu Glu
        275                 280                 285

Asn Ser Thr Ser Glu Asp Met Cys Phe Pro His Trp Ser Glu Arg Glu
    290                 295                 300

Leu Ser Asp Ser Arg Arg Ile Ile Arg Ile Glu Arg Ser Tyr Gln Ser
305                 310                 315                 320

Asn Glu Ile Val Ala Ser Phe Phe Ile Val Gly Ser Ala Ile Asp His
                325                 330                 335

Pro Glu Thr Lys Pro Ser Ser Glu Leu Asp Val Lys Val Leu Glu Val
            340                 345                 350

Ser Cys Leu Arg Cys Phe Ile Asn Asp Asn Glu Ser Glu Glu Glu Arg
        355                 360                 365

Phe Val Ser Glu Tyr Thr Ser Asp Glu Ala Arg Ala Gln His Ala Leu
    370                 375                 380

Thr Glu Pro Leu Thr Ser Gly Leu Asn Lys Glu Pro Asn Gly His Asn
385                 390                 395                 400

Pro Lys Thr Ala Gly Asn Ser Ile Gly Cys Arg
                405                 410

<210>  114
<211>  409
<212>  PRT
<213>  Artificial

<220>
<223>  Synthetic

<400>  114

Met Gln Phe Tyr Leu Ala Thr Pro Thr Thr Pro Phe Val Asp Asn Leu
1               5                   10                  15

Thr Ile Pro Ser Gly Ser Gly Ala Tyr Ser Lys Ser Asn Gly Asn Asn
            20                  25                  30

Ser Ser Ser Phe Gly His Ser Tyr Asn Gly Lys Glu Glu Leu Thr Asp
        35                  40                  45

Asp Leu Leu Thr Ala Asp Phe Val Asp Pro Pro Ile Asp Asp Ala Ile
    50                  55                  60

Leu Tyr Ser Met Leu Ser Pro Phe Met Asn Ala Thr Thr Glu Gln Pro
65                  70                  75                  80

Gln Glu Phe Gln Asn Glu Gln Gln His Gln Asn Leu Ser Gly Cys Tyr
                85                  90                  95

Gln Glu Leu Leu Val Gln Thr Glu Glu Gln Gln Gln Leu Asp Leu Leu
            100                 105                 110

Pro Asn Met His Pro Glu Asn Ile Phe Asn Ser Glu Ser Ser Met Phe
        115                 120                 125

Val Tyr Ser Ser Ile Pro Gln Arg Asn Ser Val His Ser Leu Ile Ser
    130                 135                 140

Val Asn Thr Ser Ser Leu Ala Pro Asp Ser Ala Glu Gly Gly Gly Leu
145                 150                 155                 160

Ser Phe Val Asp Thr Ile Gly Pro Ser Phe Asp His Ser Val Glu Thr
                165                 170                 175

Ser Leu Ser Tyr Gly Met Asn Ser Thr Asp Ser Thr Leu Pro Ser Ser
            180                 185                 190

Ala Arg Ser Ser Val Gly Leu Asp Leu Leu Pro Thr Val Ala Ala Pro
        195                 200                 205

Ala Ser Ser Val Ser Ser Leu Glu Thr Val Ile Asn Ser Lys Arg Gln
    210                 215                 220

Lys Leu Gln Phe Arg Gln His Asn Arg Ile Glu Glu Leu Asn Ser Pro

225                 230                 235                 240

His Gly Ile Lys Ala Gln Ala Pro Val Leu Leu Asn Tyr Ser Ser Leu
                245                 250                 255

Pro Leu Val Arg Gly Leu Ser Ser Gly Gly Asn Asn Ser Arg Pro Pro
            260                 265                 270

Lys Glu Pro Leu Ile Pro Gly Ala Gln Tyr Ile Thr Ala Leu Leu Arg
        275                 280                 285

Leu Glu Asn Ser Ser Cys Glu Asp Leu Cys Leu Pro Glu Trp Asn Glu
    290                 295                 300

Asp Glu Leu Lys Asp Cys Arg Arg Ile Ile Arg Ile Glu Arg Lys Arg
305                 310                 315                 320

His Ser Asn Glu Ile Val Ala Ser Phe Ser Ile Val Gly Ser Ala Thr
                325                 330                 335

Gln Asn Pro Gln Ile Lys Pro Ala Ser Asp Ser Asp Val Gln Val Leu
            340                 345                 350

Glu Val Ser Cys Leu Gln Cys Leu Val Asn His Asn Glu Thr Asp Glu
        355                 360                 365

Gly Lys Gln Gly Ser Ser Pro Asn Glu Ile Gln His Ser Ser Ala Glu
    370                 375                 380

Pro Pro Lys Ser Asn Leu Asn Arg Thr Leu Ser Ser Ser Ser His Arg
385                 390                 395                 400

Ala Ala Gly Ser Ser Val Gly Tyr Gln
                405

<210>  115
<211>  317
<212>  PRT
<213>  Artificial

<220>
<223>  Synthetic

<400>  115

Met Leu Thr Ala Gly Ser Val Gln Arg Leu Gln Ile Ser Met Phe Gln
1               5                   10                  15

Thr Gln Glu Gln Glu Gln Val Asp Leu Ser Ser Ser Ile His Thr Glu
            20                  25                  30

Asn Ile Phe Asn Ser Glu Ser Ser Gly Pro Ala Tyr Pro Asn Ile Pro
        35                  40                  45

Gln Ile Ser Ser Val His Ser Phe Ile Gly Ala Lys Ser Ser Leu Leu
    50                  55                  60

Ala Asp Ala Ala Pro Asn Gln Cys Leu Ser Phe Val Asp Ser Ala Lys
65                  70                  75                  80

Val Thr Ser Phe Asp Gln Ser Phe His Gly Ser Pro Ser Tyr Lys Leu
                85                  90                  95

Asn Ser Ala Lys Leu Ala Val Ser Ser Ser Glu Tyr Leu Ser Val Glu
            100                 105                 110

Ser Ser Thr Glu Val Asp Ser Thr Ile Ser Leu Glu Ala Gln Val Asp
        115                 120                 125

Arg Lys Arg Gln Lys Ile Gln Pro Glu Arg His Ser Lys Thr Asp Asn
    130                 135                 140

Leu Asn Leu Phe Ser Asp Phe Asp Leu Leu Ala Pro Ala Phe Leu Thr
145                 150                 155                 160

Tyr Tyr Leu Pro Leu Phe Arg Gly Cys Ser Ser Gly Gly Asn Asn Lys
                165                 170                 175

Pro Pro Lys Lys Arg Ser Ile Thr Gly Thr Gln Tyr Val Ala Ala Ser
            180                 185                 190

Leu Arg Leu Glu Ser Ser Thr Ile Glu Asp Ile Cys Leu Pro Lys Trp
        195                 200                 205

Asn Glu Asn Glu Leu Lys Asp Asn Arg Arg Ile Ile Arg Ile Glu Arg
    210                 215                 220

Arg Gln Gln Gly Asn Glu Ile Ala Ala Ser Phe Ser Ile Val Gly Ser
225                 230                 235                 240

Ala Leu Glu Asn Pro His Thr Lys Pro Thr Leu Asp Pro Asp Val Glu
                245                 250                 255

Val Leu Glu Val Ser Cys Leu Arg Cys Leu Val Asn Tyr Asp Glu Ile
            260                 265                 270

Asp Asp Lys Gln Thr Val His Gly Ser Leu Leu His Glu Val Gln Ser

275                 280                 285

Glu Phe Thr Glu Ile Arg Lys Gln Glu Ser Thr Gly Lys Arg Ser Ser
    290                 295                 300

Tyr Ala Cys Thr Ser Asn Gly Thr Ser Ile Glu Cys Gln
305                 310                 315

<210>  116
<211>  293
<212>  PRT
<213>  Artificial

<220>
<223>  Synthetic

<400>  116

Met Phe Gln Pro Leu Glu Gln Glu Gln Gln Leu Asp Leu Leu Phe Ala
1               5                   10                  15

Met Pro Pro Glu Asn Ile Phe Asn Ser Asp Ser Ser Ala Leu Thr Phe
            20                  25                  30

Ser Asn Thr Phe Gln Thr Ser Ser Val His Ser Val Thr Gly Val Asp
        35                  40                  45

Ser Pro Ser Leu Val Asn Ile Asn Ile Pro Lys Ser Glu Ser Phe Phe
    50                  55                  60

Met Asp Ser Met Thr Gly Ala Ser Phe Gly Gln Thr Phe Gln Asp Leu
65                  70                  75                  80

Phe Pro Ala Phe Pro Leu Leu Lys Leu Pro Thr Met Val Ala Pro Pro
                85                  90                  95

Thr Ser Leu Lys Met Val Met Asp Arg Lys Arg Arg Gln Ser Gln Ile
            100                 105                 110

Thr Gln Gln Asn Thr Thr Glu Asn Leu Asn Ser Phe His Gly Met Asn
        115                 120                 125

Met Pro Val Pro Ala Phe Leu Glu Tyr Ser Ala Phe Pro Leu Val Arg
    130                 135                 140

Gly Phe Cys Ser Gly Gly Lys Ile Lys Pro Pro Lys Asn Gln Leu Val
145                 150                 155                 160

Pro Gly Thr Glu Tyr Val Asn Val Cys Leu Glu Leu Leu Asn Val Asn
                165                 170                 175

Tyr Glu Gly Ile Cys Leu Pro Lys Trp Asn Glu Lys Glu Leu Glu Glu
            180                 185                 190

Ser Arg Arg Ile Val Arg Val Glu Lys Arg Arg Gln Tyr Asn Lys Ile
        195                 200                 205

Glu Ala Ala Phe Ser Ile Val Asp Ser Val Gln Glu Asn Pro Gln Thr
    210                 215                 220

Asn Ile Ala Val Asp Pro Asn Val Asp Val Leu Glu Phe Ser Cys Leu
225                 230                 235                 240

Leu Cys Pro Ile Lys Glu His Asn Ser Glu Val Ala Gln Thr Val Pro
                245                 250                 255

Thr Ser Ser Ser Ser Glu Glu His Lys Tyr Ser Glu Leu Ala Gly His
            260                 265                 270

Asp Phe Glu Glu Asn Leu Ser Cys Gly Ser Asp Lys Glu Ile Glu Thr
        275                 280                 285

Ser Val Arg Phe Glu
    290

<210>  117
<211>  405
<212>  PRT
<213>  Artificial

<220>
<223>  Synthetic

<400>  117

Met Arg Leu Tyr Leu Ala Thr Val Gly Thr Pro Phe Ile Gly Ala Pro
1               5                   10                  15

Pro Val Ile Ala Ser Asn Glu Asp His Ser Thr Ala His Asn Asn Asn
            20                  25                  30

Ser Asn Asp Phe Asn Tyr Glu Cys Tyr Asn Lys Glu Lys Leu Ser Gly
        35                  40                  45

Asp Leu Leu Glu Ala Asp Leu Phe Asp Pro Pro Ile Asp Asp Thr Met
    50                  55                  60

His Cys Ser Met Phe Ser Ser Ser Thr Thr Ala Asp Thr Glu Gln Leu
65                  70                  75                  80

Gln Lys Pro Lys His Glu Ser Gln His Lys Asn Pro Phe Asp Cys Cys
                85                  90                  95

Gln Glu Leu Lys Leu Lys Met Ala Asp Gln Gln Glu Leu His Leu Leu
            100                 105                 110

Pro Glu Lys Ile Thr Lys Asn Ile Phe Asn Ser Glu Ser Pro Val Phe
        115                 120                 125

Ile Cys Thr Lys Ile Pro Gln Thr Ser Pro Val Gln Ser Phe Val Asp
    130                 135                 140

Val Asn Ala Ser Leu Leu Ala Thr Asn Ser Ala Leu Thr Thr Asn Ser
145                 150                 155                 160

Phe Ser Val Asp Thr Thr Val Asp Ile Leu Phe Glu Gln Thr Val Gln
                165                 170                 175

Asp Pro Tyr Ser Tyr Arg Leu Asn Ser Lys Glu Thr Ala Pro Pro Ser
            180                 185                 190

Thr Ala Ser Phe Phe Gly Thr Leu Asp Leu Gly Pro Thr Val Glu Ala
        195                 200                 205

Ser Thr Thr Leu Leu Glu Ala Ala Val Asn Ser Lys Arg Arg Ile Val
    210                 215                 220

Glu Leu Arg Gln Gln Asn Arg Ile Glu Asp Leu Asn Phe Ser Thr Ser
225                 230                 235                 240

Met Phe Leu Asp His Ser Arg Ile Pro Leu Val Arg Gly Arg Ser Phe
                245                 250                 255

Gly Gly Asn Ser Ser Arg Pro Pro Lys Thr Pro Leu Ile Pro Gly Thr
            260                 265                 270

Arg Tyr Val Thr Ala Arg Leu Arg Leu Glu Asn Ser Thr Ser Glu Asp
        275                 280                 285

Ile Cys Leu Pro His Trp Asn Lys Ala Glu Leu Lys Asp Ser Arg Arg
    290                 295                 300

Ile Ile Arg Ile Glu Arg Arg Tyr Gln Ser Asn Glu Ile Val Ala Thr
305                 310                 315                 320

Phe Ser Ile Val Gly Ser Ala Ile Glu His Pro Lys Thr Lys Pro Ala
                325                 330                 335

Phe Asp Pro Asp Val Lys Val Leu Glu Val Ser Cys Leu Lys Cys Pro
            340                 345                 350

Thr Asn Glu Asn Glu Pro Asn Glu Glu Arg Phe Val Ser Glu Cys Thr
        355                 360                 365

Ser Asp Glu Ala Arg Ala Gln His Ala Leu Thr Glu Pro Leu Met Ser
    370                 375                 380

Gly Phe Asn Arg Glu Pro Asn Gly Arg Asn Pro Lys Thr Ala Gly Asn
385                 390                 395                 400

Ser Ile Gly Cys Arg
                405

<210>  118
<211>  391
<212>  PRT
<213>  Artificial

<220>
<223>  Synthetic

<400>  118

Met Gln Phe Tyr Leu Ala Thr Pro Thr Thr Pro Phe Val Asp Asn Leu
1               5                   10                  15

Thr Ile Pro Ser Gly Ser Gly Ala Tyr Ser Lys Ser Asn Gly Asn Asn
            20                  25                  30

Ser Ser Ser Phe Gly His Ser Tyr Asn Gly Lys Glu Glu Leu Thr Asp
        35                  40                  45

Asp Leu Leu Thr Ala Asp Phe Val Asp Pro Pro Ile Asp Asp Ala Ile
    50                  55                  60

Leu Tyr Ser Met Leu Ser Pro Phe Met Asn Ala Thr Thr Glu Gln Pro
65                  70                  75                  80

Gln Glu Phe Gln Asn Glu Gln Gln His Gln Asn Leu Ser Gly Cys Tyr
                85                  90                  95

Gln Glu Leu Leu Val Gln Thr Glu Glu Gln Gln Gln Leu Asp Leu Leu
            100                 105                 110

Pro Asn Met His Pro Glu Asn Ile Phe Asn Ser Glu Ser Ser Met Phe
        115                 120                 125

Val Tyr Ser Ser Ile Pro Gln Arg Asn Ser Val His Ser Leu Ile Ser
    130                 135                 140

Val Asn Thr Ser Ser Leu Ala Pro Asp Ser Ala Glu Gly Gly Gly Leu
145                 150                 155                 160

Ser Phe Val Asp Thr Ile Gly Pro Ser Phe Asp His Ser Val Glu Thr
                165                 170                 175

Ser Leu Ser Tyr Gly Met Asn Ser Thr Asp Ser Thr Leu Pro Ser Ser
            180                 185                 190

Ala Arg Ser Ser Val Gly Leu Asp Leu Leu Pro Thr Val Ala Ala Pro
        195                 200                 205

Ala Ser Ser Val Ser Ser Leu Glu Thr Val Ile Asn Ser Lys Arg Gln
    210                 215                 220

Lys Leu Gln Phe Arg Gln His Asn Arg Ile Glu Glu Leu Asn Ser Pro
225                 230                 235                 240

His Gly Ile Lys Ala Gln Ala Pro Val Leu Leu Asn Tyr Ser Ser Leu
                245                 250                 255

Pro Leu Val Arg Gly Leu Ser Ser Gly Gly Asn Asn Ser Arg Pro Pro
            260                 265                 270

Lys Glu Pro Leu Ile Pro Gly Ala Gln Tyr Ile Thr Ala Leu Leu Arg
        275                 280                 285

Leu Glu Asn Ser Ser Cys Glu Asp Leu Cys Leu Pro Glu Trp Asn Glu
    290                 295                 300

Asp Glu Leu Lys Asp Cys Arg Arg Ile Ile Arg Ile Glu Arg Lys Arg
305                 310                 315                 320

His Ser Asn Glu Ile Val Ala Asp Ser Asp Val Gln Val Leu Glu Val
                325                 330                 335

Ser Cys Leu Gln Cys Leu Val Asn His Asn Glu Thr Asp Glu Gly Lys
            340                 345                 350

Gln Gly Ser Ser Pro Asn Glu Ile Gln His Ser Ser Ala Glu Pro Pro
        355                 360                 365

Lys Ser Asn Leu Asn Arg Thr Leu Ser Ser Ser Ser His Arg Ala Ala
    370                 375                 380

Gly Ser Ser Val Gly Tyr Gln
385                 390

<210>  119
<211>  317
<212>  PRT
<213>  Artificial

<220>
<223>  Synthetic

<400>  119

Met Leu Thr Ala Gly Ser Val Gln Arg Leu Gln Ile Ser Met Phe Gln
1               5                   10                  15

Thr Gln Glu Gln Glu Gln Val Asp Leu Ser Ser Ser Ile His Thr Glu
            20                  25                  30

Asn Ile Phe Asn Ser Glu Ser Ser Gly Pro Ala Tyr Pro Asn Ile Pro
        35                  40                  45

Gln Ile Ser Ser Val His Ser Phe Ile Gly Ala Lys Ser Ser Leu Leu
    50                  55                  60

Ala Asp Ala Ala Pro Asn Gln Cys Leu Ser Phe Val Asp Ser Ala Lys
65                  70                  75                  80

Val Thr Ser Phe Asp Gln Ser Phe His Gly Ser Pro Ser Tyr Lys Leu
                85                  90                  95

Asn Ser Ala Lys Leu Ala Val Ser Ser Ser Glu Tyr Leu Ser Val Glu
            100                 105                 110

Ser Ser Thr Glu Val Asp Ser Thr Ile Ser Leu Glu Ala Gln Val Asp
        115                 120                 125

Arg Lys Arg Gln Lys Ile Gln Pro Glu Arg His Ser Lys Thr Asp Asn
    130                 135                 140

Leu Asn Leu Phe Ser Asp Phe Asp Leu Leu Ala Pro Ala Phe Leu Thr
145                 150                 155                 160

Tyr Tyr Leu Pro Leu Phe Arg Gly Cys Ser Ser Gly Gly Asn Asn Lys
                165                 170                 175

Pro Pro Lys Lys Arg Ser Ile Thr Gly Thr Gln Tyr Val Ala Ala Ser
            180                 185                 190

Leu Arg Leu Glu Ser Ser Thr Ile Glu Asp Ile Cys Leu Pro Lys Trp
        195                 200                 205

Asn Glu Asn Glu Leu Lys Asp Asn Arg Arg Ile Ile Arg Ile Glu Arg
    210                 215                 220

Arg Gln Gln Gly Asn Glu Ile Ala Ala Ser Phe Ser Ile Val Gly Ser
225                 230                 235                 240

Ala Leu Glu Asn Pro His Thr Lys Pro Thr Leu Asp Pro Asp Val Glu
                245                 250                 255

Val Leu Glu Val Ser Cys Leu Arg Cys Leu Val Asn Tyr Asp Glu Ile
            260                 265                 270

Asp Asp Lys Gln Thr Val His Gly Ser Leu Leu His Glu Val Gln Ser
        275                 280                 285

Glu Phe Thr Glu Ile Arg Lys Gln Glu Ser Thr Gly Lys Arg Ser Ser
    290                 295                 300

Tyr Ala Cys Thr Ser Asn Gly Thr Ser Ile Glu Cys Gln
305                 310                 315

<210>  120
<211>  406
<212>  PRT
<213>  Artificial

<220>
<223>  Synthetic

<400>  120

Met Arg Leu Ser Leu Ala Thr Arg Glu Thr Pro Ser Thr Gly Thr Ser
1               5                   10                  15

Pro Val Phe Ala Gly Asp Glu Asp His Ser Ser Ala Asn Asn Ser Ser
            20                  25                  30

Ser Asn Thr Phe Asn Tyr Asp Tyr Tyr Ile Lys Glu Glu Leu Ser Gly
        35                  40                  45

Asn Leu Leu Glu Thr Ala Phe Phe Glu Ser Pro Ile Asn Asp Thr Met
    50                  55                  60

Leu Cys Ser Met Leu Ser Tyr Pro Lys Ile Ala Gly Thr Glu Lys Ile
65                  70                  75                  80

Gln Glu Pro Asp His Glu Leu Gln Gln His Arg Asn Pro Phe Gly Cys
                85                  90                  95

Cys Gln Glu Leu Lys Ala Glu Leu Ala Glu Gln Gln Glu Leu Tyr Leu
            100                 105                 110

Leu Pro Asn Lys Leu Thr Lys Asn Ile Phe Asn Ser Gly Ser Pro Val
        115                 120                 125

Phe Ile Tyr Thr Lys Ile Pro Gln Thr Ser Ser Val Gln Ser Phe Val
    130                 135                 140

Asp Val Ser Asn Ser Leu Phe Ala Thr Asp Ser Ala Leu Pro Thr Ser
145                 150                 155                 160

Ser Phe Arg Val Asp Ser Thr Val Asp Ile Ser Phe Glu His Ser Ile
                165                 170                 175

Gln Asp Ser Tyr Cys Tyr Arg Ser Asn Ser Ala Glu Ile Ala Pro Pro
            180                 185                 190

Ser Thr Ala Leu Ser Ser Gly Thr His Asp Leu Val Ser Thr Val Glu
        195                 200                 205

Ala Ser Thr Thr Leu Leu Glu Ala Ala Val Asn Ser Lys Arg Arg Met
    210                 215                 220

Leu Glu Leu Arg Gln Gln Asn Arg Ile Glu Asp Leu Ser Phe Ser Thr
225                 230                 235                 240

Ser Ile Phe Leu Asp His Ser Arg Leu Pro Leu Val Arg Gly Arg Ser
                245                 250                 255

Phe Gly Gly Asn Ser Ser Arg Pro Pro Lys Thr Pro Leu Ile Pro Gly
            260                 265                 270

Thr Arg Tyr Val Thr Ala Arg Leu Arg Leu Glu Asn Ser Thr Ser Glu
        275                 280                 285

Asp Met Cys Leu Pro Gln Trp Asn Lys Ala Glu Leu Lys Asp Ser Arg
    290                 295                 300

Arg Ile Ile Arg Ile Glu Arg Arg Tyr Gln Ser Asp Glu Ile Val Ala
305                 310                 315                 320

Ser Phe Ser Ile Val Gly Ser Ala Ile Glu Tyr Pro Lys Thr Lys Pro
                325                 330                 335

Ala Phe Asp Pro Asp Val Lys Val Leu Glu Val Ser Cys Leu Lys Cys
            340                 345                 350

Pro Thr Asn Glu Asn Glu Pro Asn Glu Glu Arg Phe Val Ser Glu Cys
        355                 360                 365

Thr Ser Asp Glu Ala Arg Thr Gln His Ala Leu Thr Glu Pro Leu Thr
    370                 375                 380

Ser Gly Phe Asn Arg Glu Pro Asn Gly His Asn Pro Lys Thr Ala Gly
385                 390                 395                 400

Asn Ser Ile Gly Cys Arg
                405

## Description

### Technical Field

The present invention relates to a vector comprising a nucleotide sequence encoding a novel protein derived from a methanol-utilizing yeast, a mutant cell expressing a gene encoding the protein at a high level, and a method for producing a protein of interest using the mutant cell as a host.

### Background Art

Gene recombination methods are widely used for the production of industrially useful biomaterials such as antibodies, enzymes, and cytokines to be utilized in the medical treatment and diagnosis uses. Hosts used for producing a protein of interest by the gene recombination method are animals such as chicken and cow, animal cells such as CHO, insects such as silkworm, insect cells such as sf9, as well as microorganisms such as yeasts, *E. coli,* and actinomycetes. Yeasts, among the host organisms, are extremely beneficial and various studies have been conducted, since: large scale culture is possible in a low-cost medium at a high density, and thus the protein of interest may be produced at a low cost; a secretory expression into a culture medium is feasible with the use of a signal peptide, and thus the purification process of a protein of interest may be easy; and post-translational modifications such as glycosylation can be made due to being an eucaryote. If an innovative production technology which can be applied to various proteins of interest in yeasts is developed, diverse industrial expansions in addition to the improvement of cost competitiveness by the significantly improved productivity can be hopefully expected.

*Komagataella pastoris*, a yeast species, is a methanol-utilizing yeast having a excellent protein expression ability and capable of utilizing a low-cost carbon source, which is advantageous in the industrial production. For example, Non Patent Literature 1 reports a method for producing exogenous proteins using *Komagataella pastoris* such as green fluorescent protein, human serum albumin, hepatitis B virus surface antigen, human insulin, and single-chain antibody. For the production of an exogenous protein in a yeast, various attempts have been made to improve the productivity thereof such as addition of a signal sequence, use of a strong promoter, codon modification, co-expression of chaperone genes, co-expression of transcription factor genes, inactivation of a protease gene derived from a host yeast, and studies on culture conditions. For example, Patent Literature 1 reports the productivity improvement by the co-expression of transcription factors that activate the AOX promoter. Additionally, Non Patent Literatures 2 to 4 report the productivity improvement by the codon modification in consideration of the codon usage frequency of *Komagataella pastoris*, co-expression of chaperone genes, and inactivation of a protease gene, respectively.

However, all Literatures aim at improving the production of exogenous proteins but do not aim at searching factors for improving the secretion of both an endogenous protein and an exogenous protein.

### Citation List

### Patent Literature

Patent Literature 1: International Publication No. WO2012/102171

### Non Patent Literature

Non Patent Literature 1: FEMS Microbiology Reviews 24 (2000) 45-66
Non Patent Literature 2: PLoS One. 2011; 6(8): e22577
Non Patent Literature 3: Biotechnol. Bioeng. 2006 Mar 5; 93(4):771-8
Non Patent Literature 4: Methods Mol. Biol. 103, 81-94

### Summary of Invention

### Technical Problem

It is an object of the present invention to provide a new means for improving secretion amounts of an endogenous protein and an exogenous protein.

### Solution to Problem

The present inventors identified a novel protein family by a comprehensive analysis on the nucleotide sequence of chromosomal DNA of yeasts belonging to the genus *Komagataella.* The inventors further found that secretion amounts of endogenous proteins and an exogenous protein in yeasts belonging to the genus *Komagataella* are improved by expressing the gene encoding the novel protein at a high level. Then, the present inventors have accomplished the present invention.

More specifically, the present invention encompasses the following aspects.
(1) A vector comprising:
   (a) a nucleotide sequence encoding an amino acid sequence as set forth in any of SEQ ID NOs: 113, 108 to 112, and 114 to 120,
   (b) a nucleotide sequence encoding an amino acid sequence in which one or more amino acids are substituted, deleted, and/or added in the amino acid sequence as set forth in the (a),
   (c) a nucleotide sequence encoding an amino acid sequence having a sequence identity of 85% or more to the amino acid sequence as set forth in the (a), or
   (d) a nucleotide sequence of a nucleic acid that hybridizes under stringent conditions to a nucleic acid consisting of a complementary sequence to a nucleotide sequence encoding the amino acid sequence as set forth in the (a).
(2) The vector according to (1), wherein the nucleotide sequences according to the (a) to (d) are respectively:
   (a') a nucleotide sequence as set forth in any of SEQ ID NOs: 75, 65, 67, 69, 71, 73, 77, 79, 81, 83, 85, 87, and 89,
   (b') a nucleotide sequence in which one or more nucleotides are substituted, deleted, and/or added in the nucleotide sequence as set forth in the (a'),
   (c') a nucleotide sequence having a sequence identity of 85% or more to the nucleotide sequence as set forth in the (a'), and
   (d') a nucleotide sequence of a nucleic acid that hybridizes under stringent conditions to a nucleic acid consisting of a complementary sequence to the nucleotide sequence as set forth in the (a').
(3) A vector comprising:
   (e) a nucleotide sequence encoding an amino acid sequence as set forth in any of SEQ ID NOs: 100, 95 to 99, and 101 to 107,
   (f) a nucleotide sequence encoding an amino acid sequence in which one or more amino acids are substituted, deleted, and/or added in the amino acid sequence as set forth in the (e),
   (g) a nucleotide sequence encoding an amino acid sequence having a sequence identity of 85% or more to the amino acid sequence as set forth in the (e), or
   (h) a nucleotide sequence of a nucleic acid that hybridizes under stringent conditions to a nucleic acid consisting of a complementary sequence to a nucleotide sequence encoding the amino acid sequence as set forth in the (e).
(4) The vector according to (3), wherein the nucleotide sequences according to the (e) to (h) are respectively:
   (e') a nucleotide sequence as set forth in any of SEQ ID NOs: 39, 24, 27, 30, 33, 36, 42, 45, 48, 51, 54, 57, and 60,
   (f) a nucleotide sequence in which one or more nucleotides are substituted, deleted, and/or added in the nucleotide sequence as set forth in the (e'),
   (g') a nucleotide sequence having a sequence identity of 85% or more to the nucleotide sequence as set forth in the (e'), and
   (h') a nucleotide sequence of a nucleic acid that hybridizes under stringent conditions to a nucleic acid consisting of a complementary sequence to the nucleotide sequence as set forth in the (e').
(5) The vector according to any of (1) to (4), wherein the vector increases a secretion amount of a protein in a host cell.
(6) A protein secretion enhancer consisting of the vector according to any of (1) to (5).
(7) A mutant cell having an increased expression of a gene comprising the nucleotide sequence defined in any of (1) to (4) compared to that of a wild-type, and an increased secretion amount of a protein compared to that of the wild-type gene.
(8) A cell comprising the vector according to any of (1) to (5).
(9) The cell according to (7) or (8), wherein the cell is a yeast, a bacterium, a fungus, an insect cell, an animal cell, or a plant cell.
(10) The cell according to (9), wherein the yeast is a methanol-utilizing yeast, a fission yeast, or a budding yeast.
(11) The cell according to (10), wherein the methanol-utilizing yeast is a yeast belonging to the genus *Komagataella* or a yeast belonging to the genus *Ogataea.*
(12) A method for preparing a mutant strain, comprising a step of introducing the vector according to any of (1) to (5) or the protein secretion enhancer according to (6) into a host cell, wherein the mutant strain has an increased secretion amount of a protein compared to the that of host cell before the introduction.
(13) A method for producing a protein of interest, comprising:
   a step of culturing the cell according to any of (7) to (11), and
   a step of recovering the protein of interest from a culture medium.
(14) The method according to (13), wherein the protein of interest is a heterologous protein.
(15) The method according to (13) or (14), wherein a culture medium comprising one or more carbon sources selected from the group consisting of glucose, glycerol and methanol is used in the step of culturing.

The present specification encompasses the content disclosed in JP Patent Application No. 2016-064364, to which present application claims priority.

### Advantageous Effects of Invention

Secretion amounts of an endogenous protein and an exogenous protein in a host cell are improved by expressing the polypeptide of the present invention at a high level. Further, the present invention provides a method for effectively producing a protein of interest using a yeast belonging to the genus *Komagataella* as a host.

### Brief Description of Drawings

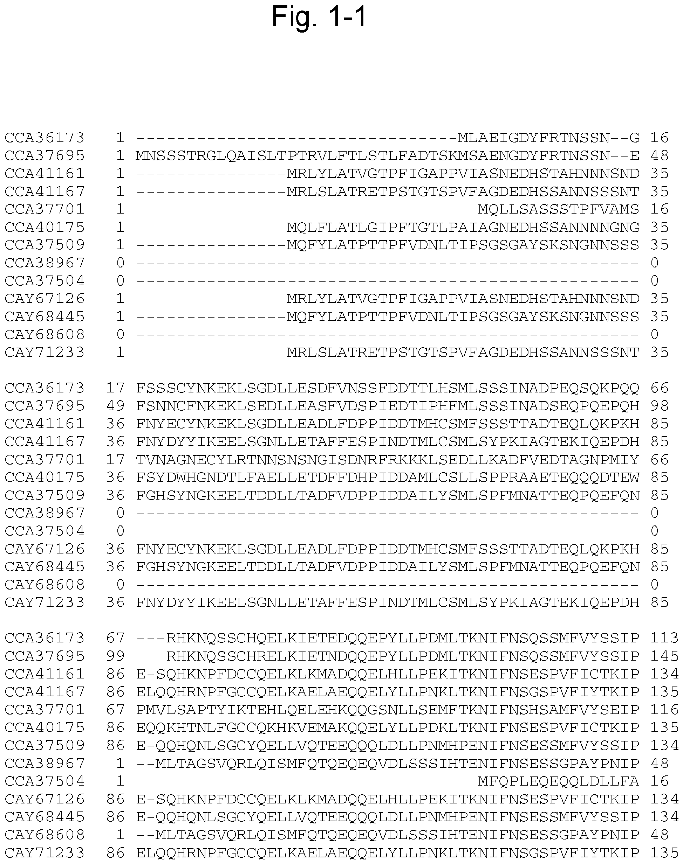

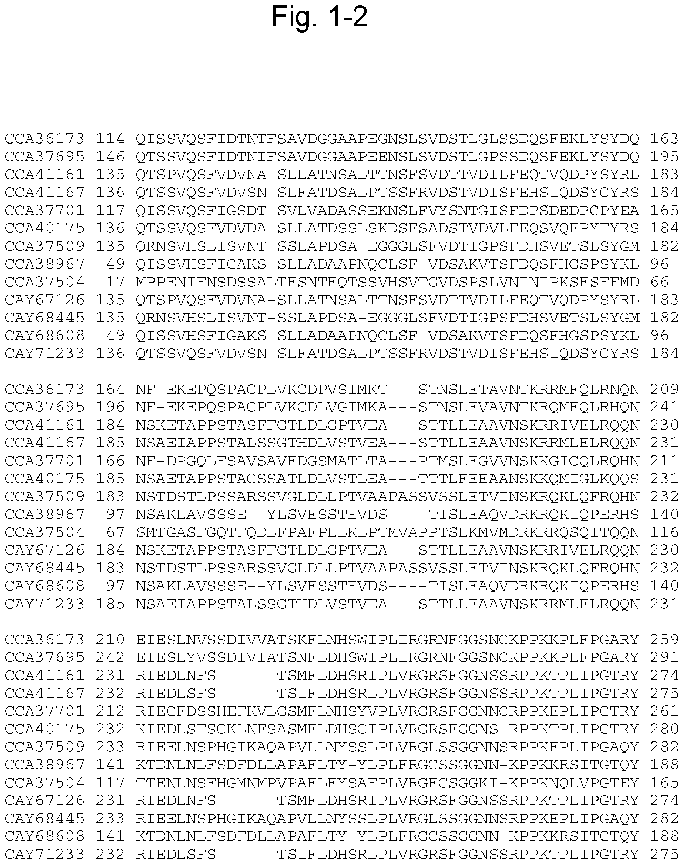

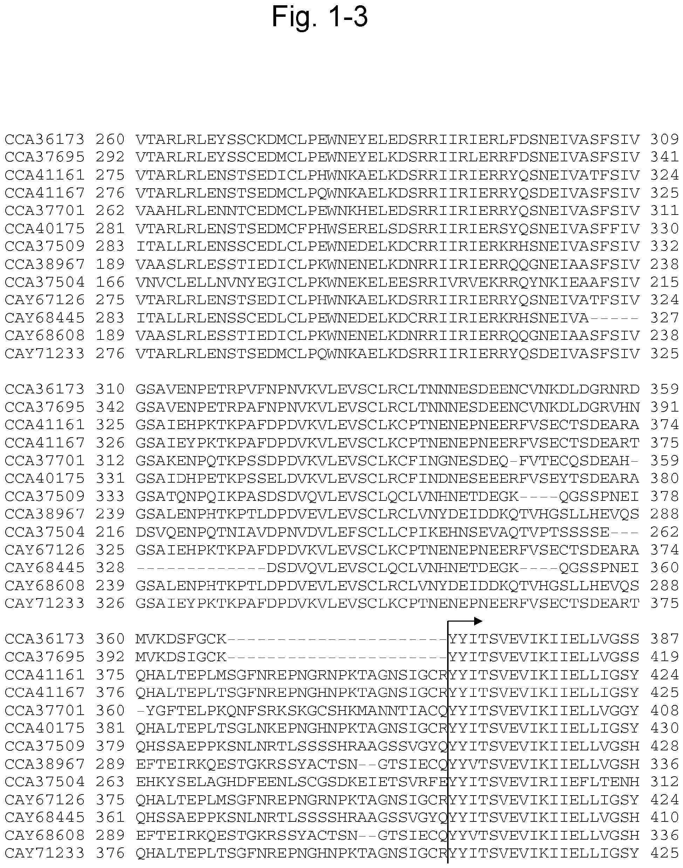

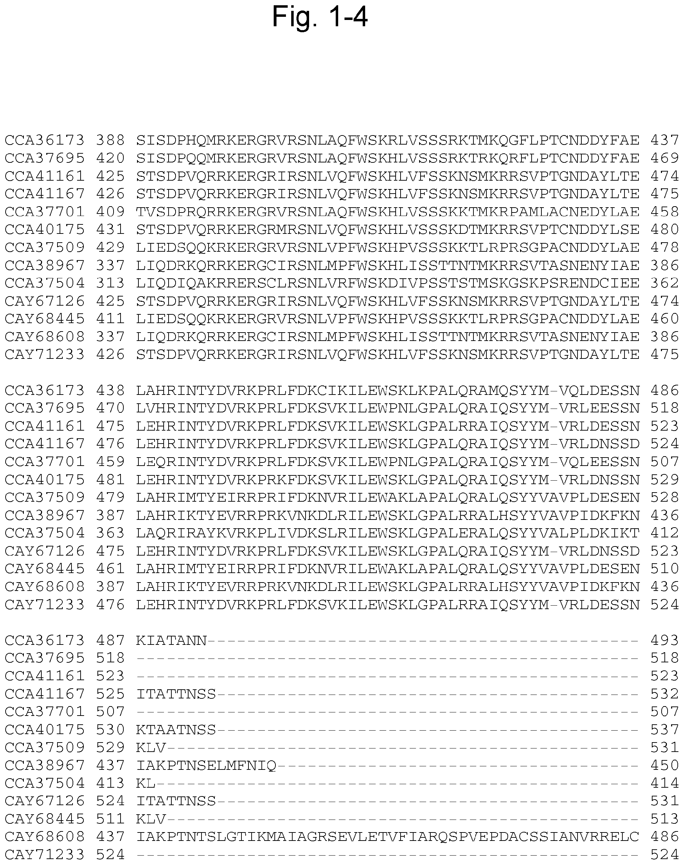

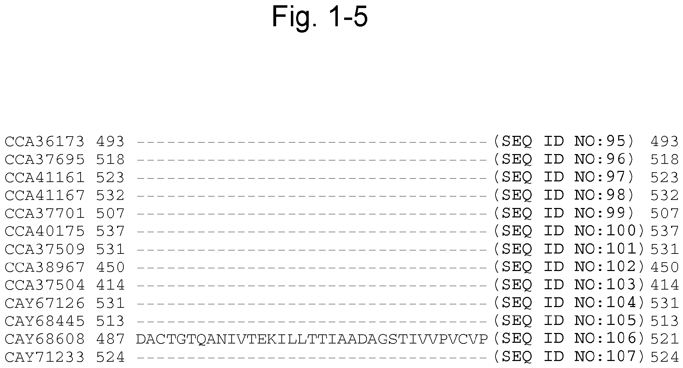

Figure 1 is a diagram showing the alignment result of the amino acid sequences of novel polypeptides discovered by the present inventors.
Figure 1-2 is the continuation of Figure 1-1.
Figure 1-3 is the continuation of Figure 1-2. The arrow indicates the start site of C-terminal regions found to have relatively high homology to one another that were deleted in the Examples.
Figure 1-4 is the continuation of Figure 1-3.
Figure 1-5 is the continuation of Figure 1-4.

### Description of Embodiment

Hereinafter, the present invention is described in detail in reference to preferable embodiments.

### 1. Definition of terms

In the present invention, two nucleic acids hybridizing under stringent conditions means, for example, as follows. For example, nucleic acid Y is considered as "the nucleic acid that hybridizes to the nucleic acid X under the stringent conditions", when the nucleic acid Y can be obtained as the nucleic acid bound on a filter by using the filter with an immobilized nucleic acid X, hybridizing it to a nucleic acid Y at 65°C in the presence of 0.7 to 1.0 M NaCl, and then washing the filter under the condition of 65°C using 2-fold concentration of an SSC solution (the composition of 1-fold concentration of the SSC solution consists of 150 mM sodium chloride and 15 mM sodium citrate) Alternatively, the nucleic acid X and the nucleic acid Y can be said to "hybridize to each other under stringent conditions." The nucleic acid Y is a nucleic acid obtained as the nucleic acid bound on a filter by preferably washing the filter at 65°C using 0.5-fold concentration of an SSC solution, more preferably washing at 65°C using 0.2-fold concentration of an SSC solution, and further preferably washing at 65°C using 0.1-fold concentration of an SSC solution. The standard nucleic acid X may be a colony- or plaque-derived nucleic acid X.

The sequence identity of a nucleotide sequence and an amino acid sequence in the present invention can be determined by a method or a sequence analysis software known by a person skilled in the art. Examples include the blastn program and blastp program of BLAST algorithm, and fasta program of FASTA algorithm. In the present invention, the "sequence identity" of a certain nucleotide sequence to be evaluated to a nucleotide sequence X is a value shown in % of the frequency with which same nucleotides appear in same sites of the nucleotide sequences including the gap parts, when the nucleotide sequence X and the nucleotide sequence to be evaluated are aligned and gaps are introduced as needed to achieve the highest nucleotide alignment between both sequences. When comparing a nucleotide sequence of DNA with a nucleotide sequence of RNA, T and U are considered as the identical nucleotide. In the present invention, the "sequence identity" of a certain amino acid sequence to be evaluated to an amino acid sequence X is a value shown in % of the frequency with which the same amino acid appears in the same site of the amino acid sequences including the gap parts, when the amino acid sequence X and the amino acid sequence to be evaluated are aligned and gaps are introduced as needed to achieve the highest amino acid alignment between both sequences.

The "nucleic acid" in the present invention may also be called as "polynucleotide" referring to DNA or RNA, but typically referring to DNA. The "polynucleotide" in the present invention may be present in the double-stranded form with a complementary strand thereto. In particular, when the "polynucleotide" is DNA, it is preferable that the DNA comprising a certain nucleotide sequence be present in the double-stranded form with DNA comprising a complementary nucleotide sequence thereto.

In the present invention, the "polypeptide" refers to those in which 2 or more amino acids are peptide bonded, and includes those having a short chain length called as peptides and oligopeptides in addition to proteins.

The "nucleotide sequence encoding a polypeptide " in the present invention refers to a nucleotide sequence of a polynucleotide that produces a polypeptide by transcription and translation, and refers to, for example, a nucleotide sequence designed based on a codon table to a polypeptide consisting of an amino acid sequence.

The "host cell" in the present invention refers to a cell to be transformed by introducing a vector thereinto, and called as a "host" or a "transformant". Herein, a host cell before and after transformation is sometimes simply called as the "cell." The cell used as the host is not particularly limited as long as a vector can be introduced to the cell.

The species of the host cell is not particularly limited, and examples include a yeast, a bacterium, a fungus, an insect cell, an animal cell, and a plant cell, preferably yeast, and more preferably a methanol-utilizing yeast. The methanol-utilizing yeast is generally defined as a yeast which can be cultured by utilizing methanol as an only carbon source, but yeast which originally was a methanol-utilizing yeast but lost the methanol-utilizing ability due to an artificial modification or mutation is also encompassed by the methanol-utilizing yeast of the present invention.

Examples of the methanol-utilizing yeast include yeasts belonging to the genus *Pichia,* the genus *Ogataea,* the genus *Candida*, the genus *Torulopsis,* and the genus *Komagataella.* Preferable examples include *Pichia methanolica* in the genus *Pichia, Ogataea angusta*, *Ogataea polymorpha*, *Ogataea parapolymorpha*, and *Ogataea minuta* in the genus *Ogataea, Candida boidinii* in the genus *Candida, Komagataella pastoris* and *Komagataella phaffii* in the genus *Komagataella.*

Among the methanol-utilizing yeasts described above, yeasts belonging to the genus *Komagataella* or yeasts belonging to the genus *Ogataea* are particularly preferable.

For the yeast belonging to the genus *Komagataella*, *Komagataella pastoris* and *Komagataella phaffii* are preferable. *Komagataella pastoris* and *Komagataella phaffii* both have another name as *Pichia pastoris.*

Specific examples of the strain to be used as the host include strains of *Komagataella pastoris* ATCC76273 (Y-11430, CBS7435) and *Komagataella pastoris* X-33. These strains are available from American Type Culture Collection and Thermo Fisher Scientific, Inc.

For the yeast belonging to the genus *Ogataea*, *Ogataea angusta*, *Ogataea polymorpha*, and *Ogataea parapolymorpha* are preferable. These 3 are closely related to each other, and all are also known as *Hansenula polymorpha* or *Pichia angusta.*

Specific examples of the strain to be used include *Ogataea angusta* NCYC495 (ATCC14754), *Ogataea polymorpha* 8V (ATCC34438), and *Ogataea parapolymorpha* DL-1 (ATCC26012). These strains are available from American Type Culture Collection.

Further, in the present invention, derivative strains from these strains of yeasts belonging to the genus *Komagataella* or yeasts belonging to the genus *Ogataea* can also be used, and examples of histidine-dependent yeasts include *Komagataella pastoris* GS115 strain (available from Thermo Fisher Scientific, Inc.), and examples of leucine-dependent yeasts include NCYC495-derived BY4329, 8V-derived BY5242, and DL-1-derived BY5243 (these can be distributed from National BioResource Project). In the present invention, derivative strains from these strains can also be used.

The "expression" in the present invention refers to the transcription and translation of the nucleotide sequence that produces a polypeptide. Further, the expression may be in a substantially constant state depending or without depending on external stimulation or growth conditions. The promoter for driving the expression is not particularly limited as long as the promoter drives the expression of a nucleotide sequence encoding a polypeptide.

The "expression at a high level" in the present invention means an increased amount of a polypeptide in a host cell or an increased amount of mRNA in a host cell compared to a normal amount, and, for example, such a level can be confirmed by measuring an amount by utilizing an antibody that recognizes the polypeptide or measuring amounts by the RT-PCR method, northern hybridization, or the hybridization using DNA array, and comparing the amount to that of non-modified strains such as a parent cell or a wild-type strain.

### 2. Vector comprising a nucleotide sequence encoding a novel polypeptide

In one aspect, the present invention relates to a vector comprising (a) a nucleotide sequence encoding an amino acid sequence as set forth in any of SEQ ID NOs: 113, 108 to 112, and 114 to 120, preferably SEQ ID NOs: 113, 108 to 112, 117, and 120, (b) a nucleotide sequence encoding an amino acid sequence in which one or more amino acids are substituted, deleted, and/or added in the amino acid sequence as set forth in the (a), (c) a nucleotide sequence encoding an amino acid sequence having a sequence identity of 85% or more, for example, 90% or more, 95% or more, 96% or more, 97% or more, 98% or more, or 99% or more, to the amino acid sequence as set forth in the (a), or (d) a nucleotide sequence of a nucleic acid that hybridizes under stringent conditions to a nucleic acid consisting of a complementary sequence to a nucleotide sequence encoding the amino acid sequence as set forth in the (a).

The "one or more" regarding the substitution, deletion, insertion and/or addition of amino acids in the present invention means, for example, in the amino acid sequence according to (b), 1 to 280 amino acids, 1 to 250 amino acids, 1 to 200 amino acids, 1 to 190 amino acids, 1 to 160 amino acids, 1 to 130 amino acids, 1 to 100 amino acids, 1 to 75 amino acids, 1 to 50 amino acids, 1 to 25 amino acids, 1 to 20 amino acids, 1 to 15 amino acids, 1 to 10 amino acids, 1 to 7 amino acids, 1 to 5 amino acids, 1 to 4 amino acids, 1 to 3 amino acids, or 1 or 2 amino acids, in the amino acid sequence as set forth in any of SEQ ID NOs: 113, 108 to 112, and 114 to 120. Examples of the amino acid sequence according to (b) include partial amino acid sequences consisting of 1 to 5, 1 to 10, 1 to 25, 1 to 50, 1 to 75, 1 to 100, 1 to 125, 1 to 150, 1 to 175, 1 to 200, 1 to 225, 1 to 250, 1 to 275, or 1 to 300 consecutive amino acids in the amino acid sequence as set forth in any of SEQ ID NOs: 113, 108 to 112, and 114 to 120.

In one embodiment, the nucleotide sequences according to (a) to (d) may be (a') a nucleotide sequence as set forth in any of SEQ ID NOs:75, 65, 67, 69, 71, 73, 77, 79, 81, 83, 85, 87, and 89, preferably SEQ ID NOs: 75, 65, 67, 69, 71, 73, 83, and 89, (b') a nucleotide sequence in which one or more amino acids are substituted, deleted, and/or added in the amino acid sequence as set forth in the (a'), (c') a nucleotide sequence having a sequence identity of 85% or more, for example, 90% or more, 95% or more, 96% or more, 97% or more, 98% or more, or 99% or more, to the nucleotide sequence as set forth in the (a'), or (d') a nucleotide sequence of a nucleic acid that hybridizes under stringent conditions to a nucleic acid consisting of a complementary sequence to the nucleotide sequence as set forth in the (a'), respectively.

The "one or more" regarding the substitution, deletion, insertion and/or addition of nucleotides in the present invention means, for example, in the nucleotide sequence according to (b'), 1 to 800 nucleotides, 1 to 700 nucleotides, 1 to 600 nucleotides, 1 to 500 nucleotides, 1 to 400 nucleotides, 1 to 300 nucleotides, 1 to 200 nucleotides, 1 to 190 nucleotides, 1 to 160 nucleotides, 1 to 130 nucleotides, 1 to 100 nucleotides, 1 to 75 nucleotides, 1 to 50 nucleotides, 1 to 25 nucleotides, 1 to 20 nucleotides, 1 to 15 nucleotides, 1 to 10 nucleotides, 1 to 7 nucleotides, 1 to 5 nucleotides, 1 to 4 nucleotides, 1 to 3 nucleotides, or 1 or 2 nucleotides in the sequence as set forth in any of SEQ ID NOs: 75, 65, 67, 69, 71, 73, 77, 79, 81, 83, 85, 87, and 89. Examples of the nucleotide sequence of (b') include partial nucleotide sequences consisting of 1 to 5, 1 to 10, 1 to 25, 1 to 50, 1 to 75, 1 to 100, 1 to 125, 1 to 150, 1 to 175, 1 to 200, 1 to 225, 1 to 250, 1 to 275, 1 to 300, 1 to 350, 1 to 400, 1 to 450, 1 to 500, 1 to 600, 1 to 700, or 1 to 800 consecutive nucleotides in the nucleotide sequence as set forth in any of SEQ ID NOs: 75, 65, 67, 69, 71, 73, 77, 79, 81, 83, 85, 87, and 89.

In one aspect, the present invention relates to a vector comprising (e) a nucleotide sequence encoding an amino acid sequence as set forth in any of SEQ ID NOs: 100, 95 to 99, and 101 to 107, (f) a nucleotide sequence encoding an amino acid sequence in which one or more amino acids are substituted, deleted, and/or added in the amino acid sequence as set forth in the (e), (g) a nucleotide sequence encoding an amino acid sequence having a sequence identity of 85% or more, for example, 90% or more, 95% or more, 96% or more, 97% or more, 98% or more, or 99% or more, to the amino acid sequence as set forth in the (e), or (h) a nucleotide sequence of a nucleic acid that hybridizes under stringent conditions to a nucleic acid consisting of a complementary sequence to a nucleotide sequence encoding the amino acid sequence as set forth in the (e).

The "one or more" regarding the substitution, deletion, insertion and/or addition of amino acids in the present invention means, for example, in the amino acid sequence according to (f), 1 to 400 amino acids, 1 to 300 amino acids, 1 to 200 amino acids, 1 to 190 amino acids, 1 to 160 amino acids, 1 to 130 amino acids, 1 to 100 amino acids, 1 to 75 amino acids, 1 to 50 amino acids, 1 to 25 amino acids, 1 to 20 amino acids, 1 to 15 amino acids, 1 to 10 amino acids, 1 to 7 amino acids, 1 to 5 amino acids, 1 to 4 amino acids, 1 to 3 amino acids, or 1 or 2 amino acids in the amino acid sequence as set forth in any of SEQ ID NOs: 100, 95 to 99, and 101 to 107. Examples of the amino acid sequence according to (f) include partial amino acid sequences consisting of 1 to 5, 1 to 10, 1 to 25, 1 to 50, 1 to 75, 1 to 100, 1 to 125, 1 to 150, 1 to 175, 1 to 200, 1 to 225, 1 to 250, 1 to 275, 1 to 300, 1 to 325, 1 to 350, 1 to 375, or 1 to 400 consecutive amino acids in the amino acid sequence as set forth in any of SEQ ID NOs: 100, 95 to 99, and 101 to 107.

In one embodiment, the nucleotide sequences according to (e) to (h) may be (e') a nucleotide sequence as set forth in any of SEQ ID NOs: 39, 24, 27, 30, 33, 36, 42, 45, 48, 51, 54, 57, and 60, (f) a nucleotide sequence in which one or more nucleotides are substituted, deleted, and/or added in the nucleotide sequence as set forth in the (e'), (g') a nucleotide sequence having a sequence identity of 85% or more, for example, 90% or more, 95% or more, 96% or more, 97% or more, 98% or more, or 99% or more, to the nucleotide sequence as set forth in the (e'), or (h') a nucleotide sequence of a nucleic acid that hybridizes under stringent conditions to a nucleic acid consisting of a complementary sequence to the nucleotide sequence as set forth in the (e'), respectively.

The "one or more" regarding the substitution, deletion, insertion and/or addition of nucleotides in the present invention means, for example, in the nucleotide sequence according to (f), 1 to 1200 nucleotides, 1 to 1000 nucleotides, 1 to 500 nucleotides, 1 to 400 nucleotides, 1 to 300 nucleotides, 1 to 200 nucleotides, 1 to 190 nucleotides, 1 to 160 nucleotides, 1 to 130 nucleotides, 1 to 100 nucleotides, 1 to 75 nucleotides, 1 to 50 nucleotides, 1 to 25 nucleotides, 1 to 20 nucleotides, 1 to 15 nucleotides, 1 to 10 nucleotides, 1 to 7 nucleotides, 1 to 5 nucleotides, 1 to 4 nucleotides, 1 to 3 nucleotides, or 1 or 2 nucleotides in the nucleotide sequence as set forth in any of SEQ ID NOs: 39, 24, 27, 30, 33, 36, 42, 45, 48, 51, 54, 57, and 60. Examples of the nucleotide sequence of (f) include partial nucleotide sequences consisting of 1 to 5, 1 to 10, 1 to 25, 1 to 50, 1 to 75, 1 to 100, 1 to 125, 1 to 150, 1 to 175, 1 to 200, 1 to 225, 1 to 250, 1 to 275, 1 to 300, 1 to 350, 1 to 400, 1 to 450, 1 to 500, 1 to 600, 1 to 700, 1 to 800, 1 to 900, 1 to 1000, 1 to 1100, or 1 to 1200 consecutive nucleotides in the nucleotide sequence as set forth in any of 39, 24, 27, 30, 33, 36, 42, 45, 48, 51, 54, 57, and 60.

The vector of the present invention may comprise a combination of 2 or more of the nucleotide sequences as set forth in the (a) to (h). Examples of the number of combination include 3 or more, 4 or more, 5 or more, 6 or more, 7 or more, 8 or more, 9 or more, or 10 or more. For example, the vector of the present invention may comprise a combination of 2 or more sequences of the (a), i.e., the nucleotide sequence encoding the amino acid sequence as set forth in any of SEQ ID NOs: 113, 108 to 112, and 114 to 120, or equivalent sequences to any of these sequences. Herein, the "equivalent sequence" to the sequences according to the (a) means the sequences according to the (b) to (d) to the respective sequences according to the (a). Thus, the vector of the present invention may comprise, for example, a combination of 2 or more sequences as set forth in the (a), a combination of 2 or more sequences as set forth in the (b), a combination of 2 or more sequences as set forth in the (c), or a combination of 2 or more sequences as set forth in the (d). Similarly, the vector of the present invention may comprise a combination of 2 or more sequences of the (e), i.e., a nucleotide sequence encoding the amino acid sequence as set forth in any of SEQ ID NOs: 100, 95 to 99, and 101 to 107, or equivalent sequences to any of these. Herein, the "equivalent sequence" to the sequences according to the (e) means the sequences according to the (f) to (h) to the respective sequences according to the (e). Thus, the vector of the present invention may comprise, for example, a combination of 2 or more sequences as set forth in the (e), a combination of 2 or more sequences as set forth in the (f), a combination of 2 or more sequences as set forth in the (g), or a combination of 2 or more sequences as set forth in the (h). The vector of the present invention may comprise, for example, a combination of 2 or more nucleotide sequences encoding the amino acid sequence as set forth in any of SEQ ID NOs: 113, 108 and 109, or equivalent sequences to any of these. The vector of the present invention preferably comprises a combination of 2 or more nucleotide sequences encoding the amino acid sequence as set forth in any of SEQ ID NOs: 100, 95, and 96, or equivalent sequences to any of these.

Further, the vector of the present invention may comprise a combination of 2 or more nucleotide sequences as set forth in the (a') to (h'). Examples of the number of combination include 3 or more, 4 or more, 5 or more, 6 or more, 7 or more, 8 or more, 9 or more, or 10 or more. For example, the vector of the present invention may comprise a combination of 2 or more sequences of the (a'), i.e., the nucleotide sequence as set forth in any of SEQ ID NOs: 75, 65, 67, 69, 71, 73, 77, 79, 81, 83, 85, 87, and 89, or equivalent sequences to any of these sequences. Herein, the "equivalent sequence" to the sequences according to the (a') means the sequences according to the (b') to (d') to the respective sequences according to the (a'). Thus, the vector of the present invention may comprise a combination of 2 or more sequences as set forth in the (a'), a combination of 2 or more sequences as set forth in the (b'), a combination of 2 or more sequences as set forth in the (c'), or a combination of 2 or more sequences as set forth in the (d'). Similarly, the vector of the present invention may comprise a combination of 2 or more sequences of the (e'), i.e., a nucleotide sequence as set forth in any of SEQ ID NOs: 39, 24, 27, 30, 33, 36, 42, 45, 48, 51, 54, 57, and 60, or equivalent sequences to any of these. Herein, the "equivalent sequence" to the sequences according to the (e') means the sequences according to the (f) to (h') to the respective sequences according to the (e'). Thus, the vector of the present invention may comprise a combination of 2 or more sequences as set forth in the (e'), a combination of 2 or more sequences as set forth in the (f), a combination of 2 or more sequences as set forth in the (g'), or a combination of 2 or more sequences as set forth in the (h'). The vector of the present invention may comprise, for example, a combination of 2 or more nucleotide sequences as set forth in any of SEQ ID NOs: 75, 65, and 67, or equivalent sequences to any of these. The vector of the present invention preferably comprises a combination of 2 or more nucleotide sequences as set forth in any of SEQ ID NOs: 39, 24, and 27, or equivalent sequences to any of these.

Further, the present invention also relates to a combination of 2 or more vectors comprising the nucleotide sequence as set forth in any of the (a) to (h) and (a') to (h'). The number of combination and examples of the vector are the same as the combinations of the nucleotide sequences comprised in the vector, and hence the description is omitted.

The above nucleotide sequences comprised in the vector of the present invention were found by the comprehensive analysis on the nucleotide sequences of 4 chromosomal DNA of *Komagataella pastoris* (CBS7435 strain: ACCESSION No. FR839628 to FR839631 (J. Biotechnol. 154 (4), 312-320 (2011), and GS115 strain: ACCESSION No. FN392319 to FN392322 (Nat. Biotechnol. 27 (6), 561-566 (2009))). Specifically, the present inventors searched for a polypeptide comprising the amino acid sequence as set forth in SEQ ID NO: 93 or 94, and a polynucleotide comprising the nucleotide sequence as set forth in SEQ ID NO: 91 or 92 encoding the amino acid sequence. As a result, the inventor found the following 9 novel polypeptides having ACCESSION Nos. beginning with CCA in the CBS7435 strain, the following 4 novel polypeptides having ACCESSION Nos. beginning with CAY in the GS115 strain, and the following polynucleotides encoding these novel polypeptides:
a polypeptide under ACCESSION No. CCA36173 comprising the amino acid sequence as set forth in SEQ ID NO: 95, and a polynucleotide comprising the nucleotide sequence as set forth in SEQ ID NO: 24 encoding the polypeptide;
a polypeptide under ACCESSION No. CCA37695 comprising the amino acid sequence as set forth in SEQ ID NO: 96, and a polynucleotide comprising the nucleotide sequence as set forth in SEQ ID NOs: 27 encoding the polypeptide;
a polypeptide under ACCESSION No. CCA41161 comprising the amino acid sequence as set forth in SEQ ID NO: 97, and a polynucleotide comprising the nucleotide sequence as set forth in SEQ ID NO: 30 encoding the polypeptide;
a polypeptide under ACCESSION No. CCA41167 comprising the amino acid sequence as set forth in SEQ ID NO: 98, and a polynucleotide comprising the nucleotide sequence as set forth in SEQ ID NO: 33 encoding the polypeptide;
a polypeptide under ACCESSION No. CCA37701 comprising the amino acid sequence as set forth in SEQ ID NO: 99, and a polynucleotide comprising the nucleotide sequence as set forth in SEQ ID NO: 36 encoding the polypeptide;
a polypeptide under ACCESSION No. CCA40175 comprising the amino acid sequence as set forth in SEQ ID NO: 100, and a polynucleotide comprising the nucleotide sequence as set forth in SEQ ID NO: 39 encoding the polypeptide;
a polypeptide under ACCESSION No. CCA37509 comprising the amino acid sequence as set forth in SEQ ID NO: 101, and a polynucleotide comprising the nucleotide sequence as set forth in SEQ ID NO: 42 encoding the polypeptide;
a polypeptide under ACCESSION No. CCA38967 comprising the amino acid sequence as set forth in SEQ ID NO: 102, and a polynucleotide comprising the nucleotide sequence as set forth in SEQ ID NO: 45 encoding the polypeptide;
a polypeptide under ACCESSION No. CCA37504 comprising the amino acid sequence as set forth in SEQ ID NO: 103, and a polynucleotide comprising the nucleotide sequence as set forth in SEQ ID NO: 48 encoding the polypeptide;
a polypeptide under ACCESSION No. CAY67126 comprising the amino acid sequence as set forth in SEQ ID NO: 104, and a polynucleotide comprising the nucleotide sequence as set forth in SEQ ID NO: 51 encoding the polypeptide;
a polypeptide under ACCESSION No. CAY68445 comprising the amino acid sequence as set forth in SEQ ID NO: 105, and a polynucleotide comprising the nucleotide sequence as set forth in SEQ ID NO: 54 encoding the polypeptide;
a polypeptide under ACCESSION No. CAY68608 comprising the amino acid sequence as set forth in SEQ ID NO: 106, and a polynucleotide comprising the nucleotide sequence as set forth in SEQ ID NO: 57 encoding the polypeptide; and
a polypeptide under ACCESSION No. CAY71233 comprising the amino acid sequence as set forth in SEQ ID NO: 107, and a polynucleotide comprising the nucleotide sequence as set forth in SEQ ID NO: 60 encoding the polypeptide.

In Examples to be described later, the present inventors confirmed that a secretion amount of the protein is improved by introducing the vector comprising any of the above nucleotide sequences into a host and allowing the host to express the above polypeptide at a high level.

Additionally, the amino acid sequences of these novel polypeptides were aligned as shown in Figure 1 and deleting the C-terminal region at which a comparatively high identity was confirmed to prepare the following mutants;
a polypeptide comprising the amino acid sequence as set forth in any of SEQ ID NOs: 108 to 120 in which about 120 to about 200 amino acids are respectively deleted in the C-terminal region of the amino acid sequence as set forth in any of SEQ ID NOs: 95 to 107; and
a polynucleotide comprising the nucleotide sequence as set forth in any of SEQ ID NOs: 65, 67, 69, 71, 73, 75, 77, 79, 81, 83, 85, 87, and 89 respectively encoding the polypeptide.

In Examples to be described later, the present inventors introduced some of the vectors comprising any of the above nucleotide sequences into a host and allowing the host to express the above polypeptide in which the C-terminal region is deleted, thereby confirming that a secretion amount of the protein is improved but the degree of the improvement in secretion amount tends to be lowered compared to the case where the polypeptide comprising the C-terminal region is expressed. Thus, it is believed that, among the amino acids sequence as set forth in any of SEQ ID NOs: 95 to 107, the sequence at the N-terminal side improves the secretion amount of a protein while the sequence at the C-terminal side plays a role of enhancing the function of the sequence at the N-terminal side.

In one embodiment, the above vectors of the present invention can increase the secretion amount of a protein from a host cell, for example, when introduced to the host cell by transformation.

The "vector" in the present invention is a nucleic acid molecule constructed artificially, and may comprise, in addition to the above specified nucleotide sequence (referred to as "specified nucleotide sequence" to describe any of the above nucleotide sequences (a) to (h) or (a') to (h')), a cloning site comprising one or more restriction enzyme recognition sites, an overlapping region for utilizing an In-Fusion cloning system of Clontech Laboratories, Inc. or a Gibson Assembly system of New England Biolabs, a nucleotide sequence of an exogenous gene or an endogenous gene, a nucleotide sequence of a selectable marker gene (auxotrophic complementary gene, drug resistance gene). The vectors of the present invention may further comprise, depending on a host, autonomous replication sequence (ARS), centromere DNA sequence, or telomeric DNA sequence.

The above specified nucleotide sequence can be comprised in the vector while inserted in an expression cassette. The "expression cassette" refers to an expression system comprising the above specified nucleotide sequence and capable of providing the state to express it as a polypeptide. The "state to express" refers to a state where the above specified nucleotide sequence comprised in the expression cassette is arranged under the control of the elements required for gene expression in such a way as to be expressed in a transformant. Examples of the element required for gene expression include a promoter, a terminator, and the like. The vectors of the present invention can be a cyclic vector, a linear vector, or an artificial chromosome.

The "promotor" herein refers to a nucleotide sequence region located upstream of the above specified nucleotide sequence, wherein various transcription regulators relating to the promotion and repression of transcription, in addition to a RNA polymerase, bind to or work on the region to read the above specified nucleotide sequence which is a template, whereby a complimentary RNA is synthesized (transcribed).

For the promoter expressing a polypeptide, a promotor achieving the expression using a selected carbon source is suitably used and not particularly limited.

When the carbon source is methanol, the promoter for expressing a polypeptide includes AOX1 promoter, AOX2 promoter, CAT promoter, DHAS promoter, FDH promoter, FMD promoter, GAP promoter, and MOX promoter, but is not particularly limited thereto.

When the carbon source is glucose or glycerol, the promoter for expressing a polypeptide include GAP promoter, TEF promoter, LEU2 promoter, URA3 promoter, ADE promoter, ADH1 promoter, and PGK1 promoter, but is not particularly limited thereto.

The vector of the present invention is typically constituted by ligating a nucleic acid fragment comprising the above specified nucleotide sequence or a nucleic acid fragment consisting of the above specified nucleotide sequence to one or more other functional nucleic acid fragments as described above at both ends or one end thereof via, for example, a restriction enzyme recognition site.

The scope of vector of the present invention encompasses, in addition to the form to which the above cloning site, overlapping region, nucleotide sequence of an exogenous gene or an endogenous gene, nucleotide sequence of a selectable marker gene, ARS, or centromere DNA sequence is added, nucleic acid molecules in the form to which these sequences can be added (for example, a form including a cloning site comprising one or more restriction enzyme recognition sites to which these sequences can be added).

The method for preparing the vector of the present invention is not particularly limited, but, for example, total synthesis, the PCR method, an In-Fusion cloning system of Clontech Laboratories, Inc. or a Gibson Assembly system of New England Biolabs can be used.

The method for introducing the vector into a host cell, i.e., the transformation method, can suitably be a known method, and examples include, when a yeast cell is used as a host, the electroporation method, the lithium acetate method, and the spheroplast method, but is not limited thereto. For example, the electroporation method described in High efficiency transformation by electroporation of *Pichia pastoris* pretreated with lithium acetate and dithiothreitol (Biotechniques. 2004 Jan; 36(1):152-4.) is a common transformation method of *Komagataella pastoris.*

Herein, the increase in a secretion amount of a protein from a host cell to a secretion amount of a protein in the parent cell or the wild-type strain may be, for example, 1.01 times, 1.02 times, 1.03 times, 1.04 times, 1.05 times, 1.1 times, 1.2 times, 1.3 times, 1.4 times, 1.5 times, 1.6 times, 1.7 times, 1.8 times, 1.9 times, 2 times, 2.5 times, 3 times, 3.5 times, 4 times, 4.5 times, or 5 times or more, and may be 100 times, 90 times, 80 times, 70 times, 60 times, 50 times, 40 times, 30 times, 20 times, 10 times, 9 times, 8 times, 7 times, 6 times, or 5 times or less. The secretion amounts of all proteins from the cell can be easily determined using a cell culture supernatant by a method known by a person skilled in the art such as the Bladford method, the Lowry method, and the BCA method. The secretion amount of a specific protein from the cell can be easily determined using a cell culture supernatant by the ELISA method.

The "parent cell", "wild-type strain", or "host cell before the introduction" as used herein means a host cell or a strain which is not treated for changing the expression of a gene comprising the nucleotide sequence as set forth in any of the above (a) to (h) and (a') to (h'). Thus, the "parent cell", "wild-type strain", or "host cell before the introduction" as used herein also includes a host cell or a strain in which a gene other than the gene comprising the nucleotide sequence as set forth in any of the above (a) to (h) and (a') to (h') is modified.

Herein, the "protein of interest" whose secretion is increased may be an endogenous protein of a host, or may be a heterologous protein or an exogenous protein. The "endogenous protein" as used herein refers to a protein produced during the culture of a host cell which is not genetically modified. On the contrary, the "heterologous protein" or the "exogenous protein" as used herein refers to a protein not typically expressed, or having an insufficient expression level or a secretion amount even when expressed in a host cell which is not genetically modified.

In one aspect, the present invention relates to a protein secretion enhancer consisting of the above vector. The protein secretion enhancer as used herein means a substance capable of increasing a secretion amount of a protein from a host cell when introduced into the host cell.

In one embodiment, the present invention relates to a composition for increasing a secretion amount of a protein in a host cell comprising the above vector or the protein secretion enhancer. The composition of the present invention may contain an excipient, a carrier, a binder, a disintegrator, a buffer, and a solvent known by a person skilled in the art, in addition to the above vector or the protein secretion enhancer.

In one aspect, the present invention relates to a use of the vector in the enhancement of protein secretion.

The vector of the present invention for expressing a polypeptide is useful in various uses such as host modification for the industrial purposes.

### 3. Mutant cell and cell comprising vector

In one aspect, the present invention relates to a mutant cell having an increased expression of a gene comprising the nucleotide sequence as set forth in any of the (a) to (h) and (a') to (h'), and an increased secretion amount of a protein. The mutant cell of the present invention may have an increased expression of a combination of 2 or more genes comprising the nucleotide sequence as set forth in the (a) to (h). Examples of the number of combination include 3 or more, 4 or more, 5 or more, 6 or more, 7 or more, 8 or more, 9 or more, or 10 or more. For example, the mutant cell of the present invention may have an increased expression of a combination of 2 or more sequences of the (a), i.e., the nucleotide sequence encoding the amino acid sequence as set forth in any of SEQ ID NOs; 113, 108 to 112, and 114 to 120, or genes comprising an equivalent sequence to any of these. Thus, the mutant cell of the present invention may have an increased expression of a combination of 2 or more genes comprising the sequence as set forth in the (a), a combination of 2 or more genes comprising the sequence as set forth in the (b), a combination of 2 or more genes comprising the sequence as set forth in the (c), or a combination of 2 or more genes comprising the sequence as set forth in the (d). Similarly, the mutant cell of the present invention may have an increased expression of a combination of 2 or more sequences of the (e), i.e., a nucleotide sequence encoding the amino acid sequence as set forth in any of SEQ ID NOs: 100, 95 to 99, and 101 to 107, or genes comprising an equivalent sequence to any of these. Thus, the mutant cell of the present invention may have an increased expression of a combination of 2 or more genes comprising the sequence as set forth in the (e), a combination of 2 or more genes comprising the sequence as set forth in the (f), a combination of 2 or more genes comprising the sequence as set forth in the (g), or a combination of 2 or more genes comprising the sequence as set forth in the (h). The mutant cell of the present invention may have an increased expression of a combination of 2 or more nucleotide sequences encoding the amino acid sequence as set forth in any of SEQ ID NOs: 113, 108, and 109, or genes comprising an equivalent sequence to any of these. The mutant cell of the present invention preferably has an increased expression of a combination of 2 or more nucleotide sequences encoding the amino acid sequence as set forth in any of SEQ ID NOs: 100, 95, and 96, or genes comprising an equivalent sequence to any of these.

Further, the mutant cell of the present invention may have an increased expression of a combination of 2 or more genes comprising the nucleotide sequence as set forth in the (a') to (h'). Examples of the number of combination include 3 or more, 4 or more, 5 or more, 6 or more, 7 or more, 8 or more, 9 or more, or 10 or more. For example, the mutant cell of the present invention may have an increased expression of a combination of 2 or more sequences of the (a'), i.e., the nucleotide sequence as set forth in any of SEQ ID NOs; 75, 65, 67, 69, 71, 73, 77, 79, 81, 83, 85, 87, and 89, or genes comprising an equivalent sequence to any of these. Thus, the mutant cell of the present invention may have an increased expression of a combination of 2 or more genes comprising the sequence as set forth in the (a'), a combination of 2 or more genes comprising the sequence as set forth in the (b'), a combination of 2 or more genes comprising the sequence as set forth in the (c'), or a combination of 2 or more genes comprising the sequence as set forth in the (d'). Similarly, the mutant cell of the present invention may have an increased expression of a combination of 2 or more sequences of the (e'), i.e., a nucleotide sequence as set forth in any of SEQ ID NOs: 39, 24, 27, 30, 33, 36, 42, 45, 48, 51, 54, 57, and 60, or genes comprising an equivalent sequence to any of these. Thus, the mutant cell of the present invention may have an increased expression of a combination of 2 or more genes comprising the sequence as set forth in the (e'), a combination of 2 or more genes comprising the sequence as set forth in the (f), a combination of 2 or more genes comprising the sequence as set forth in the (g'), or a combination of 2 or more genes comprising the sequence as set forth in the (h'). The mutant cell of the present invention may have an increased expression of a combination of 2 or more nucleotide sequences as set forth in any of SEQ ID NOs: 75, 65, and 67, or genes comprising an equivalent sequence to any of these. The mutant cell of the present invention preferably has an increased expression of a combination of 2 or more nucleotide sequences as set forth in any of SEQ ID NOs; 39, 24, and 27, or genes comprising an equivalent sequence to any of these.

The mutant cell of the present invention has an increased expression of the above genes and an increased secretion amount of a protein compared with that of the parent cell or the wild-type strain.

The increase in an expression level of the gene compared with an expression level of the gene in the parent cell or the wild-type strain may be, for example, 1.01 times, 1.02 times, 1.03 times, 1.04 times, 1.05 times, 1.1 times, 1.2 times, 1.3 times, 1.4 times, 1.5 times, 1.6 times, 1.7 times, 1.8 times, 1.9 times, 2 times, 2.5 times, 3 times, 3.5 times, 4 times, 4.5 times, or 5 times or more, and may be 100 times, 90 times, 80 times, 70 times, 60 times, 50 times, 40 times, 30 times, 20 times, 10 times, 9 times, 8 times, 7 times, 6 times, or 5 times or less. The expression level of the gene can be easily determined using a method known by a person skilled in the art such as the RT-PCR method and the Real-time PCR method.

The mutant cell of the present invention can be obtained by a method known by a person skilled in the art, for example, treating using ultraviolet irradiation or a chemical mutagen such as N-methyl-N'-nitrosoguanidine and subsequently screening the cells having an increased expression level of genes comprising the nucleotide sequence as set forth in any of the (a) to (h) and (a') to (h'). Further, the mutant cell of the present invention can also be obtained by transforming a host cell using the above vector. Furthermore, the mutant cell of the present invention can be obtained by disrupting or expressing at a high level of genes other than the gene comprising the nucleotide sequence as set forth in any of the (a) to (h) and (a') to (h'), and screening the cells having an increased expression level of genes comprising the nucleotide sequence as set forth in any of the (a) to (h) and (a') to (h').

In one embodiment, the present invention relates to a cell comprising the vector of the present invention according to the above section 2. The cell of the present invention may be, for example, a transformant obtained by transforming a host cell by using the above vector. The transformation method is as described in the above section 2.

After performing transformation, the selectable marker for selecting the transformant is not particularly limited. For example, when a yeast is used as a host cell, a vector comprising an auxotrophic complementary gene such as URA3 gene, LEU2 gene, ADE1 gene, HIS4 gene, or ARG4 gene is used as the selectable marker gene, and transformation is carried out using auxotrophic strains of uracil, leucine, adenine, histidine, and arginine, respectively as the host cell, and then the transformant can be selected by the recovery of prototrophic phenotype. Alternatively, when a vector comprising a drug resistance gene such as a G418 resistance gene, a Zeocin (tradename) resistance gene, or a hyglomycin resistance gene as the selectable marker gene is used, the transformant can be selected by the resistance on the medium containing G418, Zeocin (tradename), and hyglomycin, respectively. The auxotrophic selectable marker used for preparing a yeast host cannot be used when such a selectable marker in the host is not disrupted. In this instance, the selectable marker may be disrupted in the host, and a method known by a person skilled in the art can be used.

The cell of the present invention may be a transformant obtained by transforming the vector, or a progenic cell of such a transformant. The number of copies of the vector to be introduced into a single cell of the transformant is not particularly limited. A cell may comprise 1 copy or 2 or more multiple copies of vectors per cell. One copy of vector may exist as a cyclic vector, a linear vector, or an artificial chromosome, or may be incorporated into a chromosome derived from the host. Two or more multiple copies of vectors may all exist as cyclic vectors, linear vectors, or artificial chromosomes, or may all be incorporated into a chromosome derived from the host, or both states may occur simultaneously. The 2 or more multiple copies of vectors may be multiple copies of vectors having 2 or more copies of the same vector, or may be multiple copies of vectors having one or more copies of different vectors. For examples, the cell of the present invention may have in combination of 2 or more different vectors comprising the nucleotide sequence as set forth in any of the (a) to (h) and (a') to (h'), and may thereby have an increased expression of genes comprising the nucleotide sequence as set forth in any of the (a) to (h) and (a') to (h') in combination as described above.

In one aspect, the present invention relates to a method for preparing a mutant strain comprising a step of introducing the vector or the protein secretion enhancer of the present invention described in the above section 2 into a host cell, wherein the mutant strain has an increased secretion amount of a protein compared to that of the host cell before the introduction.

### 4. Method for producing protein of interest

In one aspect, the present invention relates to a method for producing a protein of interest comprising a step of culturing the cell described in the above section 3, and a step of recovering the protein of interest from a culture medium.

The "culture medium" as used herein means, in addition to a culture supernatant, a cultured cell, a cultured cell body, or a lysate of cell or cell body. Thus, the method for producing a protein of interest using the transformed yeast of the present invention includes a method comprising culturing the cell described in the above section 3 and allowing the protein to accumulate in the cell body thereof or the culture supernatant, preferably in the culture supernatant.

The cell culture conditions are not particularly limited and suitably selected depending on the cell. In the culture, any medium containing a nutrition source utilized by the cell can be used. Usable is a typical medium containing, as the nutrition source: a carbon source such as saccharides such as glucose, sucrose and maltose, organic acids such as lactic acid, acetic acid, citric acid, and propionic acid, alcohols such as methanol, ethanol, and glycerol, hydrocarbons such as a paraffin, oils such as a soybean oil and a rapeseed oil, or a mixture thereof; a nitrogen source such as ammonium sulfate, ammonium phosphate, urea, a yeast extract, a meat extract, peptone, and a corn steep liquor; and further nutrition sources such as other inorganic salts and vitamins, suitably mixed and added thereto. Additionally, the culture can be carried out by either batch culture or continuous culture.

As a preferable aspect of the present invention, the above carbon source may be 1 or 2 or more of glucose, glycerol, and methanol when a yeast belonging to the genus *Komagataella* or a yeast belonging to the genus *Ogataea* is used as the yeast. Additionally, these carbon sources may be present from the beginning of culture or may be added during the culture.

The "protein of interest" is a protein produced by the cell into which the vector of the present invention is introduced, and may be an endogenous protein of the host, or a heterologous protein. Examples of the protein of interest include enzymes derived from microorganisms, proteins produced by animals and plants which are multicellular organisms. Examples include phytase, protein A, protein G, protein L, amylase, glucosidase, cellulase, lipase, protease, glutaminase, peptidase, nuclease, oxidase, lactase, xylanase, trypsin, pectinase, isomerase, and fluorescent proteins, but are not limited thereto. Particularly, proteins for treating human and/or animals are preferable.

Examples of the protein for treating human and/or animals specifically include a hepatitis B virus surface antigen, hirudin, an antibody, a human antibody, a partial antibody, a human partial antibody, serum albumin, human serum albumin, an epidermal growth factor, a human epidermal growth factor, insulin, a growth hormone, erythropoietin, interferon, blood coagulation factor VIII, granulocyte-colony stimulating factor (G-CSF), granulocyte-macrophage colony stimulating factor (GM-CSF), thrombopoietin, IL-1, IL-6, tissue plasminogen activator (TPA), urokinase, leptin, and stem cell factor (SCF).

The "antibody" used herein refers to a heterotetramer protein constituted by polypeptide chains of 2 of each the L chain and H chain being disulfide bonded, and is not particularly limited as long as it has an binding ability to a specific antigen.

The "partial antibody" used herein refers to Fab antibody, (Fab)2 antibody, scFv antibody, diabody antibody, and derivatives thereof, and is not particularly limited as long as it has a binding ability to a specific antigen. The Fab antibody refers to a heteromer protein wherein the L chain and the Fd chain of the antibody are bound by an S-S bond, or a heteromer protein wherein the L chain and the Fd chain of the antibody associate without comprising an S-S bond, and is not particularly limited as long as it has an binding ability to a specific antigen.

The amino acid constituting the above protein of interest may be naturally occurred, non-naturally occurred, or modified. Additionally, the amino acid sequence of the protein may be artificially modified or de-novo designed.

A protein of interest is allowed to accumulate in a host or culture medium by culturing the transformant obtained by introducing the vector of the present invention into the cell, and can be recovered. For the method for recovering a protein of interest, known purification methods can be used in a suitable combination. For example, the transformed yeast is cultured in a suitable medium, and the cell body are removed from the culture supernatant by centrifugation of the culture medium or filtration treatment. A protein of interest is recovered from the culture supernatant by subjecting the obtained culture supernatant to a process singly or in combination such as salting out (ammonium sulfate precipitation or sodium phosphate precipitation), solvent precipitation (protein fractional precipitation such as acetone or ethanol), dialysis, gel filtration chromatography, ion exchange chromatography, hydrophobic chromatography, affinity chromatography, reversed phase chromatography, or ultrafiltration.

The cell culture can be typically carried out under general conditions, and, for examples, in the case of a yeast, can be carried out by aerobically culturing at pH 2.5 to 10.0 in a temperature range from 10°C to 48°C for 10 hours to 10 days.

The recovered protein of interest can be used directly, but can also be used after adding a modification for causing a pharmacological change such as PEGylation or a modification for adding a function such as that of an enzyme or an isotope. Additionally, various formulation treatments may be used.

To secrete a non-secretory protein of interest outside the cell body, a nucleotide sequence encoding a signal sequence may be introduced to the 5'-end of the protein of interest gene. The nucleotide sequence encoding a signal sequence is not particularly limited as long as a nucleotide sequence encodes a signal sequence by which the yeast secretory expression is achieved, but examples include Mating Factor α (MF α) of *Saccharomyces cerevisiae*, acid phosphatase of *Ogataea angusta* (PHO1), acid phosphatase of *Komagataella pastoris* (PHO1), invertase of *Saccharomyces cerevisiae* (SUC2), PLB1 of *Saccharomyces cerevisiae,* bovine serum albumin (BSA), human serum albumin (HSA), and a nucleotide sequence encoding a signal sequence of an immunoglobulin.

The promoter for a protein of interest in the present invention is not particularly limited as long as the promoter expresses, preferably expresses at a high level of the protein of interest, in the transformant. A methanol-inducible promoter is preferable. The methanol-inducible promoter is not particularly limited as long as a promoter has a transcriptional activity when the carbon source is methanol, and examples preferably include AOX1 promoter, AOX2 promoter, CAT promoter, DHAS promoter, FDH promoter, FMD promoter, GAP promoter, and MOX promoter.

The expression at a high level of a polypeptide can be achieved by a known technique such as, in addition to the expression induction of a nucleotide sequence encoding the polypeptide on the chromosome and/or the vector, a modification of a nucleotide sequence encoding a polypeptide on the chromosome and/or the vector (increase in the number of copies, insertion of a promoter, or codon modification), introduction of a nucleotide sequence encoding the polypeptide into a host cell, or obtaining a strain expressing polypeptide at a high level by introducing mutation(s) into a host cell.

The modification of a nucleotide sequence encoding a polypeptide on the chromosome can be carried out using a technique such as gene insertion utilizing the homologous recombination between the host chromosomes or site-specific mutagenesis. For example, such a modification can be carried out by introducing a nucleotide sequence encoding the polypeptide into a chromosome for increasing the number of copies, substituting the promoter with a much stronger promoter, or modifying a nucleotide sequence encoding a polypeptide to a codon suitable for a host cell.

Hereinafter, the present invention is described in detail in reference to Examples, but the present invention shall not be limited thereto.

### [Examples]

### <Example 1: Preparation of various genes used for preparing vectors>

A detailed engineering method and the like related to recombinant DNA technology used in the Examples below are described in the following books: Molecular Cloning 2nd Edition (Cold Spring Harbor Laboratory Press, 1989) and Current Protocols in Molecular Biology (Greene Publishing Associates and Wiley-Interscience).

In the Examples below, a plasmid used for transforming a yeast was prepared by introducing a constructed vector into an *Escherichia coli* (*E. coli*) DH5α, competent cell (from Takara Bio Inc.) and amplifying a resulting transformant by culturing the transformant. Preparation of the plasmid from the strain carrying the plasmid was performed by using QIAprep spin miniprep kit (from QIAGEN).

An AOX1 promoter (SEQ ID NO: 2), an AOX1 terminator (SEQ ID NO: 5), an HIS4 sequence (SEQ ID NO: 8), a GAP promoter (SEQ ID NO: 15), a nucleotide sequence encoding a polypeptide represented by ACCESSION No. CCA36173 (SEQ ID NO: 24), a nucleotide sequence encoding a polypeptide represented by ACCESSION No. CCA37695 (SEQ ID NO: 27), a nucleotide sequence encoding a polypeptide represented by ACCESSION No. CCA41161 (SEQ ID NO: 30), a nucleotide sequence encoding a polypeptide represented by ACCESSION No. CCA41167 (SEQ ID NO: 33), a nucleotide sequence encoding a polypeptide represented by ACCESSION No. CCA37701 (SEQ ID NO: 36), a nucleotide sequence encoding a polypeptide represented by ACCESSION No. CCA40175 (SEQ ID NO: 39), a nucleotide sequence encoding a polypeptide represented by ACCESSION No. CCA37509 (SEQ ID NO: 42), a nucleotide sequence encoding a polypeptide represented by ACCESSION No. CCA38967 (SEQ ID NO: 45), a nucleotide sequence encoding a polypeptide represented by ACCESSION No. CCA37504 (SEQ ID NO: 48), and a CCA38473 terminator (SEQ ID NO: 21) were used for vector construction. These sequences were prepared by PCR using as a template a mixture of chromosomal DNA from *Komagataella pastoris* strain ATCC76273 (the nucleotide sequence thereof is described in EMBL (The European Molecular Biology Laboratory) ACCESSION No. FR839628 to FR839631). The AOX1 promoter was prepared by PCR using primer 1 (SEQ ID NO: 3) and primer 2 (SEQ ID NO: 4). The AOX1 terminator was prepared by PCR using primer 3 (SEQ ID NO: 6) and primer 4 (SEQ ID NO: 7). The HIS4 sequence was prepared by PCR using primer 5 (SEQ ID NO: 9) and primer 6 (SEQ ID NO: 10). The GAP promoter was prepared by PCR using primer 9 (SEQ ID NO: 16) and primer 10 (SEQ ID NO: 17). The CCA38473 terminator was prepared by PCR using primer 13 (SEQ ID NO: 22) and primer 14 (SEQ ID NO: 23).

A Zeocin (TM)-resistance gene under promoter control (SEQ ID NO: 18), which was used for vector construction, was prepared by PCR using synthetic DNA as a template. An anti-β galactosidase single-chain antibody gene having a Mating Factorα pre-pro signal sequence added thereto (SEQ ID NO: 11), which was used for vector construction, was prepared by PCR using synthetic DNA as a template, based on a published sequence database (J Mol Biol. 1998 Jul 3;280(1):117-27.). A nucleotide sequence encoding a polypeptide represented by ACCESSION No. CAY67126 (SEQ ID NO: 51), a nucleotide sequence encoding a polypeptide represented by ACCESSION No. CAY68445 (SEQ ID NO: 54), a nucleotide sequence encoding a polypeptide represented by ACCESSION No. CAY68608 (SEQ ID NO: 57), and a nucleotide sequence encoding a polypeptide represented by ACCESSION No. CAY71233 (SEQ ID NO: 60), which were used for vector construction, were prepared by PCR using synthetic DNA as a template.

PCR was performed by using Prime STAR HS DNA Polymerase (from Takara Bio Inc.) etc. under a reaction condition described in the accompanying manual. Preparation of the chromosomal DNA from *Komagataella pastoris* strain ATCC76273 was performed by using Dr. GenTLE (TM) (from Takara Bio Inc.) etc. under the condition described therein.

### <Example 2: Construction of anti-β galactosidase single-chain antibody expression vector>

A gene fragment having a multiple cloning site, HindIII-BamHI-BglII-XbaI-EcoRI, (SEQ ID NO: 1) was totally synthesized and this was inserted into a HindIII-EcoRI site of pUC19 (from Takara Bio Inc., Code No. 3219), thereby constructing pUC-1.

Furthermore, a nucleic acid fragment in which BamHI recognition sequences were added to both sides of an AOX1 promoter (SEQ ID NO: 2) was prepared by PCR using primer 1 (SEQ ID NO: 3) and primer 2 (SEQ ID NO: 4) and inserted into the BamHI site of pUC-1 after treatment with BamHI, thereby constructing pUC-Paox.

Then, a nucleic acid fragment in which XbaI recognition sequences were added to both sides of an AOX1 terminator (SEQ ID NO: 5) was prepared by PCR using primer 3 (SEQ ID NO: 6) and primer 4 (SEQ ID NO: 7) and inserted into the XbaI site of pUC-Paox after treatment with XbaI, thereby constructing pUC-PaoxTaox.

Then, a nucleic acid fragment in which EcoRI recognition sequences were added to both sides of an HIS4 sequence (SEQ ID NO: 8) was prepared by PCR using primer 5 (SEQ ID NO: 9) and primer 6 (SEQ ID NO: 10) and inserted into the EcoRI site of pUC-PaoxTaox after treatment with EcoRI, thereby constructing pUC-PaoxTaoxHIS4.

Then, a nucleic acid fragment in which BglII recognition sequences were added to both sides of an anti-β galactosidase single-chain antibody gene having a Mating Factorα pre-pro signal sequence added thereto (SEQ ID NO: 11) was prepared by PCR using primer 7 (SEQ ID NO: 12) and primer 8 (SEQ ID NO: 13) and inserted into the BglII site of pUC-PaoxTaoxHIS4 after treatment with BglII, thereby constructing pUC-PaoxscFvTaoxHIS4. This pUC-PaoxscFvTaoxHIS4 is designed to express the anti-β galactosidase single-chain antibody under the control of the AOX1 promoter.

### <Example 3: Construction of polypeptide expression vectors>

A gene fragment having a multiple cloning site, HindIII-BamHI-SpeI-XbaI-EcoRI, (SEQ ID NO: 14) was totally synthesized and this was inserted into a HindIII-EcoRI site of pUC19 (from Takara Bio Inc., Code No. 3219), thereby constructing pUC-2.

Furthermore, a nucleic acid fragment in which BamHI recognition sequences were added to both sides of a GAP promoter (SEQ ID NO: 15) was prepared by PCR using primer 9 (SEQ ID NO: 16) and primer 10 (SEQ ID NO: 17) and inserted into the BamHI site of pUC-2 after treatment with BamHI, thereby constructing pUC-Pgap.

Then, a nucleic acid fragment in which EcoRI recognition sequences were added to both sides of a Zeocin (TM)-resistance gene under promoter control (SEQ ID NO: 18) was prepared by PCR using primer 11 (SEQ ID NO: 19) and primer 12 (SEQ ID NO: 20) and inserted into the EcoRI site of pUC-Pgap after treatment with EcoRI, thereby constructing pUC-PgapZeo.

Then, a nucleic acid fragment in which XbaI recognition sequences were added to both sides of a CCA38473 terminator (SEQ ID NO: 21) was prepared by PCR using primer 13 (SEQ ID NO: 22) and primer 14 (SEQ ID NO: 23) and inserted into the XbaI site of pUC-PgapZeo after treatment with XbaI, thereby constructing pUC-PgapT38473Zeo.

Then, nucleotide sequences encoding novel polypeptides listed in Table 1 were prepared by PCR using a mixture of chromosomal DNA from *Komagataella pastoris* strain ATCC76273 (the nucleotide sequences thereof are described under in EMBL (The European Molecular Biology Laboratory) ACCESSION No. FR839628 to FR839631) or synthetic DNA as a template. The ACCESSION Nos. of the novel polypeptides, amino acid sequences of the novel polypeptides, nucleotide sequences encoding the novel polypeptides, the numbers and sequences of the primers (Fw and Rev) used for amplification of the nucleic acid fragments, and the templates are shown in Table 1 below.

**[Table 1]**

| ACCESSION NO. | Amino acid sequence | Nucleotide sequence | Number and sequence of primers (Fw) | Number and sequence of primers (Rev) | Template |
|---|---|---|---|---|---|
| CCA36173 | SEQ ID NO:95 | SEQ ID NO:24 | 15(SEQ ID NO:25) | 16(SEQ ID NO:26) | Chromosomal DNA of strain ATCC76273 |
| CCA37695 | SEQ ID NO:96 | SEQ ID NO:27 | 17(SEQ ID NO:28) | 18(SEQ ID NO:29) | Chromosomal DNA of strain ATCC76273 |
| CCA41161 | SEQ ID NO:97 | SEQ ID NO:30 | 19(SEQ ID NO:31) | 20(SEQ ID NO:32) | Chromosomal DNA of strain ATCC76273 |
| CCA41167 | SEQ ID NO:98 | SEQ ID NO:33 | 21(SEQ ID NO:34) | 22(SEQ ID NO:35) | Chromosomal DNA of strain ATCC76273 |
| CCA37701 | SEQ ID NO:99 | SEQ ID NO:36 | 23(SEQ ID NO:37) | 24(SEQ ID NO:38) | Chromosomal DNA of strain ATCC76273 |
| CCA40175 | SEQ ID NO:100 | SEQ ID NO:39 | 25(SEQ ID NO:40) | 26(SEQ ID NO:41) | Chromosomal DNA of strain ATCC76273 |
| CCA37509 | SEQ ID NO:101 | SEQ ID NO:42 | 27(SEQ ID NO:43) | 28(SEQ ID NO:44) | Chromosomal DNA of strain ATCC76273 |
| CCA38967 | SEQ ID NO:102 | SEQ ID NO:45 | 29(SEQ ID NO:46) | 30(SEQ ID NO:47) | Chromosomal DNA of strain ATCC76273 |
| CCA37504 | SEQ ID NO:103 | SEQ ID NO:48 | 31(SEQ ID NO:49) | 32(SEQ ID NO:50) | Chromosomal DNA of strain ATCC76273 |
| CAY67126 | SEQ ID NO:104 | SEQ ID NO:51 | 33(SEQ ID NO:52) | 34(SEQ ID NO:53) | Synthetic DNA |
| CAY68445 | SEQ ID NO:105 | SEQ ID NO:54 | 35(SEQ ID NO:55) | 36(SEQ ID NO:56) | Synthetic DNA |
| CAY68608 | SEQ ID NO:106 | SEQ ID NO:57 | 37(SEQ ID NO:58) | 38(SEQ ID NO:59) | Synthetic DNA |
| CAY71233 | SEQ ID NO:107 | SEQ ID NO:60 | 39(SEQ ID NO:61) | 40(SEQ ID NO:62) | Synthetic DNA |

In each of these nucleic acid fragments, the GAP promoter sequence is added, as an overlapping region, upstream of the nucleotide sequence encoding the novel polypeptide and the CCA38473 terminator sequence is added, as an overlapping region, downstream of the nucleotide sequence encoding the novel polypeptide.

After treating pUC-PgapT38473Zeo with SpeI, a nucleic acid fragment was prepared by PCR using a Re primer for the GAP promoter (primer 41 (SEQ ID NO: 63)) and a Fw primer for the CCA38473 terminator (primer 42 (SEQ ID NO: 64)). This nucleic acid fragment was mixed with the nucleic acid fragment prepared by PCR that has the above-mentioned nucleotide sequence encoding the novel polypeptide. Then, these fragments were linked together by using a Gibson Assembly system from New England Biolabs Inc., thereby constructing pUC-PgapCCA36173T38473Zeo, pUC-PgapCCA37695T38473Zeo, pUC-PgapCCA41161T38473Zeo, pUC-PgapCCA41167T38473Zeo, pUC-PgapCCA37701T38473Zeo, pUC-PgapCCA40175T38473Zeo, pUC-PgapCCA37509T3 8473Zeo, pUC-PgapCCA3 8967T3 8473Zeo, pUC-PgapCCA37504T38473Zeo, pUC-PgapCAY67126T38473Zeo, pUC-PgapCAY68445T38473Zeo, pUC-PgapCAY68608T38473Zeo, or pUC-PgapCAY71233T38473Zeo. These vectors are designed to express each polypeptide under the control of the GAP promoter.

### <Example 4: Generation of transformed yeast>

The anti-β galactosidase single-chain antibody expression vector pUC-PaoxscFvTaoxHIS4 constructed in Example 2 and the polypeptide expression vectors constructed in Example 3 were used to transform *Komagataella pastoris* as described below.

A histidine auxotrophic strain derived from *Komagataella pastoris* strain ATCC76273 was inoculated in 3 ml of YPD medium (1% yeast extract bacto (from *Becton, Dickinson and Company*), 2% polypeptone (from NIHON PHARMACEUTICAL CO., LTD.), 2% glucose) and cultured with shaking overnight at 30°C to obtain a preculture medium. 500 µl of the preculture medium thus obtained was inoculated in 50 ml of YPD medium and cultured with shaking up to an OD600 of 1 to 1.5. Then, the yeast was harvested (3000 ×g, 10 minutes, 20°C) and resuspended in 10 ml of 50 mM potassium phosphate buffer, pH 7.5 supplemented with 250 µl of 1M DTT (final concentration of 25 mM).

After incubating this suspension for 15 minutes at 30°C, the yeast was harvested (3000 ×g, 10 minutes, 20°C) and washed with 50 ml of STM buffer precooled in ice (270 mM sucrose, 10 mM Tris-HCl, 1 mM magnesium chloride, pH7.5). After harvesting the yeast from the washing solution (3000 ×g, 10 minutes, 4°C) and washing again with 25 ml of STM buffer, the yeast was harvested (3000 ×g, 10 minutes, 4°C). Finally, the obtained yeast was suspended in 250 µl of the ice cold STM buffer and this suspension was used as a competent cell solution.

*E. coli* was transformed by using the anti-β galactosidase single-chain antibody expression vector pUC-PaoxscFvTaoxHIS4 constructed in Example 2 and the transformant obtained was cultured in 5 ml of 2YT medium containing ampicillin (1.6% tryptone bacto (from *Becton, Dickinson and Company),* 1% yeast extract bacto (from *Becton, Dickinson and Company*), 0.5% sodium chloride, 0.01% ampicillin sodium (from FUJIFILM Wako Pure Chemical Corporation)). pUC-PaoxscFvTaoxHIS4 was obtained from the resulting cells by using a QIAprep spin miniprep kit (from QIAGEN). This plasmid was linearized with SacI treatment by utilizing a SacI recognition sequence within an AOX1 promoter.

60 µl of the above-mentioned competent cell solution and 1 µl of solution of the linearized pUC-PaoxscFvTaoxHIS4 were mixed and transferred into an electroporation cuvette (disposable cuvette electrode, electrode gap of 2 mm (from BM Equipment Co., Ltd.)). After the mixture was subjected to electroporation at 7.5 kV/cm, 25 µF, and 200 Ω, cell bodies were suspended in 1 ml of YPD medium and allowed to stand for one hour at 30°C. After allowing to stand for one hour, the yeast was harvested (3000 ×g, 5 minutes, 20°C) and suspended in 1 ml of YNB medium (0.67% yeast nitrogen base Without Amino Acid (from Becton, Dickinson and Company). Then, yeast was harvested again (3000 ×g, 5 minutes, 20°C). The cell bodies were resuspended in an adequate amount of YNB medium and then spread on a selective YNB agar plate (0.67% yeast nitrogen base Without Amino Acid (from Becton, Dickinson and Company), 1.5% agarose, 2% glucose). A strain that grew in static culture for 3 days at 30°C was selected to obtain a yeast expressing the anti-β galactosidase single-chain antibody.

Subsequently, *Komagataella pastoris* strain ATCC76273 and the yeast expressing the anti-β galactosidase single-chain antibody were inoculated individually in 3 ml of YPD medium (1% yeast extract bacto (from *Becton, Dickinson and Company),* 2% polypeptone (from NIHON PHARMACEUTICAL CO., LTD.), 2% glucose) and cultured with shaking overnight at 30°C to obtain a preculture medium. 500 µl of the preculture medium thus obtained was inoculated in 50 ml of YPD medium and cultured with shaking up to an OD600 of 1 to 1.5. Then, The yeast was harvested (3000 ×g, 10 minutes, 20°C) and resuspended in 10 ml of 50 mM potassium phosphate buffer, pH 7.5 supplemented with 250 µl of 1M DTT (final concentration of 25 mM).

After incubating this suspension for 15 minutes at 30°C, the yeast was harvested (3000 ×g, 10 minutes, 20°C) and washed with 50 ml of STM buffer precooled in ice (270 mM sucrose, 10 mM Tris-HCl, 1 mM magnesium chloride, pH7.5). After harvesting the yeast from the washing solution (3000 ×g, 10 minutes, 4°C) and washing again with 25 ml of STM buffer, the yeast was harvested (3000 ×g, 10 minutes, 4°C). Finally, the obtained yeast was suspended in 250 µl of the ice cold STM buffer and this suspension was used as a competent cell solution.

*E. coli* was transformed by using the polypeptide expression vectors constructed in Example 3 and the obtained transformant was cultured in 5 ml of 2YT medium containing ampicillin (1.6% tryptone bacto (from *Becton, Dickinson and Company),* 1% yeast extract bacto (from *Becton, Dickinson and Company),* 0.5% sodium chloride, 0.01% ampicillin sodium (from FUJIFILM Wako Pure Chemical Corporation)). Each polypeptide expression vector was obtained from the resulting cell bodies by using a QIAprep spin miniprep kit (from QIAGEN). This plasmid was linearized with NruI treatment by utilizing an NruI recognition sequence within a CCA38473 terminator.

60 µl of the above-mentioned competent cell solution and 1 µl of solution of the linearized polypeptide expression vector were mixed and transferred into the electroporation cuvette (disposable cuvette electrode, electrode gap of 2 mm (from BM Equipment Co., Ltd.)). After the mixture was subjected to electroporation at 7.5 kV/cm, 25 µF, and 200 Ω, cell bodies were suspended in 1 ml of YPD medium and allowed to stand for one hour at 30°C. After allowing to stand for one hour, the yeast was harvested (3000 ×g, 5 minutes, 20°C) and 961 µl of the supernatant was discarded. The yeast was resuspended in 100 µl of the remaining solution and then 100 µl of the suspension was spread on a selective YPDZeocin (TM) agar plate (1% yeast extract bacto (from Becton, Dickinson and Company), 2% polypeptone (from NIHON PHARMACEUTICAL CO., LTD.), 2% glucose, 1.5% agarose, 0.01% Zeocin (TM) (from Thermo Fisher Scientific Inc.). A strain that grew in static culture for 3 days at 30°C was selected to obtain a yeast expressing the polypeptide and a yeast expressing both the anti-β galactosidase single-chain antibody and the polypeptide.

### <Example 5: Construction of expression vector of c-terminally deleted polypeptide>

A nucleotide sequence encoding a polypeptide, in which C-terminal was deleted, was prepared by PCR using the polypeptide expression vector constructed in Example 3 as a template. The alignment of amino acid sequences of novel polypeptides and deleted C-terminal regions having relatively high homology to one another are shown in Figure 1. The name of the C-terminally deleted polypeptides, the amino acid sequences of the polypeptides, the nucleotide sequences encoding the polypeptides, the sequences of primers used for amplification of nucleic acid fragments, and the templates are shown in Table 2 below.

**[Table 2]**

| Name | Amino acid sequence | Nucleotide sequence | Number and sequence of primers(Fw) | Number and sequence of primers(Rev) | Template |
|---|---|---|---|---|---|
| CCA36173Cterdel | SEQ ID NO:108 | SEQ ID NO:65 | 15(SEQ ID NO:25) | 43(SEQ ID NO:66) | pUC-PgapCCA 36173T38473Zeo |
| CCA37695Cterdel | SEQ ID NO:109 | SEQ ID NO:67 | 17(SEQ ID NO:28) | 44(SEQ ID NO:68) | pUC-PgapCCA 37695T38473Zeo |
| CCA41161Cterdel | SEQ ID NO:110 | SEQ ID NO:69 | 19(SEQ ID NO:31) | 45(SEQ ID NO:70) | pUC-PgapCCA 41161T38473Zeo |
| CCA41167Cterdel | SEQ ID NO:111 | SEQ ID NO:71 | 21(SEQ ID NO:34) | 46(SEQ ID NO:72) | pUC-PgapCCA 41167T38473Zeo |
| CCA37701Cterdel | SEQ ID NO:112 | SEQ ID NO:73 | 23(SEQ ID NO:37) | 47(SEQ ID NO:74) | pUC-PgapCCA 37701T38473Zeo |
| CCA40175Cterdel | SEQ ID NO:113 | SEQ ID NO:75 | 25(SEQ ID NO:40) | 48(SEQ ID NO:76) | pUC-PgapCCA 40175T38473Zeo |
| CCA37509Cterdel | SEQ ID NO:114 | SEQ ID NO:77 | 27(SEQ ID NO:43) | 49(SEQ ID NO:78) | pUC-PgapCCA 37509T38473Zeo |
| CCA38967Cterdel | SEQ ID NO:115 | SEQ ID NO:79 | 29(SEQ ID NO:46) | 50(SEQ ID NO:80) | pUC-PgapCCA 38967T38473Zeo |
| CCA37504Cterdel | SEQ ID NO:116 | SEQ ID NO:81 | 31(SEQ ID NO:49) | 51(SEQ ID NO:82) | pUC-PgapCCA 37504T38473Zeo |
| CAY67126Cterdel | SEQ ID NO:117 | SEQ ID NO:83 | 33(SEQ ID NO:52) | 52(SEQ ID NO:84) | pUC-PgapCAY 67126T38473Zeo |
| CAY68445Cterdel | SEQ ID NO:118 | SEQ ID NO:85 | 35(SEQ ID NO:55) | 53(SEQ ID NO:86) | pUC-PgapCAY 68445T38473Zeo |
| CAY68608Cterdel | SEQ ID NO:119 | SEQ ID NO:87 | 37(SEQ ID NO:58) | 54(SEQ ID NO:88) | pUC-PgapCAY 68608T38473Zeo |
| CAY71233Cterdel | SEQ ID NO:120 | SEQ ID NO:89 | 39(SEQ ID NO:61) | 55(SEQ ID NO:90) | pUC-PgapCAY 71233T38473Zeo |

In each of these nucleic acid fragments, a GAP promoter sequence is added, as an overlapping region, upstream of the nucleotide sequence encoding the polypeptide and a CCA38473 terminator sequence is added, as an overlapping region, downstream of the nucleotide sequence encoding the polypeptide.

After treating pUC-PgapT38473Zeo constructed in Example 3 with SpeI, a nucleic acid fragment was prepared by PCR using a Re primer for the GAP promoter (primer 41 (SEQ ID NO: 63)) and a Fw primer for the CCA38473 terminator (primer 42 (SEQ ID NO: 64)). This nucleic acid fragment was mixed with the nucleic acid fragment prepared by PCR that has the above-mentioned nucleotide sequence encoding the C-terminally deleted polypeptide. Then, these fragments were linked together by using a Gibson Assembly system from New England Biolabs Inc., thereby constructing pUC-PgapCCA36173CterdelT38473Zeo, pUC-PgapCCA37695CterdelT38473Zeo, pUC-PgapCCA41161CterdelT38473Zeo, pUC-PgapCCA41167CterdelT38473Zeo, pUC-PgapCCA37701CterdelT38473Zeo, pUC-PgapCCA40175CterdelT38473Zeo, pUC-PgapCCA37509CterdelT38473Zeo, pUC-PgapCCA38967CterdelT38473Zeo, pUC-PgapCCA37504CterdelT38473Zeo, pUC-PgapCAY67126CterdelT38473Zeo, pUC-PgapCAY68445CterdelT38473Zeo, pUC-PgapCAY68608CterdelT38473Zeo, or pUC-PgapCAY71233CterdelT38473Zeo. These vectors are designed to express each C-terminally deleted polypeptide under the control of the GAP promoter.

### <Example 6: Generation of transformed yeast>

The yeast expressing an anti-β galactosidase single-chain antibody obtained in Example 4 was inoculated in 3 ml of YPD medium (1% yeast extract bacto (from *Becton*, *Dickinson and Company*), 2% polypeptone (from NIHON PHARMACEUTICAL CO., LTD.), 2% glucose) and cultured with shaking overnight at 30°C to obtain a preculture medium. 500 µl of the preculture medium thus obtained was inoculated in 50 ml of YPD medium and cultured with shaking up to an OD600 of 1 to 1.5. Then, the yeast was harvested (3000 ×g, 10 minutes, 20°C) and resuspended in 10 ml of 50 mM potassium phosphate buffer, pH 7.5 supplemented with 250 µl of 1M DTT (final concentration of 25 mM).

After incubating this suspension for 15 minutes at 30°C, the yeast was harvested (3000 ×g, 10 minutes, 20°C) and washed with 50 ml of STM buffer precooled in ice (270 mM sucrose, 10 mM Tris-HCl, 1 mM magnesium chloride, pH7.5). After harvesting the yeast from the washing solution (3000 ×g, 10 minutes, 4°C) and washing again with 25 ml of STM buffer, the yeast was harvested (3000 ×g, 10 minutes, 4°C). Finally, the obtained yeast was suspended in 250 µl of the ice cold STM buffer and this suspension was used as a competent cell solution.

Some of the expression vectors of C-terminally deleted polypeptides constructed in Example 5 were selected randomly and used to transform *E. coli.* The transformant obtained was cultured in 5 ml of 2YT medium containing ampicillin (1.6% tryptone bacto (from *Becton, Dickinson and Company*), 1% yeast extract bacto (from *Becton, Dickinson and Company),* 0.5% sodium chloride, 0.01% ampicillin sodium (from FUJIFILM Wako Pure Chemical Corporation)). Each of the expression vectors of C-terminally deleted polypeptides was obtained from the resulting cell bodies by using a QIAprep spin miniprep kit (from QIAGEN). This plasmid was linearized with NruI treatment by utilizing an NruI recognition sequence within a CCA38473 terminator.

60 µl of the above-mentioned competent cell solution and 1 µl of solution of the linearized expression vector of the C-terminally deleted polypeptide were mixed and transferred into an electroporation cuvette (disposable cuvette electrode, electrode gap of 2 mm (from BM Equipment Co., Ltd.)). After the mixture was subjected to electroporation at 7.5 kV/cm, 25 µF, and 200 Ω, cell bodies were suspended in 1 ml of YPD medium and allowed to stand for one hour at 30°C. After allowing to stand for one hour, the yeast was harvested (3000 ×g, 5 minutes, 20°C) and 961 µl of the supernatant was discarded. The yeast was resuspended in 100 µl of the remaining solution and then 100 µl of the suspension was spread on a selective YPDZeocin (TM) agar plate (1% yeast extract bacto (from Becton, Dickinson and Company), 2% polypeptone (from NIHON PHARMACEUTICAL CO., LTD.), 2% glucose, 1.5% agarose, 0.01% Zeocin (TM) (from Thermo Fisher Scientific Inc.). A strain that grew in static culture for 3 days at 30°C was selected to obtain a yeast expressing both the anti-β galactosidase single-chain antibody and the C-terminally deleted polypeptide.

### <Example 7: Culture of Transformed Yeast>

*Komagataella pastoris* strain ATCC76273, or the yeast expressing an anti-β galactosidase single-chain antibody, the yeast expressing a polypeptide, the yeast expressing an anti-β galactosidase single-chain antibody and a polypeptide, or the yeast expressing an anti-β galactosidase single-chain antibody and a C-terminally deleted polypeptide, which were obtained in Examples 4 and 6, was inoculated in 2 ml of BMMY medium (1% yeast extract bacto (from Becton, Dickinson and Company), 2% polypeptone (from NIHON PHARMACEUTICAL CO., LTD.), 0.34% yeast nitrogen base Without Amino Acid and Ammonium Sulfate (from Becton, Dickinson and Company), 1% Ammonium Sulfate, 0.4 mg/l Biotin, 100 mM potassium phosphate (pH6.0), 2% Methanol). After culturing with shaking at 170 rpm for 72 hours at 30°C, culture supernatant was collected by centrifugation (12000 rpm, 5 minutes, 4°C). The cell body concentration was determined based on OD600.

### <Example 8: Measurement of amount of protein secreted in culture supernatant by Bradford assay

The expression level of proteins secreted into culture supernatant in Example 7 was determined by a Bradford assay as described below.

300 µl of culture supernatant of *Komagataella pastoris* strain ATCC76273 and 300 µl of culture supernatant of a yeast expressing a polypeptide were applied onto a centrifugal filter (Amicon Ultra-0.5, PLGC Ultracel-10 membrane, 10 kDa (from Merck Millipore)) and centrifuged (14000 ×g, 20 minutes, 4°C). The flow-through was discarded and 300 µl of PBS buffer (8 g/L sodium chloride, 0.2g/L potassium chloride, 1.15 g/L sodium hydrogenphosphate (anhydrous), 0.2 g/L potassium dihydrogenphosphate (anhydrous)) was added to the centrifugal filter. After washing the filter, centrifugation was performed (14000 ×g, 20 minutes, 4°C). The flow-through was discarded and 300 µl of PBS buffer was added to the centrifugal filter. After washing the filter, centrifugation was performed (14000 ×g, 20 minutes, 4°C). The filter was inverted and placed in a new tube and centrifugation was performed (1000 ×g, 2 minutes, 4°C) to recover the remaining solution of the secreted protein. PBS buffer was added to this solution of the secreted protein to make 300 µl.

Serially diluted standard bovine serum albumin and diluted solution of the secreted protein were added to wells of a microplate (a cell culture plate from TPP) at 150 µL/well. 150 µl of 1 × dye reagent (Quick Start Protein Assay from Bio-Rad Laboratories, Inc.) was added thereto and allowed to react for 5 minutes at room temperature. Then, the absorbance at 595 nm was measured using a microplate reader (Spectra Max Paradigm from Molecular Devices, LLC.). Quantification of the secreted proteins was performed by using a standard curve of the standard bovine serum albumin. The expression level of the secreted proteins determined by this method and their respective cell body concentrations (OD600) were shown in Table 3.

In Table 3, control (1) represents the results when the culture supernatant of *Komagataella pastoris* strain ATCC76273 was used and (2) to (14) represents the results when the culture supernatants of *Komagataella pastoris* strain ATCC76273 transformed by expression vectors of the respective polypeptides were used.

**[Table 3]**

| Expressed protein | Source of yeast | Total protein (mg/L) | OD600 |
|---|---|---|---|
| 1. Control (Strain ATCC76273) | Example 4 | 30.3 | 34.1 |
| 2. CCA36173(SEQ ID NO:95) | Example 4 | 88.6 | 33.6 |
| 3. CCA37695(SEQ ID NO:96) | Example 4 | 69.2 | 34.8 |
| 4. CCA41161(SEQ ID NO:97) | Example 4 | 88.7 | 27.7 |
| 5. CCA41167(SEQ ID NO:98) | Example 4 | 76.4 | 34.1 |
| 6. CCA37701(SEQ ID NO:99) | Example 4 | 56.0 | 36.2 |
| 7. CCA40175(SEQ ID NO:100) | Example 4 | 80.8 | 32.8 |
| 8. CCA37509(SEQ ID NO:101) | Example 4 | 36.3 | 35.2 |
| 9. CCA38967(SEQ ID NO:102) | Example 4 | 42.5 | 34.1 |
| 10. CCA37504(SEQ ID NO:103) | Example 4 | 74.6 | 33.6 |
| 11. CAY67126(SEQ ID NO:104) | Example 4 | 67.3 | 35.7 |
| 12. CAY68445(SEQ ID NO:105) | Example 4 | 116.4 | 31.6 |
| 13. CAY68608(SEQ ID NO:106) | Example 4 | 67.4 | 31.7 |
| 14. CAY71233(SEQ ID NO:107) | Example 4 | 86.5 | 34.5 |

As a result, the yeasts expressing polypeptides (2 to 14) clearly showed a higher secretory expression level than *Komagataella pastoris* strain ATCC76273 (1). This indicates that expression of a polypeptide having an amino acid sequence shown in any of SEQ ID NOs: 95 to 107 improves secretion productivity of endogenous proteins.

<Example 9: Measurement of amount of secreted anti-β galactosidase single-chain antibody by elisa method

The expression level of anti-β galactosidase single-chain antibodies secreted into culture supernatant obtained in Example 7 was determined by a sandwich ELISA assay (Enzyme-Linked Immunosorbent Assay) as described below.

β-galactosidase (5 mg/mL, from F. Hoffmann-La Roche, Ltd.) diluted 2500-fold with an immobilization buffer (8 g/L sodium chloride, 0.2 g/L potassium chloride, 1.15 g/L sodium hydrogenphosphate (anhydrous), 0.2g/L potassium dihydrogenphosphate (anhydrous), 1 mM magnesium chloride) was added to an ELISA plate (Nunc Immuno Plate Maxisorp (from Thermo Fisher Scientific Inc.)) at 50 µL/well and was incubated overnight at 4°C. After incubation, the solution in the wells was removed and the plate was blocked with 200 µl of *ImmunoBlock (from Sumitomo Dainippon Pharma Co., Ltd.) and* allowed to stand for one hour at room temperature. After washing with PBST buffer (8 g/L sodium chloride, 0.2 g/L potassium chloride, 1.15 g/L sodium hydrogenphosphate (anhydrous), 0.2 g/L potassium dihydrogenphosphate (anhydrous), 0.1% Tween20) three times, serially diluted standard anti-β galactosidase single-chain antibody and diluted culture supernatant were added at 50 µl/well and allowed to react for one hour at room temperature. After washing with PBST buffer three times, secondary antibody solution diluted 8000-fold in PBST buffer (secondary antibody: Anti-His-tag mAb-HRP-DirecT (from MBL)) was added at 50 µl/well and allowed to react for one hour at room temperature. After washing with PBST buffer three times, 50 µl of TMB-1 Component Microwell Peroxidase Substrate SureBlue (from KPL) was added and allowed to stand for 20 minutes at room temperature. The reaction was stopped by adding 50 µl of TMB Stop Solution (from KPL), and then the absorbance at 450 nm was measured using a microplate reader (Spectra Max Paradigm from Molecular Devices, LLC.). Quantification of the anti-β galactosidase single-chain antibody in the culture supernatant was performed by using a standard curve of the standard anti-β galactosidase single-chain antibody. The secretory expression of anti-β galactosidase single-chain antibodies determined by this method and their respective cell body concentrations (OD600) were shown in Table 4.

In Table 4, control (1) represents the results when the culture supernatant of a yeast expressing an anti-β galactosidase single-chain antibody was used and (2) to (22) represents the results when the culture supernatants of yeasts expressing an anti-β galactosidase single-chain antibody and respective polypeptides were used.

As a result, the yeasts expressing the anti-β galactosidase single-chain antibody and polypeptides (2 to 14) clearly showed a higher secretory expression level than the yeast expressing the anti-β galactosidase single-chain antibody (1). This indicates that expression of a polypeptide having an amino acid sequence shown in any of SEQ ID NOs: 95 to 107 improves secretory expression of heterologous proteins.

Yeasts expressing an anti-β galactosidase single-chain antibody and a C-terminally deleted polypeptide (15 to 22) also showed a higher secretory expression level than the yeast expressing the anti-β galactosidase single-chain antibody (1). However, the secretory expression level tended to be lower compared to the yeasts expressing the anti-β galactosidase single-chain antibody and polypeptides (2 to 14). This suggests that SEQ ID NOs: 108 to 113, 117, and 120, which are N-terminal side sequences of the polypeptides have an effect of improving secretory expression of the proteins and the C-terminal side sequences enhance this effect.

**[Table 4]**

| Expressed protein | Source of yeast | Single-chain antibody (mg/L) | OD600 |
|---|---|---|---|
| 1. Control (yeast expressing anti-β galactosidase single-chain antibody) | Example 4 | 455.1 | 34.9 |
| 2. CCA36173(SEQ ID NO:95) | Example 4 | 635.4 | 32.2 |
| 3. CCA37695(SEQ ID NO:96) | Example 4 | 734.8 | 33.6 |
| 4. CCA41161(SEQ ID NO:97) | Example 4 | 688.0 | 33.5 |
| 5. CCA41167(SEQ ID NO:98) | Example 4 | 696.4 | 31.8 |
| 6. CCA37701(SEQ ID NO:99) | Example 4 | 637.1 | 30.6 |
| 7. CCA40175(SEQ ID NO:100) | Example 4 | 751.3 | 29.8 |
| 8. CCA37509(SEQ ID NO:101) | Example 4 | 553.2 | 34.6 |
| 9. CCA38967(SEQ ID NO:102) | Example 4 | 500.6 | 34.3 |
| 10. CCA37504(SEQ ID NO:103) | Example 4 | 515.7 | 31.5 |
| 11. CAY67126(SEQ ID NO:104) | Example 4 | 565.8 | 33.3 |
| 12. CAY68445(SEQ ID NO:105) | Example 4 | 516.4 | 33.7 |
| 13. CAY68608(SEQ ID NO:106) | Example 4 | 533.9 | 33.2 |
| 14. CAY71233(SEQ ID NO:107) | Example 4 | 653.3 | 32.7 |
| 15. CCA36173Cterdel(SEQ ID NO:108) | Example 6 | 592.6 | 33.6 |
| 16. CCA37695Cterdel(SEQ ID NO:109) | Example 6 | 673.4 | 34.7 |
| 17. CCA41161Cterdel(SEQ ID NO:110) | Example 6 | 555.8 | 34.4 |
| 18. CCA41167Cterdel(SEQ ID NO:111) | Example 6 | 572.7 | 32.6 |
| 19. CCA37701Cterdel(SEQ ID NO:112) | Example 6 | 583.7 | 34.2 |
| 20. CCA40175Cterdel(SEQ ID NO:113) | Example 6 | 568.5 | 32.6 |
| 21. CAY67126Cterdel(SEQ ID NO:117) | Example 6 | 555.0 | 34.0 |
| 22. CAY71233Cterdel(SEQ ID NO:120) | Example 6 | 626.1 | 32.8 |

All publications, patents, and patent applications cited herein are herein incorporated by citation in their entireties.

## Claims

1. A vector comprising:
(a) a nucleotide sequence encoding an amino acid sequence as set forth in any of SEQ ID NOs: 113, 108 to 112, and 114 to 120,
(b) a nucleotide sequence encoding an amino acid sequence in which one or more amino acids are substituted, deleted, and/or added in the amino acid sequence as set forth in the (a),
(c) a nucleotide sequence encoding an amino acid sequence having a sequence identity of 85% or more to the amino acid sequence as set forth in the (a), or
(d) a nucleotide sequence of a nucleic acid that hybridizes under stringent conditions to a nucleic acid consisting of a complementary sequence to a nucleotide sequence encoding the amino acid sequence as set forth in the (a).

2. The vector according to claim 1, wherein the nucleotide sequences according to the (a) to (d) are respectively:
(a') a nucleotide sequence as set forth in any of SEQ ID NOs: 75, 65, 67, 69, 71, 73, 77, 79, 81, 83, 85, 87, and 89,
(b') a nucleotide sequence in which one or more nucleotides are substituted, deleted, and/or added in the nucleotide sequence as set forth in the (a'),
(c') a nucleotide sequence having a sequence identity of 85% or more to the nucleotide sequence as set forth in the (a'), and
(d') a nucleotide sequence of a nucleic acid that hybridizes under stringent conditions to a nucleic acid consisting of a complementary sequence to the nucleotide sequence as set forth in the (a').

3. A vector comprising:
(e) a nucleotide sequence encoding an amino acid sequence as set forth in any of SEQ ID NOs: 100, 95 to 99, and 101 to 107,
(f) a nucleotide sequence encoding an amino acid sequence in which one or more amino acids are substituted, deleted, and/or added in the amino acid sequence as set forth in the (e),
(g) a nucleotide sequence encoding an amino acid sequence having a sequence identity of 85% or more to the amino acid sequence as set forth in the (e), or
(h) a nucleotide sequence of a nucleic acid that hybridizes under stringent conditions to a nucleic acid consisting of a complementary sequence to a nucleotide sequence encoding the amino acid sequence as set forth in the (e).

4. The vector according to claim 3, wherein the nucleotide sequences according to the (e) to (h) are respectively:
(e') a nucleotide sequence as set forth in any of SEQ ID NOs: 39, 24, 27, 30, 33, 36, 42, 45, 48, 51, 54, 57, and 60,
(f) a nucleotide sequence in which one or more nucleotides are substituted, deleted, and/or added in the nucleotide sequence as set forth in the (e'),
(g') a nucleotide sequence having a sequence identity of 85% or more to the nucleotide sequence as set forth in the (e'), and
(h') a nucleotide sequence of a nucleic acid that hybridizes under stringent conditions to a nucleic acid consisting of a complementary sequence to the nucleotide sequence as set forth in the (e').

5. The vector according to any one of claims 1 to 4, wherein the vector increases a secretion amount of a protein in a host cell.

6. A protein secretion enhancer consisting of the vector according to any one of claims 1 to 5.

7. A mutant cell having an increased expression of a gene comprising the nucleotide sequence defined in any one of claims 1 to 4 compared to that of a wild-type, and an increased secretion amount of a protein compared to that of the wild-type.

8. A cell comprising the vector according to any one of claims 1 to 5.

9. The cell according to claim 7 or 8, wherein the cell is a yeast, a bacterium, a fungus, an insect cell, an animal cell, or a plant cell.

10. The cell according to claim 9, wherein the yeast is a methanol-utilizing yeast, a fission yeast, or a budding yeast.

11. The cell according to claim 10, wherein the methanol-utilizing yeast is a yeast belonging to the genus *Komagataella* or a yeast belonging to the genus *Ogataea.*

12. A method for preparing a mutant strain, comprising a step of introducing the vector according to any one of claims 1 to 5 or the protein secretion enhancer according to claim 6 into a host cell, wherein the mutant strain has an increased secretion amount of a protein compared to that of the host cell before the introduction.

13. A method for producing a protein of interest, comprising:
a step of culturing the cell according to any one of claims 7 to 11, and
a step of recovering the protein of interest from a culture medium.

14. The method according to claim 13, wherein the protein of interest is a heterologous protein.

15. The method according to claim 13 or 14, wherein a culture medium comprising one or more carbon sources selected from the group consisting of glucose, glycerol and methanol is used in the step of culturing.
